(19) Europäisches Patentamt
European Patent Office
Office européen des brevets

(11) **EP 3 955 891 B1**

(12) **EUROPEAN PATENT SPECIFICATION**

(45) Date of publication and mention of the grant of the patent:
**30.07.2025 Bulletin 2025/31**

(21) Application number: **20724998.8**

(22) Date of filing: **17.04.2020**

(51) International Patent Classification (IPC):
*A61K 9/00* (2006.01)     *A61K 9/19* (2006.01)
*A61K 31/195* (2006.01)     *A61K 35/76* (2015.01)
*A61P 7/00* (2006.01)

(52) Cooperative Patent Classification (CPC):
**A61K 9/19; A61K 9/0019; A61K 31/195;**
**A61P 7/00; C12N 7/00; C12N 15/86;**
C12N 2750/14121; C12N 2750/14143;
C12N 2750/14151

(86) International application number:
**PCT/US2020/028716**

(87) International publication number:
**WO 2020/214929 (22.10.2020 Gazette 2020/43)**

(54) **ADENO-ASSOCIATED VIRUS VECTOR FORMULATIONS AND METHODS**

ADENO-ASSOZIIERTE VIRUSVEKTORFORMULIERUNGEN UND VERFAHREN

FORMULATIONS DE VECTEURS DE VIRUS ADÉNO-ASSOCIÉS ET MÉTHODES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2019 US 201962836115 P**

(43) Date of publication of application:
**23.02.2022 Bulletin 2022/08**

(60) Divisional application:
**23186932.2 / 4 272 817**

(73) Proprietor: **REGENXBIO Inc.**
**Rockville, MD 20850 (US)**

(72) Inventors:
• **MARSHALL, Tristan**
  **Potomac, MD 20854 (US)**
• **BEE, Jared**
  **Gaithersburg, MD 20878 (US)**
• **ZHANG, Yu**
  **Gaithersburg, MD 20878 (US)**
• **DEPAZ, Roberto**
  **Bethesda, MD 20814 (US)**

(74) Representative: **Jones Day**
**Rechtsanwälte, Attorneys-at-Law, Patentanwälte**
**Gewürzmühlstraße 11**
**80538 München (DE)**

(56) References cited:
**WO-A1-00/29024          WO-A1-2018/128689**
**US-A1- 2002 031 527**

• **WRIGHT J FRASER ET AL: "Recombinant adeno-associated virus: formulation challenges and strategies for a gene therapy vector", CURRENT OPINION IN DRUG DISCOVERY AND DEVELOPMENT, CURRENT DRUGS, LONDON, GB, vol. 6, no. 2, 1 March 2003 (2003-03-01), pages 174 - 178, XP009092903, ISSN: 1367-6733**

Remarks:
The complete document including Reference Table(s) and the Sequence Listing(s) can be downloaded from the EPO website

**Description**

**2. INTRODUCTION**

Stable formulations and methods of formulating recombinant adeno-associated virus (rAAV) products are described and are suitable for the delivery of therapeutic rAAV products to human subjects to treat a variety of diseases and disorders.

**3. BACKGROUND OF THE INVENTION**

Recombinant Adeno-Associated Virus (AAV)-based vectors are currently the most widely used gene therapy products in development. The preferred use of rAAV vector systems is due, in part, to the lack of disease associated with the wild-type virus, the ability of AAV to transduce non-dividing as well as dividing cells, and the resulting long-term robust transgene expression observed in clinical trials and that indicate great potential for delivery in gene therapy indications. Additionally, different naturally occurring and recombinant rAAV vector serotypes, specifically target different tissues, organs, and cells, and help evade any pre-existing immunity to the vector, thus expanding the therapeutic applications of AAV-based gene therapies.

In addition to demonstrating safety and efficacy, an AAV product must remain stable and potent during manufacture, shipping, storage, and administration. For the commercialization of any pharmaceutical product, it would be advantageous to identify formulations that offer stability for extended periods of time, whether the requirement for shipment or storage be at ambient temperatures, under refrigerated conditions, or under frozen conditions. For rAAV products, the formulation must be able to withstand the stresses introduced by various conditions and still maintain product quality. Thus, there is a need for improved formulations comprising recombinant AAV particles. WO 2018/128689 A1 describes a pharmaceutical composition comprising AAV, and L-histidine is used as the buffering agent regardless of AAV serotype. US 2002/031527 A1 and WO 00/29024 A1 relate to formulations of adenovirus. In the article by Wright J Fraser et al published in Current Opinion in Drug Discovery and Development, Current Drugs, London, GB, vol 6, no 2, 1 March 2003, pages 174-178, formulation challenges and strategies for rAAV are discussed.

**4. SUMMARY OF THE INVENTION**

The disclosure provides a formulation comprising recombinant adeno-associated virus (rAAV) particles and a buffering agent Tris, a sugar as a cryoprotective and lyoprotective excipient, and an amorphous salt. Hereinafter, reference to a rAAV particle in the context of the "invention" and its "emobodiments" means a rAAV particle comprising an AAV8 capsid protein. In some embodiments, provided herein is thus a stable formulation comprising recombinant adeno-associated virus (rAAV) particles and a buffering agent Tris, a sugar, and an amorphous salt. Accoring to the invention, the formulation is suitable for lyophilization.

In some embodiments, the buffer comprises Tris, sucrose, and less than about 100 mM sodium citrate. In some embodiments, the formulation further comprises a non-ionic surfactant, for example, poloxamer 188. In some embodiments, the formulation further comprises a plasticizer or stabilizer, for example, glycerol. The rAAV may comprise a capsid protein of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16. In some examples, the rAAV comprises a capsid protein of the AAV-8 or AAV-9 serotype.

The disclosure provides a formulation comprising recombinant adeno-associated virus (rAAV) particles and a buffering agent Tris, a sugar, and a salt comprising sodium citrate. In some embodiments, the buffer comprises Tris, sucrose, and less than about 100 mM sodium citrate. In some embodiments, the formulation further comprises a non-ionic surfactant, for example, poloxamer 188. In some embodiments, the formulation further comprises a plasticizer or stabilizer, for example, glycerol. The rAAV may comprise a capsid protein of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16. In some examples, the rAAV comprises a capsid protein of the AAV-8 or AAV-9 serotype.

According to the invention, the formulation is suitable for lyophilization. In some embodiments, the pharmaceutical composition is a lyophilized composition from a liquid composition disclosed herein. In some embodiments, the pharmaceutical composition is a reconstituted lyophilized formulation.

In some embodiments, the formulation is a liquid formulation. In some embodiments, the formulation is a frozen formulation. In some embodiments, the formulation is a formulation lyophilized from a liquid formulation disclosed herein. In some embodiments, the formulation is a reconstituted lyophilized formulation.

In some embodiments, the formulation is a lyophilized formulation comprising a residual moisture content between about 1% and about 7%.

The disclosure also provides a method of producing a stable formulation comprising recombinant adeno-associated virus (rAAV) particles.

The disclosure also provides a method of reducing rAAV genome release from rAAV particles.

The disclosure also provides a use of a sugar for reducing rAAV genome release from rAAV particles.

The disclosure also provides a use of a plasticizer for reducing rAAV genome release from rAAV particles.

The disclosure also provides a method of treating a disease or disorder in a subject in need thereof comprising administering a formulation comprising recombinant AAV particles disclosed herein.In the following formulations and methods, wherein in the context of the invention and its embodiments, rAAV particles comprise an AAV8 capsid protein.

In some embodiments, the disclosure provides :

[1.] A stable formulation comprising recombinant adeno-associated virus (rAAV) particles and

a) a buffering agent Tris,
b) a sugar, and
c) an amorphous salt comprising sodium citrate;

[2.] the formulation of [1], wherein the buffering agent comprises between about 1 mM and about 50 mM Tris;

[3.] the formulation of [2] comprising between about 1 mM and about 30 mM, between about 1 mM and about 20 mM, between about 5 mM and about 30 mM, between about 5 mM and about 20 mM, between about 10 mM and about 30 mM, between about 10 mM and about 20 mM, or between about 20 mM and about 50 mM Tris;

[4.] the formulation of [2] comprising about 1 mM, about 2 mM, about 3 mM, about 5 mM, about 10 mM, about 15 mM, about 20 mM, about 25 mM, about 30 mM, or about 40 mM Tris;

[5.] the formulation of [2] comprising about 5 mM Tris;

[6.] the formulation of any one of [1] to [4] having a pH of between about 6.5 and 8.0;

[7.] the formulation of [6] having a pH of between about 7.2 and 7.8;

[8.] the formulation of [6] having a pH of about 7.2, 7.3, 7.4, 7.5, 7.6, 7.7, or 7.8;

[9.] the formulation of [6] having a pH of about 7.5;

[10.] the formulation of any one of [1] to [9] comprising between about 50 mM and about 400 mM sugar;

[11.] the formulation of [10] comprising between about 50 mM and about 350 mM, between about 50 mM and about 300 mM, between about 50 mM and about 250 mM, between about 50 mM and about 200 mM, or between about 50 mM and about 150 mM sugar;

[12.] the formulation of [10] comprising between about 100 mM and about 400 mM, between about 150 mM and about 400 mM, between about 200 mM and about 400 mM, between about 250 mM and about 400 mM, or between about 300 mM and about 400 mM sugar;

[13.] the formulation of [10] comprising between about 100 mM and about 300 mM, between about 150 mM and about 250 mM, between about 200 mM and about 300 mM, or between about 250 mM and about 350 mM sugar;

[14.] the formulation of [10] comprising about 50 mM, about 100 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, or about 350 mM sugar;

[15.] the formulation of [10] comprising between about 190 mM and about 230 mM, between about 170 mM and about 250 mM, or between about 150 mM and about 270 mM sugar;

[16.] the formulation of [10] comprising about 210 mM sugar;

[17.] the formulation of any one of [1] to [16], wherein the sugar is a non-reducing sugar;

[18.] the formulation of [17], wherein the non-reducing sugar is sucrose, trehalose, or raffinose;

[19.] the formulation of [17], wherein the non-reducing sugar is sucrose;

[20.] the formulation of any one of [1] to [16], wherein the sugar is a reducing sugar;

[21.] the formulation of [20], wherein the reducing sugar is glucose, fructose, mannose, galactose, or lactose;

[22.] the formulation of [20], wherein the reducing sugar is dextrose;

[23.] the formulation of any one of [1] to [22] comprising less than about 100 mM sodium citrate;

[24.] the formulation of [23] comprising between about 10 mM and about 100 mM sodium citrate;

[25.] the formulation of [23] comprising between about 10 mM and about 100 mM, between about 20 mM and about 100 mM, between about 30 mM and about 100 mM, between about 40 mM and about 100 mM, between about 50 mM and about 100 mM, between about 10 mM and about 80 mM, between about 10 mM and about 60 mM, between about 10 mM and about 50 mM, between about 10 mM and about 40 mM, or between about 10 mM and about 30 mM sodium citrate;

[26.] the formulation of [23] comprising between about 10 mM and about 50 mM, between about 20 mM and about 60 mM, between about 30 mM and about 70 mM, or between about 10 mM and about 30 mM sodium citrate;

[27.] the formulation of [23] comprising about 10 mM, about 20 mM, about 30 mM, about 40 mM, about 50 mM, or about 60 mM sodium citrate;

[28.] the formulation of [23] comprising about 20 mM sodium citrate;

[29.] the formulation of any one of [1] to [28], further comprising between about 0.0005% and about 0.01% nonionic surfactant;

[30.] the formulation of [29], comprising about 0.002% nonionic surfactant;

[31.] the formulation of [28] or [29], wherein the nonionic surfactant comprises poloxamer 188, poloxamer 407, polysorbate 80, polysorbate 20, Pluronic F-68, or BRIJ 35;

[32.] the formulation of [31], wherein the nonionic surfactant comprises poloxamer 188;

[33.] a stable formulation comprising recombinant adeno-associated virus (rAAV) particles and

    a) between about 1 mM and about 25 mM Tris,
    b) between about 50 mM and about 400 mM sugar,
    c) between about 10 mM and about 100 mM sodium citrate, and
    d) between about 0.0005% and about 0.01 % non-ionic surfactant,

wherein the formulation has a pH of between about 7.2 and about 7.8;

[34.] a stable formulation comprising recombinant adeno-associated virus (rAAV) particles and

    a) between about 2 mM and about 10 mM Tris,
    b) between about 150 mM and about 250 mM sugar,
    c) between about 10 mM and about 20 mM sodium citrate, and
    d) between about 0.001% and about 0.005 % non-ionic surfactant,

wherein the formulation has a pH of between about 7.2 and about 7.8;

[35.] a stable formulation comprising recombinant adeno-associated virus (rAAV) particles and

    a) about 5 mM Tris,
    b) about 210 mM sugar,
    c) about 20 mM sodium citrate, and
    d) about 0.002 % non-ionic surfactant,

wherein the formulation has a pH of about 7.5;

[36.] the formulation of any one of [33] to [35], wherein the sugar is a non-reducing sugar;

[37.] the formulation of [36], wherein the non-reducing sugar is sucrose, trehalose, or raffinose;

[38.] the formulation of [17], wherein the non-reducing sugar is sucrose;

[39.] the formulation of any one of [1] to [38] comprising between about 1.0E+11 genome copy/ mL (GC/mL) and about 1.0E+15 GC/mL rAAV particles;

[40.] the formulation of [39] comprising about 1.0E+11 GC/mL, about 1.0E+12 GC/mL, about 1.0E+13 GC/mL, about 1.0E+14 GC/mL, or about 1.0E+15 GC/ml rAAV particles;

[41.] the formulation of any one of [1] to [40], wherein the rAAV particles comprise a capsid protein of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16;

[42.] the formulation of [41], wherein the rAAV particles comprise a capsid protein of the AAV-8 or AAV-9 serotype;

[43.] the formulation of any one of [1] to [42] further comprising a plasticizer selected from the group consisting of glycerol, xylitol, sorbitol, or mannitol;

[44.] the formulation of any one of [1] to [42] further comprising glycerol;

[45.] the formulation of [44], comprising between about 0.1% and between about 5% glycerol;

[46.] the formulation of [44], comprising between about 0.1% and between about 2% glycerol;

[47.] the formulation of [44], comprising between about 0.25% and between about 2% glycerol;

[48.] the formulation of any one of [1] to [47] that is a liquid formulation;

[49.] the formulation of any one of [1] to [47] that is a frozen formulation;

[50.] the formulation of any one of [1] to [47] that is a lyophilized formulation or a reconstituted lyophilized formulation;

[51.] the formulation of [50] having a residual moisture content between about 1% and about 7%;

[52.] the formulation of [51], wherein the residual moisture content is between about 1% and about 7%, between about 2% and about 7%, between about 3% and about 7%, between about 4% and about 7%, between about 5% and about 7%, between about 1% and about 6%, between about 1% and about 5%, between about 1% and about 4%, or between about 1% and about 3%;

[53.] the formulation of [51], wherein the residual moisture content is between about 3% and about 7%, between about 3% and about 6%, or between about 3% and about 5%;

[54.] the formulation of [51], wherein the residual moisture content is about 3%, about 3.5%, about 4%, about 4.5%, about 5%, about 5.5%, or about 6%;

[55.] the formulation of any one of [1] to [54], wherein the % relative potency of the rAAV particles is at least about 60%, at least about 70%, or at least about 80% after storing the formulation for 3 months at room temperature; wherein the reference rAAV particles are stored at -70°C in Dulbecco's phosphate-buffered saline (DPBS) with 0.001% poloxamer 188 buffer;

[56.] the formulation of any one of [1] to [54], wherein the % relative potency of the rAAV particles is at least about 60%, at least about 70%, or at least about 80% after storing the formulation for 6 months at room temperature, wherein the reference rAAV particles are stored at -70°C in DPBS with 0.001% poloxamer 188 buffer;

[57.] the formulation of any one of [1] to [54], wherein the % relative potency of the rAAV particles is at least about 30%, at least about 40%, at least about 50%, or at least 60% after storing the formulation for 1 week at 35°C, wherein the reference rAAV particles are stored at -70°C in DPBS with 0.001% poloxamer 188 buffer;

[58.] the formulation of any one of [1] to [54], wherein the % relative potency of the rAAV particles is at least about 30%, at least about 40%, at least about 50%, or at least 60% after storing the formulation for 2 weeks at 35°C, wherein the reference rAAV particles are stored at -70°C in DPBS with 0.001% poloxamer 188 buffer;

[59.] the formulation of any one of [1] to [54], wherein the % relative potency of the rAAV particles is at least about 30%, at least about 40%, at least about 50%, or at least 60% after storing the formulation for 4 weeks at 35°C, wherein the reference rAAV particles are stored at -70°C in DPBS with 0.001% poloxamer 188 buffer;

[60.] a method of producing a stable formulation comprising recombinant adeno-associated virus (rAAV) particles, comprising combining rAAV particles with a buffering agent Tris, a sugar, an amorphous salt, optionally a plasticizer, and optionally a nonionic surfactant of the formulation according to any one of [1] to [47, thereby producing the formulation comprising rAAV;

[61.] a method of reducing rAAV genome release from rAAV particles, comprising producing a formulation comprising rAAV particles, a buffering agent Tris, a sugar, an amorphous salt, and optionally a nonionic surfactant , wherein rAAV genome release from the rAAV particles after three freeze-thaw cycles is reduced compared to rAAV genome release in a formulation not comprising the sugar;

[62.] a method of reducing rAAV genome release from rAAV particles, comprising producing a formulation comprising rAAV particles, a buffering agent Tris, a sugar, an amorphous salt, and optionally a nonionic surfactant, wherein rAAV genome release from the rAAV particles after lyophilization and reconstitution is reduced compared to rAAV genome release in a formulation not comprising the sugar;

[63.] the method of [61] or [62], further comprising lyophilizing the formulation to achieve a residual moisture content between about 1% and about 5%;

[64.] a method of reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar;

[65.] a method of reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar;

[66.] use of a sugar for reducing rAAV genome release from rAAV particles, comprising producing a formulation comprising rAAV particles, a buffering agent, a sugar, a salt, and optionally a nonionic surfactant, wherein rAAV genome release from the rAAV particles after three freeze-thaw cycles is reduced compared to rAAV genome release in a formulation not comprising the sugar;

[67.] use of a sugar for reducing rAAV genome release from rAAV particles, comprising producing a formulation comprising rAAV particles, a buffering agent, a sugar, a salt, and optionally a nonionic surfactant, wherein rAAV genome release from the rAAV particles after lyophilization and reconstitution is reduced compared to rAAV genome release in a formulation not comprising the sugar;

[68.] the use of [66] or [67], further comprising lyophilizing the formulation to achieve a residual moisture content between about 1% and about 7%;

[69.] use of a plasticizer for reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar;

[70.] use of a plasticizer for reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar;

[71.] the method of any one of [61]-[65] or the use of any one of [66-70], wherein rAAV genome release is determined by measuring relative fluorescence in the presence of a DNA specific fluorescent stain;

[72.] the method of any one of [61]-[65] and [71] or the use of any one of [66]-[71], wherein freezing-induced rAAV genome release is reduced by at least about 10%, 20%, 50%, 80%, or 90%;

[73.] the method of any one of [61]-[65] and [71] or the use of any one of [66]-[71], wherein freezing-induced rAAV genome release is substantially eliminated;

[74.] the method of any one of [61]-[65] and [71]-[73] or the use of any one of [66]-[73], wherein the sugar is a non-reducing sugar;

[75.] the method of [74] or the use of [74], wherein the non-reducing sugar is sucrose, trehalose, or raffinose;

[76.] the method of [74] or the use of [74], wherein the non-reducing sugar is sucrose;

[77.] the method of any one of [61]-[65] and [71]-[73] or the use of any one of [66]-[73], wherein the sugar is a reducing sugar;

[78.] the method of [77] or the use of [77], wherein the reducing sugar is glucose, fructose, mannose, galactose, or lactose;

[79.] the method of [77] or the use of [77], wherein the reducing sugar is dextrose;

[80.] the method of any one of [61]-[65] and [71]-[79] or the use of any one of [66]-[79], wherein the plasticizer comprises glycerol;

[81.] the method of any one of [61]-[65] and [71]-[80] or the use of any one of [66]-[80], wherein the formulation is according to any one of [1] to [47].

In some examples, a method disclosed herein comprises producing a stable formulation comprising recombinant adeno-associated virus (rAAV) particles, wherein the rAAV particles are produced by isolating rAAV particles from a feed comprising an impurity (for example, rAAV production culture), wherein the method for isolating rAAV particles comprises one or more processing steps. In some embodiments, the processing is at least one of harvest of a cell culture, clarification of the harvested cell culture (e.g., by centrifugation or depth filtration), tangential flow filtration, affinity chromatography, anion exchange chromatography, cation exchange chromatography, size exclusion chromatography, hydrophobic interaction chromatography, sterile filtration. In further embodiments, the processing includes at least 2, at least 3, at least 4, at least 5, or at least 6 of harvest of a cell culture, clarification of the harvested cell culture (e.g., by centrifugation or depth filtration), tangential flow filtration, affinity chromatography, anion exchange chromatography, cation exchange chromatography, size exclusion chromatography, hydrophobic interaction chromatography, and sterile filtration. In some examples, the processing does not include centrifugation of the harvested cell culture.

The disclosure provides a method for producing a stable formulation comprising isolated recombinant adeno-associated virus (rAAV) particles, comprising (a) isolating rAAV particles from a feed comprising an impurity by one or more of centrifugation, depth filtration, tangential flow filtration, ultrafiltration, affinity chromatography, size exclusion chromatography, ion exchange chromatography, and hydrophobic interaction chromatography, and formulating the isolated rAAV particles to produce a stable formulation.

The disclosure provides a method for producing a pharmaceutical unit dosage of a stable formulation comprising isolated recombinant adeno-associated virus (rAAV) particles, comprising (a) isolating rAAV particles from a feed comprising an impurity by one or more of centrifugation, depth filtration, tangential flow filtration, ultrafiltration, affinity chromatography, size exclusion chromatography, ion exchange chromatography, and hydrophobic interaction chromatography, and formulating the isolated rAAV particles.

In certain embodiments, provided herein is a stable formulation comprising recombinant adeno-associated virus (rAAV) particles and

a) about 5 mM Tris,
b) about 210 mM sucrose,
c) about 20 mM sodium citrate, and
d) about 0.002 % (w/v) poloxamer 188.

In certain embodiments, provided herein is a stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.25% (w/v) glycerol.

In certain embodiments, provided herein is a stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.5% (w/v) sorbitol.

In certain embodiments, provided herein is a stable formulation comprising recombinant adeno-associated virus (rAAV) particles and

a) about 5 mM Tris,
a) about 30 mM sodium sulfate,
b) about 263 mM sucrose, and

about 0.005 % (w/v) poloxamer 188.

In certain embodiments, provided herein is a stable formulation comprising recombinant adeno-associated virus (rAAV) particles and

a) Tris,
b) sucrose,
c) sodium citrate, and
d) poloxamer 188,

wherein the formulation has an ionic strength between 60 mM and 150 mM.

In certain embodiments, provided herein is a stable formulation comprising recombinant adeno-associated virus (rAAV) particles and

a) Tris,
b) sucrose,
c) sodium citrate,
d) poloxamer 188, and
e) glycerol,

wherein the formulation has an ionic strength between 60 mM and 150 mM.

In certain embodiments, provided herein is a stable formulation comprising recombinant adeno-associated virus (rAAV) particles and

a) Tris,
b) sucrose,
c) sodium citrate,
d) poloxamer 188, and
e) sorbitol,

wherein the formulation has an ionic strength between 60 mM and 150 mM.

In certain embodiments, provided herein is a stable formulation comprising recombinant adeno-associated virus (rAAV) particles and

a) Tris,
b) sodium sulfate,
c) sucrose, and
d) poloxamer 188,

wherein the formulation has an ionic strength between 60 mM and 150 mM.

In certain embodiments, provided herein is a stable formulation comprising recombinant adeno-associated virus (rAAV) particles and

a) about 5 mM Tris,
b) about 210 mM sucrose,
c) about 20 mM sodium citrate, and
d) about 0.002 % (w/v) poloxamer 188.

In certain embodiments, provided herein is a stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.25% (w/v) glycerol.

In certain embodiments, provided herein is a stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.5% (w/v) sorbitol.

In certain embodiments, provided herein is a stable formulation comprising recombinant adeno-associated virus (rAAV) particles and

a) about 5 mM Tris,
b) about 30 mM sodium sulfate,
c) about 263 mM sucrose, and
d) about 0.005 % (w/v) poloxamer 188.

In certain embodiments, provided herein is a stable formulation comprising recombinant adeno-associated virus (rAAV) particles and

a) Tris,
b) sucrose,
c) sodium citrate, and
d) poloxamer 188,

wherein the formulation has an ionic strength between 60mM and 150 mM.

In certain embodiments, provided herein is a stable formulation comprising recombinant adeno-associated virus (rAAV) particles and

a) Tris,
b) sucrose,
c) sodium citrate,
d) poloxamer 188, and
e) glycerol,

wherein the formulation has an ionic strength between 60mM and 150 mM.

In certain embodiments, provided herein is a stable formulation comprising recombinant adeno-associated virus (rAAV) particles and

a) Tris,
b) sucrose,
c) sodium citrate,
d) poloxamer 188, and
e) sorbitol,

wherein the formulation has an ionic strength between 60mM and 150 mM.

In certain embodiments, provided herein is a stable formulation comprising recombinant adeno-associated virus (rAAV) particles and

a) Tris,
b) sodium sulfate,
c) sucrose, and
d) poloxamer 188,

wherein the formulation has an ionic strength between 60mM and 150 mM.

In certain embodiments, provided herein is a stable formulation comprising recombinant adeno-associated virus (rAAV) particles and

a) about 5 mM Tris,
b) about 210 mM sucrose,
c) about 20 mM sodium citrate, and
d) about 0.002 % (w/v) poloxamer 188.

In certain embodiments, provided herein is a stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.25% (w/v) glycerol.

In certain embodiments, provided herein is a stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.5% (w/v) sorbitol.

In certain embodiments, provided herein is a stable formulation comprising recombinant adeno-associated virus (rAAV) particles and

a) about 5 mM Tris,
e) about 30 mM sodium sulfate,
f) about 263 mM sucrose, and
g) about 0.005 % (w/v) poloxamer 188.

In certain embodiments, provided herein is a stable formulation comprising recombinant adeno-associated virus (rAAV) particles and

a) Tris,

    b) sucrose,
    c) sodium citrate, and
    d) poloxamer 188,

wherein the formulation has an ionic strength between 60mM and 150 mM.

In certain embodiments, provided herein is a stable formulation comprising recombinant adeno-associated virus (rAAV) particles and

    a) Tris,
    b) sucrose,
    c) sodium citrate,
    d) poloxamer 188, and
    e) glycerol,

wherein the formulation has an ionic strength between 60mM and 150 mM.

In certain embodiments, provided herein is a stable formulation comprising recombinant adeno-associated virus (rAAV) particles and

    a) Tris,
    b) sucrose,
    c) sodium citrate,
    d) poloxamer 188, and
    e) sorbitol,

wherein the formulation has an ionic strength between 60mM and 150 mM.

In certain embodiments, provided herein is a stable formulation comprising recombinant adeno-associated virus (rAAV) particles and

    a) Tris,
    b) sodium sulfate,
    c) sucrose, and
    d) poloxamer 188,

wherein the formulation has an ionic strength between 60mM and 150 mM.

In certain embodiments, provided herein is a stable formulation comprising recombinant adeno-associated virus (rAAV) particles and

    a) Tris,
    b) sucrose,
    c) sodium citrate, and
    d) poloxamer 188,

wherein the formulation has an ionic strength less than 200 mM.

In certain embodiments, provided herein is a stable formulation comprising recombinant adeno-associated virus (rAAV) particles and

    a) Tris,
    b) sucrose,
    c) sodium citrate,
    d) poloxamer 188, and
    e) glycerol,

wherein the formulation has an ionic strength less than 200 mM.

In certain embodiments, provided herein is a stable formulation comprising recombinant adeno-associated virus (rAAV) particles and

    a) Tris,
    b) sucrose,

c) sodium citrate,
d) poloxamer 188, and
e) sorbitol,

wherein the formulation has an ionic strength less than 200 mM.

In certain embodiments, provided herein is a stable formulation comprising recombinant adeno-associated virus (rAAV) particles and

a) Tris,
b) sodium sulfate,
c) sucrose, and
d) poloxamer 188,

wherein the formulation has an ionic strength less than 200 mM.

In certain embodiments, the stable formulation does not comprise mannitol.

In certain embodiments, the stable formulation comprises less than about 10 mM, about 20 mM, about 30mM, about 40mM, about 50mM, about 60mM, about 70mM, about 80mM, about 90mM, about 100mM, about 110mM, about 120mM, about 130mM, about 140mM, or about 150mM mannitol.

In certain embodiments, the stable formulation comprises sucrose at a concentration of about 210 mM. In certain embodiments, the stable formulation comprises sucrose at a concentration of about 263 mM. In certain embodiments, the stable formulation comprises sucrose at a concentration of about 409 mM. In certain embodiments, the stable formulation comprises sucrose at a concentration of about 14.6 mM. In certain embodiments, the stable formulation comprises sucrose at a concentration of about 45 mM. In certain embodiments, the stable formulation comprises sucrose at a concentration between about 0 mM and about 20 mM. In certain embodiments, the stable formulation comprises sucrose at a concentration between about 20 mM and about 50 mM. In certain embodiments, the stable formulation comprises sucrose at a concentration between about 50 mM and about 100 mM. In certain embodiments, the stable formulation comprises sucrose at a concentration between about 100 mM and about 200 mM. In certain embodiments, the stable formulation comprises sucrose at a concentration between about 200 mM and about 300 mM. In certain embodiments, the stable formulation comprises sucrose at a concentration between about 300 mM and about 400 mM. In certain embodiments, the stable formulation comprises sucrose at a concentration between about 400 mM and about 500 mM. In certain embodiments, the stable formulation comprises sucrose at a concentration between about 500 mM and about 600 mM. In certain embodiments, the stable formulation comprises sucrose at a concentration between about 600 mM and about 700 mM.

In certain embodiments, the stable formulation comprises sorbitol at a concentration of about 0.5%. In certain embodiments, the stable formulation comprises sorbitol at a concentration of about 0.25%. In certain embodiments, the stable formulation comprises sorbitol at a concentration of about 0.10%.

In certain embodiments, the stable formulation comprises sorbitol at a concentration of about 0% to about 0.10%. In certain embodiments, the stable formulation comprises sorbitol at a concentration of about 0.10% to about 0.20%. In certain embodiments, the stable formulation comprises sorbitol at a concentration of about 0.20% to about 0.30%. In certain embodiments, the stable formulation comprises sorbitol at a concentration of about 0.30% to about 0.40%. In certain embodiments, the stable formulation comprises sorbitol at a concentration of about 0.40% to about 0.50%. In certain embodiments, the stable formulation comprises sorbitol at a concentration of about 0.50% to about 0.60%. In certain embodiments, the stable formulation comprises sorbitol at a concentration of about 0.60% to about 0.70%. In certain embodiments, the stable formulation comprises sorbitol at a concentration of about 0.70% to about 0.80%. In certain embodiments, the stable formulation comprises sorbitol at a concentration of about 0.80% to about 0.90%. In certain embodiments, the stable formulation comprises sorbitol at a concentration of about 0.90% to about 1.00%.

In certain embodiments, the stable formulation comprises glycerol at a concentration of about 0.5%. In certain embodiments, the stable formulation comprises glycerol at a concentration of about 0.25%. In certain embodiments, the stable formulation comprises glycerol at a concentration of about 0.10%.

In certain embodiments, the stable formulation comprises glycerol at a concentration of about 0% to about 0.10%. In certain embodiments, the stable formulation comprises glycerol at a concentration of about 0.10% to about 0.20%. In certain embodiments, the stable formulation comprises glycerol at a concentration of about 0.20% to about 0.30%. In certain embodiments, the stable formulation comprises glycerol at a concentration of about 0.30% to about 0.40%. In certain embodiments, the stable formulation comprises glycerol at a concentration of about 0.40% to about 0.50%. In certain embodiments, the stable formulation comprises glycerol at a concentration of about 0.50% to about 0.60%. In certain embodiments, the stable formulation comprises glycerol at a concentration of about 0.60% to about 0.70%. In certain embodiments, the stable formulation comprises glycerol at a concentration of about 0.70% to about 0.80%. In certain embodiments, the stable formulation comprises glycerol at a concentration of about 0.80% to about 0.90%. In certain embodiments, the stable formulation comprises glycerol at a concentration of about 0.90% to about 1.00%.

In certain embodiments, the stable formulation comprises poloxamer 188 at a concentration of about 0.001% (weight/volume, 0.01 g/L). In certain embodiments, the stable formulation comprises poloxamer 188 at a concentration of about 0.002% (weight/volume, 0.02 g/L). In certain embodiments, the stable formulation comprises poloxamer 188 at a concentration of about 0.005% (weight/volume, 0.05 g/L).

In certain embodiments, the stable formulation comprises poloxamer 188 at a concentration of about 0.0005% (weight/volume, 0.005 g/L) to about 0.05% (weight/volume, 0.5 g/L). In certain embodiments, the stable formulation comprises poloxamer 188 at a concentration of about 0.0001% (weight/volume, 0.001 g/L) to about 0.01% (weight/volume, 0.1 g/L). In certain embodiments, the stable formulation comprises poloxamer 188 at a concentration of about 0.0005% (weight/volume, 0.005 g/L) to about 0.001% (weight/volume, 0.01 g/L). In certain embodiments, the stable formulation comprises poloxamer 188 at a concentration of about 0.001% (weight/volume, 0.01 g/L) to about 0.05% (weight/volume, 0.5 g/L). In certain embodiments, the stable formulation comprises poloxamer 188 at a concentration of about 0.0005% (weight/volume, 0.005 g/L). In certain embodiments, the stable formulation comprises poloxamer 188 at a concentration of about 0.0006% (weight/volume, 0.006 g/L). In certain embodiments, the stable formulation comprises poloxamer 188 at a concentration of about 0.0007% (weight/volume, 0.007 g/L). In certain embodiments, the stable formulation comprises poloxamer 188 at a concentration of about 0.0008% (weight/volume, 0.008 g/L). In certain embodiments, the stable formulation comprises poloxamer 188 at a concentration of about 0.0009% (weight/volume, 0.009 g/L). In certain embodiments, the stable formulation comprises poloxamer 188 at a concentration of about 0.001% (weight/volume, 0.01 g/L). In certain embodiments, the stable formulation comprises poloxamer 188 at a concentration of about 0.002% (weight/volume, 0.02 g/L). In certain embodiments, the stable formulation comprises poloxamer 188 at a concentration of about 0.003% (weight/volume, 0.03 g/L). In certain embodiments, the stable formulation comprises poloxamer 188 at a concentration of about 0.004% (weight/volume, 0.04 g/L). In certain embodiments, the stable formulation comprises poloxamer 188 at a concentration of about 0.005% (weight/volume, 0.05 g/L). In certain embodiments, the stable formulation comprises poloxamer 188 at a concentration of about 0.01% (weight/volume, 0.1 g/L). In certain embodiments, the stable formulation comprises poloxamer 188 at a concentration of about 0.05% (weight/volume, 0.5 g/L).

In some examples, the disclosure provides a stable formulation comprises a recombinant adeno-associated virus (AAV), a salt excipient, a sugar. In some examples, the stable formulation also comprises a surfactant. In some examples, the stable formulation also comprises poloxamer 188. In some examples, the stable formulation is suitable for lyophilization.

In some embodiments, provided herein is a stable formulation comprising recombinant adeno-associated virus (rAAV) particles and a buffering agent Tris; a sugar, and an amorphous salt having an ionic strength between 60mM and 150 mM, wherein the formulation is suitable for lyophilization, and wherein the rAAV particle comprises an AAV8 capsid protein. In some embodiments, the stable formulation also comprises a surfactant. In some embodiments, the stable formulation also comprises poloxamer 188. In some embodiments, the formulation is a pre-lyophilization formulation.

In some embodiments, provided herein is a stable formulation comprising recombinant adeno-associated virus (rAAV) particles and a buffering agent Tris; a sugar, and an amorphous salt having an ionic strength between 30mM and 100 mM, wherein the formulation is suitable for lyophilization, and wherein the rAAV particle comprises an AAV8 capsid protein. In some embodiments, the stable formulation also comprises a surfactant. In some embodiments, the stable formulation also comprises poloxamer 188. In some embodiments, the formulation is a pre-lyophilization formulation.

In some examples, provided herein is a stable formulation comprising recombinant adeno-associated virus (rAAV) particles and a buffering agent, a sugar, and an amorphous salt having an ionic strength higher than 200mM, wherein the formulation is suitable for lyophilization. In some examples, the stable formulation also comprises a surfactant. In some examples, the stable formulation also comprises poloxmer 188. In some examples, the formulation is a pre-lyophilization formulation.

In some embodiments, provided herein is a stable formulation comprising recombinant adeno-associated virus (rAAV) particles and a buffering agent Tris, a sugar, an amorphous salt having an ionic strength between 60mM and 150 mM, and a surfactant, wherein the formulation is suitable for lyophilization, and wherein the rAAV particle comprises an AAV8 capsid protein. In some embodiments, the stable formulation also comprises a surfactant. In some embodiments, the stable formulation also comprises poloxamer 188. In some embodiments, the formulation is a pre-lyophilization formulation.

In some embodiments, provided herein is a stable formulation comprising recombinant adeno-associated virus (rAAV) particles and a buffering agent Tris; a sugar, an amorphous salt having an ionic strength between 30mM and 100 mM, and a surfactant, wherein the formulation is suitable for lyophilization, and wherein the rAAV particle comprises an AAV8 capsid protein. In some embodiments, the stable formulation also comprises a surfactant. In some embodiments, the stable formulation also comprises poloxamer 188. In some embodiments, the formulation is a pre-lyophilization formulation.

In some examples, provided herein is a stable formulation comprising recombinant adeno-associated virus (rAAV) particles and a buffering agent, a sugar, and an amorphous salt having an ionic strength higher than 200mM, and a surfactant wherein the formulation is suitable for lyophilization. In some examples, the stable formulation also comprises a surfactant. In some examples, the stable formulation also comprises poloxamer 188. In some examples, the formulation is a pre-lyophilization formulation.

In some embodiments, a formulation disclosed herein comprises between about 0.0001% and about 0.5% nonionic surfactant. In some embodiments, a formulation disclosed herein comprises between about 0.0005% and about 0.1% nonionic surfactant. In some embodiments, a formulation disclosed herein comprises about 0.001%, about 0.002%, about 0.003%, about 0.004%, about 0.005%, about 0.007%, or about 0.01% nonionic surfactant.

In some embodiments, a formulation disclosed herein comprises between about 0.0001% and about 0.5% poloxamer 188. In some embodiments, a formulation disclosed herein comprises between about 0.0005% and about 0.1% poloxamer 188. In some embodiments, a formulation disclosed herein comprises about 0.001%, about 0.002%, about 0.003%, about 0.004%, about 0.005%, about 0.007%, or about 0.01% poloxamer 188.

In some embodiments, a formulation disclosed herein comprises about 0.0005% poloxamer 188. In some embodiments, a formulation disclosed herein comprises about 0.001% poloxamer 188. In some embodiments, a formulation disclosed herein comprises about 0.002% poloxamer 188. In some embodiments, a formulation disclosed herein comprises about 0.003% poloxamer 188. In some embodiments, a formulation disclosed herein comprises about 0.004% poloxamer 188. In some embodiments, a formulation disclosed herein comprises about 0.005% poloxamer 188. In some embodiments, a formulation disclosed herein comprises about 0.008% poloxamer 188.

In some embodiments, the salt is a pharmaceutically acceptable salt. In some embodiments, the salt is a non-crystallizing, amorphous salt. In some embodiments, the salt is a sodium salt. In some embodiments, the salt is sodium citrate, sodium acetate, or sodium chloride. In some embodiments, the salt is sodium citrate, sodium sulfate, ammonium sulfate or magnesium sulfate. In some embodiment, the salt is a multivalent salt, which is made up of multiply charged ions having higher ionic strength (per molecule excipient added compared to mono-sodium chloride), and may inhibit AAV aggregation while minimizing crystallization.

In some embodiment, the salt excipient is added or adjusted to achieve a desirable ionic strength. In some embodiments, the amorphous salt has an ionic strength no greater than about 150 mM, about 145 mM, about 140 mM, about 135 mM, about 130 mM, about 125 mM, about 120 mM, about 115 mM, about 110 mM, about 110 mM, about 105 mM, or about 100 mM. In certain embodiments, the stable formulation has a buffering agent ionic strength no greater than about 150 mM, about 145 mM, about 140 mM, about 135 mM, about 130 mM, about 125 mM, about 120 mM, about 115 mM, about 110 mM, about 105 mM, or about 100 mM. According to the invention, the rAAV particles in the formulation are rAAV8 particles.

In some embodiments, the stable formulation has an ionic strength no greater than 150 mM, 145 mM, 140 mM, 135 mM, 130 mM, 125 mM, 120 mM, 115 mM, or 110 mM. In certain embodiments, the stable formulation has a buffering agent ionic strength no greater than 150 mM, 145 mM, 140 mM, 135 mM, 130 mM, 125 mM, 120 mM, 115 mM, or 110 mM. Accoring to the invention, the rAAV particles in the formulation have AAV8 capsids.

In certain embodiments, the stable formulation has an ionic strength greater than 60 mM. In certain embodiments, the stable formulation has an ionic strength about 60 mM to 150 mM. In certain embodiments, the stable formulation has an ionic strength about 60 mM to 115 mM. In certain embodiments, the stable formulation has an ionic strength about 60 mM to 100 mM. In a specific embodiment, the stable formulation has an ionic strength about 60 mM. In a specific embodiment, the stable formulation has an ionic strength about 65 mM. In a specific embodiment, the stable formulation has an ionic strength about 70 mM. In a specific embodiment, the stable formulation has an ionic strength about 75 mM. In a specific embodiment, the stable formulation has an ionic strength about 80 mM. In a specific embodiment, the stable formulation has an ionic strength about 85 mM. In a specific embodiment, the stable formulation has an ionic strength about 90 mM. According to the invention, the rAAV particles in the formulation have AAV8 capsids.

In certain embodiments, the stable formulation has an ionic strength greater than 30 mM. In certain embodiments, the stable formulation has an ionic strength about 30 mM to 100 mM. In a specific embodiment, the stable formulation has an ionic strength about 30 mM. In a specific embodiment, the stable formulation has an ionic strength about 35 mM. In a specific embodiment, the stable formulation has an ionic strength about 40 mM. In a specific embodiment, the stable formulation has an ionic strength about 45 mM. In a specific embodiment, the stable formulation has an ionic strength about 50 mM. In a specific embodiment, the stable formulation has an ionic strength about 55 mM. In a specific embodiment, the stable formulation has an ionic strength about 60 mM. In a specific embodiment, the stable formulation has an ionic strength about 65 mM. In a specific embodiment, the stable formulation has an ionic strength about 70 mM. In a specific embodiment, the stable formulation has an ionic strength about 75 mM. In a specific embodiment, the stable formulation has an ionic strength about 80 mM. In a specific embodiment, the stable formulation has an ionic strength about 85 mM. In a specific embodiment, the stable formulation has an ionic strength about 90 mM. In a specific embodiment, the stable formulation has an ionic strength about 95 mM. In a specific embodiment, the stable formulation has an ionic strength about 100 mM. In some examples, the rAAV particles in the formulation have AAV9 capsids.

In certain embodiments, the stable formulation has an ionic strength about 60 mM to 150 mM. In certain embodiments, the stable formulation has an ionic strength about 60 mM to 115 mM. In certain embodiments, the stable formulation has an ionic strength about 65 mM to 95 mM. In certain embodiments, the stable formulation has an ionic strength about 70 mM to 90 mM. In certain embodiments, the stable formulation has an ionic strength about 75 mM to 85 mM. According to the invention, the rAAV particles in the formulation have AAV8 capsids.

In certain embodiments, the stable formulation has a ionic strength about 30 mM to 100 mM. In certain embodiments, the stable formulation has an ionic strength about 35 mM to 95 mM. In certain embodiments, the stable formulation has an ionic strength about 40 mM to 90 mM. In certain embodiments, the stable formulation has an ionic strength about 45 mM to 85 mM. In certain embodiments, the stable formulation has an ionic strength about 50 mM to 80 mM. In certain embodiments, the stable formulation has an ionic strength about 55 mM to 75 mM. In certain embodiments, the stable formulation has an ionic strength about 60 mM to 70 mM. In some embodiments, the rAAV particles in the formulation do not have AAV8 capsids. In some examples, the rAAV particles in the formulation have AAV9 capsids.

In some embodiments, the stable formulation has an ionic strength greater than about 200 mM. In some examples, the rAAV particles in the formulation have AAV2 capsids. In some examples, the rAAV particles in the formulation do not have rAAV8 or rAAV9 capsids.

In some embodiments, the stable formulation has an ionic strength greater than 200 mM, 210 mM, 220 mM, 230 mM, 240 mM, 250 mM, 260 mM, 270 mM, 280 mM, 290mM, or 300mM. In some examples, the rAAV particles in the formulation have AAV2 capsids. In some examples, the rAAV particles in the formulation do not have rAAV8 or rAAV9 capsids.

In certain embodiments, the stable formulation has an ionic strength about 200 mM to about 210 mM. In certain embodiments, the stable formulation has an ionic strength about 210 mM to about 220 mM. In certain embodiments, the stable formulation has an ionic strength about 220 mM to about 230 mM. In certain embodiments, the stable formulation has an ionic strength about 230 mM to about 240 mM. In certain embodiments, the stable formulation has an ionic strength about 240 mM to about 250 mM. In certain embodiments, the stable formulation has an ionic strength about 250 mM to about 260 mM. In certain embodiments, the stable formulation has an ionic strength about 260 mM to about 270 mM. In certain embodiments, the stable formulation has an ionic strength about 270 mM to about 280 mM. In certain embodiments, the stable formulation has an ionic strength about 280 mM to about 290 mM. In certain embodiments, the stable formulation has an ionic strength about 290 mM to about 300 mM. In some examples, the rAAV particles in the formulation do not have AAV8 or AAV9 capsids.

In some embodiments, a formulation disclosed herein comprises between about 0.0001% and about 0.5% nonionic surfactant. In some embodiments, a formulation disclosed herein comprises between about 0.0005% and about 0.1% nonionic surfactant. In some embodiments, a formulation disclosed herein comprises about 0.001%, about 0.002%, about 0.003%, about 0.004%, about 0.005%, about 0.007%, or about 0.01% nonionic surfactant.

In some embodiments, a formulation disclosed herein comprises between about 0.0001% and about 0.5% poloxamer 188. In some embodiments, a formulation disclosed herein comprises between about 0.0005% and about 0.1% poloxamer 188. In some embodiments, a formulation disclosed herein comprises about 0.001%, about 0.002%, about 0.003%, about 0.004%, about 0.005%, about 0.007%, or about 0.01% poloxamer 188.

In some embodiments, a formulation disclosed herein comprises about 0.0005% poloxamer 188. In some embodiments, a formulation disclosed herein comprises about 0.001% poloxamer 188. In some embodiments, a formulation disclosed herein comprises about 0.002% poloxamer 188. In some embodiments, a formulation disclosed herein comprises about 0.003% poloxamer 188. In some embodiments, a formulation disclosed herein comprises about 0.004% poloxamer 188. In some embodiments, a formulation disclosed herein comprises about 0.005% poloxamer 188. In some embodiments, a formulation disclosed herein comprises about 0.008% poloxamer 188.

In some embodiments, the salt is a pharmaceutically acceptable salt. In some embodiments, the salt is a non-crystallizing, amorphous salt. In some embodiments, the salt is a sodium salt. In some embodiments, the salt is sodium citrate, sodium acetate, or sodium chloride. In some embodiments, the salt is sodium citrate, sodium sulfate, ammonium sulfate or magnesium sulfate. In some embodiment, the salt is a multivalent salts, which is made up of multiply charged ions having higher ionic strength (per molecule excipient added compared to mono-sodium chloride), and may inhibit AAV aggregation while minimizing crystallization.

In some embodiments, the stable formulation has an ionic strength no greater than about 200mM, about 195mM, 180mM, 175mM, 170mM, 165mM, 160mM, 155mM, 150 mM, 145 mM, 140 mM, 135 mM, 130 mM, 125 mM, 120 mM, 115 mM, or 110 mM.

In some embodiment, the salt excipient is added or adjusted to achieve a desirable ionic strength. In some embodiments, the stable formulation has an ionic strength no greater than about 200mM, about 190mM, about 180mM, about 170mM about 160mM, about 155mM, about 150 mM, about 145 mM, about 140 mM, about 135 mM, about 130 mM, about 125 mM, about 120 mM, about 115 mM, or about 110 mM. In certain embodiments, the stable formulation has a buffering agent ionic strength no greater than about 150 mM, about 145 mM, about 140 mM, about 135 mM, about 130 mM, about 125 mM, about 120 mM, about 115 mM, or about 110 mM. According to the invention, the rAAV particles in the formulation are rAAV8 particles. In some examples, the rAAV particles in the formulation have AAV9 capsids.

In certain embodiments, the stable formulation has a ionic strength about 60 mM to 150 mM. In certain embodiments, the stable formulation has a ionic strength about 60 mM to 115 mM. In certain embodiments, the stable formulation has a ionic strength about 60 mM to 100 mM. In a specific embodiment, the stable formulation has a ionic strength about 60 mM. In a specific embodiment, the stable formulation has a ionic strength about 65 mM. In a specific embodiment, the stable formulation has a ionic strength about 70 mM. In a specific embodiment, the stable formulation has a ionic strength about 75 mM. In a specific embodiment, the stable formulation has a ionic strength about 80 mM. In a specific embodiment, the stable formulation has a ionic strength about 85 mM. In a specific embodiment, the stable formulation has a ionic strength about 90 mM. According ot the invention, the rAAV particles in the formulation have AAV8 capsids.

In certain embodiments, the stable formulation has a ionic strength about 30 mM to 100 mM. In a specific embodiment, the stable formulation has a ionic strength about 30 mM. In a specific embodiment, the stable formulation has a ionic strength about 35 mM. In a specific embodiment, the stable formulation has a ionic strength about 40 mM. In a specific embodiment, the stable formulation has a ionic strength about 45 mM. In a specific embodiment, the stable formulation has a ionic strength about 50 mM. In a specific embodiment, the stable formulation has a ionic strength about 55 mM. In a specific embodiment, the stable formulation has a ionic strength about 60 mM. In a specific embodiment, the stable formulation has a ionic strength about 65 mM. In a specific embodiment, the stable formulation has a ionic strength about 70 mM. In a specific embodiment, the stable formulation has a ionic strength about 75 mM. In a specific embodiment, the stable formulation has a ionic strength about 80 mM. In a specific embodiment, the stable formulation has a ionic strength about 85 mM. In a specific embodiment, the stable formulation has a ionic strength about 90 mM. In a specific embodiment, the stable formulation has a ionic strength about 95 mM. In a specific embodiment, the stable formulation has a ionic strength about 100 mM. In some examples, the rAAV particles in the formulation have AAV9 capsids.

In certain embodiments, the stable formulation has a ionic strength about 60 mM to 150 mM. In certain embodiments, the stable formulation has a ionic strength about 60 mM to 140 mM. In certain embodiments, the stable formulation has a ionic strength about 60 mM to 130 mM. In certain embodiments, the stable formulation has a ionic strength about 60 mM to 120 mM. In certain embodiments, the stable formulation has a ionic strength about 60 mM to 115 mM. In certain embodiments, the stable formulation has a ionic strength about 65 mM to 95 mM. In certain embodiments, the stable formulation has a ionic strength about 70 mM to 90 mM. In certain embodiments, the stable formulation has a ionic strength about 75 mM to 85 mM. According to the invention, the rAAV particles in the formulation have AAV8 capsids.

In certain embodiments, the stable formulation has a ionic strength about 30 mM to 100 mM. In certain embodiments, the stable formulation has a ionic strength about 35 mM to 95 mM. In certain embodiments, the stable formulation has a ionic strength about 40 mM to 90 mM. In certain embodiments, the stable formulation has a ionic strength about 45 mM to 85 mM. In certain embodiments, the stable formulation has a ionic strength about 50 mM to 80 mM. In certain embodiments, the stable formulation has a ionic strength about 55 mM to 75 mM. In certain embodiments, the stable formulation has a ionic strength about 60 mM to 70 mM. In some examples, the rAAV particles in the formulation have AAV9 capsids.

In certain embodiments, the pH of the stable formulation is about 7.4.

In certain embodiments, the pH of the stable formulation is about 6.0 to 8.8. In certain embodiments, the pH of the stable formulation is about 6.0 to 9.0. In certain embodiments, the pH of the stable formulation is about 6.0. In certain embodiments, the pH of the stable formulation is about 6.1. In certain embodiments, the pH of the stable formulation is about 6.2. In certain embodiments, the pH of the stable formulation is about 6.3. In certain embodiments, the pH of the stable formulation is about 6.4. In certain embodiments, the pH of the stable formulation is about 6.5. In certain embodiments, the pH of the stable formulation is about 6.6. In certain embodiments, the pH of the stable formulation is about 6.7. In certain embodiments, the pH of the stable formulation is about 6.8. In certain embodiments, the pH of the stable formulation is about 6.9. In certain embodiments, the pH of the stable formulation is about 7.0. In certain embodiments, the pH of the stable formulation is about 7.1. In certain embodiments, the pH of the stable formulation is about 7.2. In certain embodiments, the pH of the stable formulation is about 7.3. In certain embodiments, the pH of the stable formulation is about 7.4. In certain embodiments, the pH of the stable formulation is about 7.5. In certain embodiments, the pH of the stable formulation is about 7.6. In certain embodiments, the pH of the stable formulation is about 7.7. In certain embodiments, the pH of the stable formulation is about 7.8. In certain embodiments, the pH of the stable formulation is about 7.9. In certain embodiments, the pH of the stable formulation is about 8.0. In certain embodiments, the pH of the stable formulation is about 8.1. In certain embodiments, the pH of the stable formulation is about 8.2. In certain embodiments, the pH of the stable formulation is about 8.3. In certain embodiments, the pH of the stable formulation is about 8.4. In certain embodiments, the pH of the stable formulation is about 8.5. In certain embodiments, the pH of the stable formulation is about 8.6. In certain embodiments, the pH of the stable formulation is about 8.7. In certain embodiments, the pH of the stable formulation is about 8.8. In certain embodiments, the pH of the stable formulation is about 8.9. In certain embodiments, the pH of the stable formulation is about 9.0.

In certain embodiments, the vector genome concentration (VGC) of the stable formulation is about $3 \times 10^9$

GC/mL, $4 \times 10^9$ GC/mL, $5 \times 10^9$ GC/mL, $6 \times 10^9$ GC/mL, $7 \times 10^9$ GC/mL, $8 \times 10^9$ GC/mL, $9 \times 10^9$ GC/mL, about $1 \times 10^{10}$ GC/mL, about $2 \times 10^{10}$ GC/mL, about $3 \times 10^{10}$ GC/mL, about $4 \times 10^{10}$ GC/mL, about $5 \times 10^{10}$ GC/mL, about $6 \times 10^{10}$ GC/mL, about $7 \times 10^{10}$ GC/mL, about $8 \times 10^{10}$ GC/mL, about $9 \times 10^{10}$ GC/mL, about $1 \times 10^{11}$ GC/mL, about $2 \times 10^{11}$ GC/mL, about $3 \times 10^{11}$ GC/mL, about $4 \times 10^{11}$ GC/mL, about $5 \times 10^{11}$ GC/mL, about $6 \times 10^{11}$ GC/mL, about $7 \times 10^{11}$ GC/mL, about $8 \times 10^{11}$ GC/mL, about $9 \times 10^{11}$ GC/mL, about $1 \times 10^{12}$ GC/mL, about $2 \times 10^{12}$ GC/mL, about $3 \times 10^{12}$ GC/mL, about $4 \times 10^{12}$ GC/mL, about $5 \times 10^{12}$ GC/mL, about $6 \times 10^{12}$ GC/mL, about $7 \times 10^{12}$ GC/mL, about $8 \times 10^{12}$ GC/mL, about $9 \times 10^{12}$ GC/mL, about $1 \times 10^{13}$ GC/mL, about $1 \times 10^{13}$ GC/mL, about $2 \times 10^{13}$ GC/mL, or about $3 \times 10^{13}$ GC/mL.

In certain embodiments, the recombinant adeno-associated virus (rAAV) particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 more stable to lyophilization or reconstitution process, than compared to the same rAAV particles in a reference formulation. In certain embodiments, the stability of the rAAV particles is determined by an assay or assays disclosed in Section 6.8

In certain embodiments, the rAAV particles in the stable formulation has at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 more infectivity, than compared to the same rAAV particles in a reference formulation. In certain embodiments, the virus infectivity of the rAAV particles is determined by an assay or assays disclosed in Section 6.8. In certain embodiments, the infectivity is measured prior to or after lyophilization. In certain embodiments, the infectivity is measured prior to or after reconstitution of the lyophilized formulation.

In certain embodiments, the rAAV particles in the stable formulation has at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times less aggregation, than compared to the same rAAV particles in a reference formulation. In certain embodiments, the aggregation of the rAAV particles is determined by an assay or assays disclosed in Section 6.8. In certain embodiments, the aggregation is measured prior to or after lyophilization. In certain embodiments, the aggregation is measured prior to or after reconstitution of the lyophilized formulation.

In certain embodiments, the stable formulation is lyophilized prior to storing.

In certain embodiment, the stable formulation reconstituted after storing.

In certain embodiments, the formulation is stored at -80°C, -70°C, -20°C, 4°C, 20°C, 25°C, 30°C, 35°C, 37°C or 40 °C.

In certain embodiments, the formulation is stored about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, or about 4 years,

In certain embodiments, the recombinant adeno-associated virus (rAAV) particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times more stable to lyophilization or reconstitution process, than compared to the same rAAV particles in a reference formulation. In certain embodiments, the stability of the rAAV particles is determined by an assay or assays disclosed in Section 6.8.

In certain embodiments, the rAAV particles in the stable formulation has at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times more infectivity, than compared to the same rAAV particles in a reference formulation. In certain embodiments, the virus infectivity of the rAAV particles is determined by an assay or assays disclosed in Section 6.8. In certain embodiments, the infectivity is measured prior to or after lyophilization. In certain embodiments, the infectivity is measured prior to or after reconstitution of the lyophilized formulation.

In certain embodiments, the rAAV particles in the stable formulation has at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times less aggregation, than compared to the same rAAV particles in a reference formulation. In certain embodiments, the aggregation of the rAAV particles is determined by an assay or assays disclosed in Section 6.8. In certain embodiments, the aggregation is measured prior to or after lyophilization. In certain embodiments, the aggregation is measured prior to or after reconstitution of the lyophilized formulation.

In certain embodiments, the stable formulation is lyophilized prior to storing.

In certain embodiment, the stable formulation reconstituted after storing.

In certain embodiments, the formulation is stored at -80°C, -70°C, -20°C, 4°C, 20°C, 25°C, 30°C, 35°C, 37°C or 40 °C.

In certain embodiments, the formulation is stored about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, about 4 years, or about 5 years. In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 more stable after storing the formulation over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, about 4 years or about 5 years, than compared to the same rAAV particles in a reference formulation stored under the same condition. In certain embodiments, the stability over a period of time of the rAAV particles is determined by an assay or assays disclosed in Section 6.8.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 more stable after storing the formulation over a period of time, at least for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, about 4 years, or about 5 years, than compared to the same rAAV particles in a reference formulation. In certain embodiments, the stability over a period of time of the rAAV particles is determined by an assay or assays disclosed in Section 6.8.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times higher in vitro relative potency (IVRP), than compared to the same rAAV particles in a reference formulation. In certain embodiments, the in vitro relative potency (IVRP) of the rAAV particles is determined by an assay or assays disclosed in Section 6.8. In certain embodiments, the IVRP is measured prior to or after lyophilization. In certain embodiments, the IVRP is measured prior to or after reconstitution of the lyophilized formulation.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times less free DNA, than compared to the same rAAV particles in a reference formulation. In certain embodiments, the free DNA of the rAAV particles is determined by an assay or assays disclosed in Section 6.8. In certain embodiments, the free DNA is measured prior to or after lyophilization. In certain embodiments, the free DNA is measured prior to or after reconstitution of the lyophilized formulation.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times less rAAV genome release, than compared to the same rAAV particles in a reference formulation. In certain embodiments, the rAAV genome release is determined by measuring relative fluorescence in the presence of a DNA specific fluorescent stain. In certain embodiments, the rAAV genome release is determined by an assay or assays disclosed in Section 6.8. In certain embodiments, the rAAV genome release is measured prior to or after lyophilization. In certain embodiments, the rAAV genome release is measured prior to or after reconstitution of the lyophilized formulation.

In certain embodiments, the rAAV particles in the stable formulation has at most about 20%, about 15%, about 10%, about 8%, about 5%, about 4%, about 3%, about 2%, or about 1% change in size after storing the formulation over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the size of the rAAV particles is determined by an assay or assays disclosed in Section 6.8. In certain embodiments, the size is measured prior to or after freeze/thaw cycles.

In certain embodiments, the rAAV particles in the stable formulation has at most 20%, 15%, 10%, 8%, 5%, 4%, 3%, 2%, or 1% change in size after storing the formulation over a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the size of the rAAV particles is determined by an assay or assays disclosed in Section 6.8. In certain embodiments, the size is measured prior to or after lyophilization. In certain embodiments, the size is measured prior to or after reconstitution of the lyophilized formulation.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times more stable, than compared to the same rAAV particles in a reference formulation when stored at -80°C, -70°C, -20°C, 4°C, 20°C, 25°C, 30°C, 35°C, 37°C or 40 °C. In certain embodiments, the stability of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more infectivity, than compared to the same rAAV particles in a reference formulation when stored at -80°C, - 70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the virus infectivity of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more infectivity, than compared to the same rAAV particles in a reference formulation when stored at -80°C, - 70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the virus infectivity of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less aggregation, than compared to the same rAAV particles in a reference formulation when stored at -80°C, - 70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the aggregation of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less aggregation, than compared to the same rAAV particles in a reference formulation when stored at -80°C, - 70°C, -20°C, 4°C, 20°C, 25°C, 30°C, 35°C, 37°C or 40 °C for a period of time, at least for example, at least about 1 week, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the aggregation of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more stable, than compared to the same rAAV particles in a reference formulation when stored at -80°C, -70°C, - 20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the stability over a period of time of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more stable, than compared to the same rAAV particles in a reference formulation when stored at -80°C, -70°C, - 20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the stability over a period of time of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years, than compared to the same rAAV particles in a reference formulation. In certain embodiments, the in vitro relative potency (IVRP) of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less free DNA, than compared to the same rAAV particles in a reference formulation when stored at -80°C, - 70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the free DNA of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less free DNA, than compared to the same rAAV particles in a reference formulation when stored at -80°C, - 70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the free DNA of the rAAV particles is determined by an assay or assays disclosed in in Section 6.8.

In certain embodiments, the rAAV particles in the stable formulation has at most 20%, 15%, 10%, 8%, 5%, 4%, 3%, 2%, or 1% change in particle size when stored at -80°C, -70°C, -20°C, 4°C, 20°C, 25°C, 30°C, 35°C, 37°C or 40 °C over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the size of the rAAV particles is determined by an assay or assays disclosed in Section 6.8.

In certain embodiments, the rAAV particles in the stable formulation has at most 20%, 15%, 10%, 8%, 5%, 4%, 3%, 2%, or 1% change in particle size when stored at -80°C, -70°C, -20°C, 4°C, 20°C, 25°C, 30°C, 35°C, 37°C or 40 °C over a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years, when compared to the same rAAV particles in a reference formulation. In certain embodiments, the size of the rAAV particles is determined by an assay or assays disclosed in Section 6.8.

In certain embodiments, the size of the rAAV particles is determined by an assay or assays disclosed in Section 6.8.

In certain embodiments, the reference formulation is DPBS with 0.001% poloxamer 188 buffer.

In certain embodiments, the reference formulation is a formulation not comprising sugar. In certain embodiments, the reference formulation is a formulation not comprising plasticizer.

In certain embodiments, the formulation is frozen to a temperature of about - 20 °C in the process of lyophilization. In certain embodiments, the frozen formulation maintains pH between about pH 6 to about pH 9 when freezing down to -20 °C. In certain embodiments, the frozen formulation maintains a pH value within a range of plus or minus 1 unit of the pH value prior to freezing when freezing down to -20 °C.

In certain embodiments, the glass transition temperature (Tg) of the lyophilized cakes of the formulation is higher than 35 °C.

In certain embodiments, the glass transition temperature of the maximally freeze-concentrated solution (Tg') of the formulation is higher than -40 °C.

In certain embodiments, the moisture content is between about 0.5% and about 1%. In certain embodiments, the moisture content is between about 1% and about 2%. In certain embodiments, the moisture content is between about 2% and about 3%. In certain embodiments, the moisture content is between about 3% and about 4%. In certain embodiments, the moisture content is between about 4% and about 5%.

In certain embodiments, the moisture content is about 0.1%, about 0.2%, about 0.3%, about 0.4%, about 0.5%, about 0.6%, about 0.8%, about 1.0%, about 1.2%, about 1.2%, about 1.3%, about 1.4%, about 1.5%, about 1.6%, about 1.7%, about 1.8%, about 1.9%, about 2.0%, about 3.0%, about 4.0%, or about 5.0%.

In some embodiments, a formulation disclosed herein comprises a plasticizer or a stabilizer. Pharmaceutically acceptable stabilizers or plasticizers include, but are not limited to, glycerol, xylitol, and sorbitol. In some embodiments, a formulation disclosed herein comprises glycerol, xylitol, sorbitol, or a combination thereof. In some embodiments, a formulation disclosed herein comprises glycerol.

In some embodiments, a formulation disclosed herein comprises between about 0.1% and between about 5% plasticizer or a stabilizer. In some embodiments, a formulation disclosed herein comprises between about 0.1% and between about 2% plasticizer or a stabilizer. In some embodiments, a formulation disclosed herein comprises between about 0.25% and between about 2% plasticizer.

In some embodiments, a formulation disclosed herein comprises between about 0.1% and between about 5% glycerol. In some embodiments, a formulation disclosed herein comprises between about 0.1% and between about 2% glycerol. In some embodiments, a formulation disclosed herein comprises between about 0.25% and between about 2% glycerol.

In some embodiments, the term "about" means within plus or minus 10% of a given value or range. In some embodiments, the term "about" refers to ranges of approximately 10-20% greater than or less than the indicated number or range. In certain embodiments, the term "about" encompasses the exact number recited.

Still other features and advantages of the compositions and methods described herein will become more apparent from the following detailed description when read in conjunction with the accompanying drawings.

### 4.1 ILLUSTRATIVE EMBODIMENTS

Reference to a rAAV particle in the context of the illustrative embodiments means a rAAV particle comprising an AAV8 capsid protein. Reference to rAAV particle comprising an capsid protein other than AAV8 is for information only.

### 4.1.1 Set 1

1. A stable formulation comprising recombinant adeno-associated virus (rAAV) particles and

    a) a buffering agent Tris,
    b) a sugar, and

c) an amorphous salt comprising sodium citrate.

2. The formulation of paragraph 1, wherein the buffering agent comprises between about 1 mM and about 50 mM Tris.

3. The formulation of paragraph 2 comprising between about 1 mM and about 30 mM, between about 1 mM and about 20 mM, between about 5 mM and about 30 mM, between about 5 mM and about 20 mM, between about 10 mM and about 30 mM, between about 10 mM and about 20 mM, or between about 20 mM and about 50 mM Tris.

4. The formulation of paragraph 2 comprising about 1 mM, about 2 mM, about 3 mM, about 5 mM, about 10 mM, about 15 mM, about 20 mM, about 25 mM, about 30 mM, or about 40 mM Tris.

5. The formulation of paragraph 2 comprising about 5 mM Tris.

6. The formulation of any one of paragraphs 1 to 4 having a pH of between about 6.5 and 8.0.

7. The formulation of paragraph 6 having a pH of between about 7.2 and 7.8.

8. The formulation of paragraph 6 having a pH of about 7.2, 7.3, 7.4, 7.5, 7.6, 7.7, or 7.8.

9. The formulation of paragraph 6 having a pH of about 7.5.

10. The formulation of any one of paragraphs 1 to 9 comprising between about 50 mM and about 400 mM sugar.

11. The formulation of paragraph 10 comprising between about 50 mM and about 350 mM, between about 50 mM and about 300 mM, between about 50 mM and about 250 mM, between about 50 mM and about 200 mM, or between about 50 mM and about 150 mM sugar.

12. The formulation of paragraph 10 comprising between about 100 mM and about 400 mM, between about 150 mM and about 400 mM, between about 200 mM and about 400 mM, between about 250 mM and about 400 mM, or between about 300 mM and about 400 mM sugar.

13. The formulation of paragraph 10 comprising between about 100 mM and about 300 mM, between about 150 mM and about 250 mM, between about 200 mM and about 300 mM, or between about 250 mM and about 350 mM sugar.

14. The formulation of paragraph 10 comprising about 50 mM, about 100 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, or about 350 mM sugar.

15. The formulation of paragraph 10 comprising between about 190 mM and about 230 mM, between about 170 mM and about 250 mM, or between about 150 mM and about 270 mM sugar.

16. The formulation of paragraph 10 comprising about 210 mM sugar.

17. The formulation of any one of paragraphs 1 to 16, wherein the sugar is a non-reducing sugar.

18. The formulation of paragraph 17, wherein the non-reducing sugar is sucrose, trehalose, or raffinose.

19. The formulation of paragraph 17, wherein the non-reducing sugar is sucrose.

20. The formulation of any one of paragraphs 1 to 16, wherein the sugar is a reducing sugar.

21. The formulation of paragraph 20, wherein the reducing sugar is glucose, fructose, mannose, galactose, or lactose.

22. The formulation of paragraph 20, wherein the reducing sugar is dextrose.

23. The formulation of any one of paragraphs 1 to 22 comprising less than about 100 mM sodium citrate.

24. The formulation of paragraph 23 comprising between about 10 mM and about 100 mM sodium citrate.

25. The formulation of paragraph 23 comprising between about 10 mM and about 100 mM, between about 20 mM and about 100 mM, between about 30 mM and about 100 mM, between about 40 mM and about 100 mM, between about 50 mM and about 100 mM, between about 10 mM and about 80 mM, between about 10 mM and about 60 mM, between about 10 mM and about 50 mM, between about 10 mM and about 40 mM, or between about 10 mM and about 30 mM sodium citrate.

26. The formulation of paragraph 23 comprising between about 10 mM and about 50 mM, between about 20 mM and about 60 mM, between about 30 mM and about 70 mM, or between about 10 mM and about 30 mM sodium citrate.

27. The formulation of paragraph 23 comprising about 10 mM, about 20 mM, about 30 mM, about 40 mM, about 50 mM, or about 60 mM sodium citrate.

28. The formulation of paragraph 23 comprising about 20 mM sodium citrate.

29. The formulation of any one of paragraphs 1 to 28, further comprising between about 0.0005% and about 0.01% nonionic surfactant.

30. The formulation of paragraph 29, comprising about 0.002% nonionic surfactant.

31. The formulation of paragraph 28 or paragraph 29, wherein the nonionic surfactant comprises poloxamer 188, poloxamer 407, polysorbate 80, polysorbate 20, Pluronic F-68, or BRIJ 35.

32. The formulation of paragraph 31, wherein the nonionic surfactant comprises poloxamer 188.

33. A stable formulation comprising recombinant adeno-associated virus (rAAV) particles and

a) between about 1 mM and about 25 mM Tris,
b) between about 50 mM and about 400 mM sugar,
c) between about 10 mM and about 100 mM sodium citrate, and
d) between about 0.0005% and about 0.01 % non-ionic surfactant,

wherein the formulation has a pH of between about 7.2 and about 7.8.

34. A stable formulation comprising recombinant adeno-associated virus (rAAV) particles and

a) between about 2 mM and about 10 mM Tris,
b) between about 150 mM and about 250 mM sugar,
c) between about 10 mM and about 20 mM sodium citrate, and
d) between about 0.001% and about 0.005 % non-ionic surfactant,

wherein the formulation has a pH of between about 7.2 and about 7.8.

35. A stable formulation comprising recombinant adeno-associated virus (rAAV) particles and

a) about 5 mM Tris,
b) about 210 mM sugar,
c) about 20 mM sodium citrate, and
d) about 0.002 % non-ionic surfactant,

wherein the formulation has a pH of about 7.5.

36. The formulation of any one of paragraphs 33 to 35, wherein the sugar is a non-reducing sugar.

37. The formulation of paragraph 36, wherein the non-reducing sugar is sucrose, trehalose, or raffinose.

38. The formulation of paragraph 17, wherein the non-reducing sugar is sucrose.

39. The formulation of any one of paragraphs 1 to 38 comprising between about 1.0E+11 genome copy/ mL (GC/mL) and about 1.0E+15 GC/mL rAAV particles.

40. The formulation of paragraph 39 comprising about 1.0E+11 GC/mL, about 1.0E+12 GC/mL, about 1.0E+13 GC/mL, about 1.0E+14 GC/mL, or about 1.0E+ 15 GC/mL rAAV particles.

41. The formulation of any one of paragraphs 1 to 40, wherein the rAAV particles comprise a capsid protein of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

42. The formulation of paragraph 41, wherein the rAAV particles comprise a capsid protein of the AAV-8 or AAV-9 serotype.

43. The formulation of any one of paragraphs 1 to 42 further comprising a plasticizer selected from the group consisting of glycerol, xylitol, sorbitol, or mannitol.

44. The formulation of any one of paragraphs 1 to 42 further comprising glycerol.

45. The formulation of paragraph 44, comprising between about 0.1% and between about 5% glycerol.

46. The formulation of paragraph 44, comprising between about 0.1% and between about 2% glycerol.

47. The formulation of paragraph 44, comprising between about 0.25% and between about 2% glycerol.

48. The formulation of any one of paragraphs 1 to 47 that is a liquid formulation.

49. The formulation of any one of paragraphs 1 to 47 that is a frozen formulation.

50. The formulation of any one of paragraphs 1 to 47 that is a lyophilized formulation or a reconstituted lyophilized formulation.

51. The formulation of paragraph 50 having a residual moisture content between about 1% and about 7%.

52. The formulation of paragraph 51, wherein the residual moisture content is between about 1% and about 7%, between about 2% and about 7%, between about 3% and about 7%, between about 4% and about 7%, between about 5% and about 7%, between about 1% and about 6%, between about 1% and about 5%, between about 1% and about 4%, or between about 1% and about 3%.

53. The formulation of paragraph 51, wherein the residual moisture content is between about 3% and about 7%, between about 3% and about 6%, or between about 3% and about 5%.

54. The formulation of paragraph 51, wherein the residual moisture content is about 3%, about 3.5%, about 4%, about 4.5%, about 5%, about 5.5%, or about 6%.

55. The formulation of any one of paragraphs 1 to 54, wherein the % relative potency of the rAAV particles is at least about 60%, at least about 70%, or at least about 80% after storing the formulation for 3 months at room temperature; wherein the reference rAAV particles are stored at -70°C in DPBS with 0.001% poloxamer 188 buffer.

56. The formulation of any one of paragraphs 1 to 54, wherein the % relative potency of the rAAV particles is at least about 60%, at least about 70%, or at least about 80% after storing the formulation for 6 months at room temperature; wherein the reference rAAV particles are stored at -70°C in Dulbecco's phosphate-buffered saline (DPBS) with 0.001% poloxamer 188 buffer.

57. The formulation of any one of paragraphs 1 to 54, wherein the % relative potency of the rAAV particles is at least about 30%, at least about 40%, at least about 50%, or at least 60% after storing the formulation for 1 week at 35°C, wherein the reference rAAV particles are stored at -70°C in DPBS with 0.001% poloxamer 188 buffer.

58. The formulation of any one of paragraphs 1 to 54, wherein the % relative potency of the rAAV particles is at least about 30%, at least about 40%, at least about 50%, or at least 60% after storing the formulation for 2 weeks at 35°C, wherein the reference rAAV particles are stored at -70°C in DPBS with 0.001% poloxamer 188 buffer.

59. The formulation of any one of paragraphs 1 to 54, wherein the % relative potency of the rAAV particles is at least about 30%, at least about 40%, at least about 50%, or at least 60% after storing the formulation for 4 weeks at 35°C, wherein the reference rAAV particles are stored at -70°C in DPBS with 0.001% poloxamer 188 buffer.

60. A method of producing a stable formulation comprising recombinant adeno-associated virus (rAAV) particles, comprising combining rAAV particles with a buffering agent, a sugar, a salt, optionally a plasticizer, and optionally a nonionic surfactant of the formulation according to any one of paragraphs 1 to 47, thereby producing the formulation comprising rAAV.

61. A method of reducing rAAV genome release from rAAV particles, comprising producing a formulation comprising rAAV particles, a buffering agent, a sugar, a salt, and optionally a nonionic surfactant , wherein rAAV genome release from the rAAV particles after three freeze-thaw cycles is reduced compared to rAAV genome release in a formulation not comprising the sugar.

62. A method of reducing rAAV genome release from rAAV particles, comprising producing a formulation comprising rAAV particles, a buffering agent, a sugar, a salt, and optionally a nonionic surfactant, wherein rAAV genome release from the rAAV particles after lyophilization and reconstitution is reduced compared to rAAV genome release in a formulation not comprising the sugar.

63. The method of paragraph 61 or 62, further comprising lyophilizing the formulation to achieve a residual moisture content between about 1% and about 5%.

64. A method of reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar.

65. A method of reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar.

66. Use of a sugar for reducing rAAV genome release from rAAV particles, comprising producing a formulation comprising rAAV particles, a buffering agent, a sugar, a salt, and optionally a nonionic surfactant, wherein rAAV genome release from the rAAV particles after three freeze-thaw cycles is reduced compared to rAAV genome release in a formulation not comprising the sugar.

67. Use of a sugar for reducing rAAV genome release from rAAV particles, comprising producing a formulation comprising rAAV particles, a buffering agent, a sugar, a salt, and optionally a nonionic surfactant, wherein rAAV genome release from the rAAV particles after lyophilization and reconstitution is reduced compared to rAAV genome release in a formulation not comprising the sugar.

68. The use of paragraph 66 or 67, further comprising lyophilizing the formulation to achieve a residual moisture content between about 1% and about 7%.

69. Use of a plasticizer for reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar.

70. Use of a plasticizer for reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar.

71. The method of any one of paragraphs 61-65 or the use of any one of paragraphs 66-70, wherein rAAV genome release is determined by measuring relative fluorescence in the presence of a DNA specific fluorescent stain.

72. The method of any one of paragraphs 61-65 and 71 or the use of any one of paragraphs 66-71, wherein freezing-induced rAAV genome release is reduced by at least about 10%, 20%, 50%, 80%, or 90%.

73. The method of any one of paragraphs 61-65 and 71 or the use of any one of paragraphs 66-71, wherein freezing-induced rAAV genome release is substantially eliminated.

74. The method of any one of paragraphs 61-65 and 71-73 or the use of any one of paragraphs 66-73, wherein the sugar is a non-reducing sugar.

75. The method of paragraph 74 or the use of paragraph 74, wherein the non-reducing sugar is sucrose, trehalose, or raffinose.

76. The method of paragraph 74 or the use of paragraph 74, wherein the non-reducing sugar is sucrose.

77. The method of any one of paragraphs 61-65 and 71-73 or the use of any one of paragraphs 66-73, wherein the sugar is a reducing sugar.

78. The method of paragraph 77 or the use of paragraph 77, wherein the reducing sugar is glucose, fructose, mannose, galactose, or lactose.

79. The method of paragraph 77 or the use of paragraph 77, wherein the reducing sugar is dextrose.

80. The method of any one of paragraphs 61-65 and 71-79 or the use of any one of paragraphs 66-79, wherein the plasticizer comprises glycerol.

81. The method of any one of paragraphs 61-65 and 71-80 or the use of any one of paragraphs 66-80, wherein the formulation is according to any one of paragraphs 1 to 47.

**4.1.2 Set 2**

1. A stable formulation comprising recombinant adeno-associated virus (rAAV) particles and

    a) a buffering agent Tris,
    b) a sugar, and
    c) an amorphous salt,

wherein the formulation is suitable for lyophilization.

2. The formulation of paragraph 1, wherein the buffering agent comprises between about 1 mM and about 50 mM Tris.

3. The formulation of paragraph 2 comprising between about 1 mM and about 30 mM, between about 1 mM and about 20 mM, between about 5 mM and about 30 mM, between about 5 mM and about 20 mM, between about 10 mM and about 30 mM, between about 10 mM and about 20 mM, or between about 20 mM and about 50 mM Tris.

4. The formulation of paragraph 2 comprising about 1 mM, about 2 mM, about 3 mM, about 5 mM, about 10 mM, about 15 mM, about 20 mM, about 25 mM, about 30 mM, or about 40 mM Tris.

5. The formulation of paragraph 2 comprising about 5 mM Tris.

6. A stable formulation comprising recombinant adeno-associated virus (rAAV) particles and

    a) a buffering agent Tris,
    b) a sugar, and

c) an amorphous salt having an ionic strength higher than 60 mM,

wherein the formulation is suitable for lyophilization.

7. The formulation of paragraph 6, wherein the rAAV particles have AAV8 capsids.

8. A stable formulation comprising recombinant adeno-associated virus (rAAV) particles and

a) a buffering agent Tris,
b) a sugar, and

an amorphous salt having an ionic strength between 60mM and 150 mM, wherein the formulation is suitable for lyophilization.

9. The formulation of paragraph 8, wherein the rAAV particles have AAV8 capsids.

10. A stable formulation comprising recombinant adeno-associated virus (rAAV) particles and

a) a buffering agent Tris,
b) a sugar, and
c) an amorphous salt having an ionic strength between 30-100 mM,

wherein the formulation is suitable for lyophilization.

11. The formulation of paragraph 10, wherein the rAAV particles have rAAV9 capsids.

12. A stable formulation comprising recombinant adeno-associated virus (rAAV) particles and

a) a buffering agent,
b) a sugar, and
c) an amorphous salt having an ionic strength higher than 200mM,

wherein the formulation is suitable for lyophilization.

13. The formulation of paragraph 12, wherein the rAAV particles do not have rAAV8 or rAAV9 capsids.

14. The formulation of any one of paragraphs 1 to 13, wherein the amorphous salt is sodium citrate.

15. The formulation of any one of paragraphs 1 to 13, wherein the amorphous salt is sodium sulfate.

16. The formulation of any one of paragraphs 1 to 13, wherein the amorphous salt is ammonium sulfate.

17. The formulation of any one of paragraphs 1 to 13, wherein the amorphous salt is magnesium sulfate.

18. The formulation of any one of paragraphs 1 to 13, wherein the amorphous salt is sodium citrate, sodium sulfate, ammonium sulfate, magnesium sulfate, or a combination thereof.

19. The formulation of any one of paragraphs 1 to 13 comprising sodium citrate.

20. The formulation of any one of paragraphs 1 to 13 comprising sodium sulfate.

21. The formulation of any one of paragraphs 1 to 13 comprising ammonium sulfate.

22. The formulation of any one of paragraphs 1 to 13 comprising magnesium sulfate.

23. The formulation of any one of paragraphs 1 to 13 comprising sodium citrate, sodium sulfate, ammonium sulfate, magnesium sulfate, or a combination thereof.

24. The formulation of any one of paragraphs 1 to 13, wherein the formulation comprises about 10 mM, about 20 mM, about 30 mM, about 40 mM, about 50 mM, about 60 mM, about 70 mM, about 80 mM, about 90 mM, about 100 mM, about 120 mM, about 140 mM, about 150 mM, or about 200 mM sodium sulfate.

25. The formulation of any one of paragraphs 1 to 24 having a pH of between about 6.5 and 8.0.

26. The formulation of paragraph 25 having a pH of between about 7.2 and 7.8.

27. The formulation of paragraph 25 having a pH of about 7.2, 7.3, 7.4, 7.5, 7.6, 7.7, or 7.8.

28. The formulation of paragraph 25 having a pH of about 7.5.

29. The formulation of any one of paragraphs 1 to 28 comprising between about 50 mM and about 400 mM sugar.

30. The formulation of paragraph 29 comprising between about 50 mM and about 350 mM, between about 50 mM and about 300 mM, between about 50 mM and about 250 mM, between about 50 mM and about 200 mM, or between about 50 mM and about 150 mM sugar.

31. The formulation of paragraph 29 comprising between about 100 mM and about 400 mM, between about 150 mM and about 400 mM, between about 200 mM and about 400 mM, between about 250 mM and about 400 mM, or between about 300 mM and about 400 mM sugar.

32. The formulation of paragraph 29 comprising between about 100 mM and about 300 mM, between about 150 mM and about 250 mM, between about 200 mM and about 300 mM, or between about 250 mM and about 350 mM sugar.

33. The formulation of paragraph 29 comprising about 50 mM, about 100 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, or about 350 mM sugar.

34. The formulation of paragraph 29 comprising between about 190 mM and about 230 mM, between about 170 mM and about 250 mM, or between about 150 mM and about 270 mM sugar.

35. The formulation of paragraph 29 comprising about 210 mM sugar.

36. The formulation of any one of paragraphs 1 to 35, wherein the sugar is a non-reducing sugar.

37. The formulation of paragraph 36, wherein the non-reducing sugar is sucrose, trehalose, or raffinose.

38. The formulation of paragraph 36, wherein the non-reducing sugar is sucrose.

39. The formulation of any one of paragraphs 1 to 35, wherein the sugar is a reducing sugar.

40. The formulation of paragraph 39, wherein the reducing sugar is glucose, fructose, mannose, galactose, or lactose.

41. The formulation of any one of paragraphs 1 to 40 comprising less than about 100 mM sodium citrate.

42. The formulation of paragraph 41 comprising between about 10 mM and about 100 mM sodium citrate.

43. The formulation of paragraph 41 comprising between about 10 mM and about 100 mM, between about 20 mM and about 100 mM, between about 30 mM and about 100 mM, between about 40 mM and about 100 mM, between about 50 mM and about 100 mM, between about 10 mM and about 80 mM, between about 10 mM and about 60 mM, between about 10 mM and about 50 mM, between about 10 mM and about 40 mM, or between about 10 mM and about 30 mM sodium citrate.

44. The formulation of paragraph 41 comprising between about 10 mM and about 50 mM, between about 20 mM and about 60 mM, between about 30 mM and about 70 mM, or between about 10 mM and about 30 mM sodium citrate.

45. The formulation of paragraph 41 comprising about 10 mM, about 20 mM, about 30 mM, about 40 mM, about 50 mM, or about 60 mM sodium citrate.

46. The formulation of paragraph 41 comprising about 20 mM sodium citrate.

47. The formulation of any one of paragraphs 1 to 46, further comprising between about 0.0005% and about 0.01% nonionic surfactant.

48. The formulation of paragraph 47, comprising about 0.002% nonionic surfactant.

49. The formulation of paragraph 46 or paragraph 47, wherein the nonionic surfactant comprises poloxamer 188, poloxamer 407, polysorbate 80, polysorbate 20, Pluronic F-68, or BRIJ 35.

50. The formulation of paragraph 49, wherein the nonionic surfactant comprises poloxamer 188.

51. A stable formulation comprising recombinant adeno-associated virus (rAAV) particles and

   a) between about 1 mM and about 25 mM Tris,
   b) between about 50 mM and about 400 mM sugar,
   c) between about 10 mM and about 100 mM sodium citrate, and
   d) between about 0.0005% and about 0.01 % non-ionic surfactant,

wherein the formulation has a pH of between about 7.2 and about 7.8.

52. A stable formulation comprising recombinant adeno-associated virus (rAAV) particles and

   a) between about 2 mM and about 10 mM Tris,
   b) between about 150 mM and about 250 mM sugar,
   c) between about 10 mM and about 20 mM sodium citrate, and
   d) between about 0.001% and about 0.005 % non-ionic surfactant,

wherein the formulation has a pH of between about 7.2 and about 7.8.

53. A stable formulation comprising recombinant adeno-associated virus (rAAV) particles and

   a) about 5 mM Tris,
   b) about 210 mM sugar,
   c) about 20 mM sodium citrate, and
   d) about 0.002 % non-ionic surfactant,

wherein the formulation has a pH of about 7.5.

54. The formulation of any one of paragraphs 51 to 53, wherein the sugar is a non-reducing sugar.

55. The formulation of paragraph 54, wherein the non-reducing sugar is sucrose, trehalose, or raffinose.

56. The formulation of paragraph 55, wherein the non-reducing sugar is sucrose.

57. A stable formulation comprising recombinant adeno-associated virus (rAAV) particles and

   a) about 5 mM Tris,
   b) about 210 mM sucrose,
   c) about 20 mM sodium citrate, and
   d) about 0.002 % (w/v) poloxamer 188.

58. A stable formulation comprising recombinant adeno-associated virus (rAAV) particles and

   a) about 5 mM Tris,
   b) about 210 mM sucrose,

c) about 20 mM sodium citrate,
d) about 0.002 % (w/v) poloxamer 188, and
e) about 0.25% (w/v) glycerol.

59. A stable formulation comprising recombinant adeno-associated virus (rAAV) particles and

a) about 5 mM Tris,
b) about 210 mM sucrose,
c) about 20 mM sodium citrate,
d) about 0.002 % (w/v) poloxamer 188, and
e) about 0.5% (w/v) sorbitol.

60. A stable formulation comprising recombinant adeno-associated virus (rAAV) particles and

a) about 5 mM Tris,
b) about 30 mM sodium sulfate,
c) about 263 mM sucrose, and
d) about 0.005 % (w/v) poloxamer 188.

61. The formulation of any one of paragraphs 1-60, wherein the formulation is suitable for lyophilization.

62. The formulation of any one of paragraphs 1-61, wherein the formulation has a pH of about 7.5.

63. The formulation of any one of paragraphs 1-61, wherein the formulation has a pH of about 7.2, 7.3, 7.4, 7.5, 7.6, 7.7, or 7.8.

64. The formulation of any one of paragraphs 1-61, wherein the formulation has a pH of about 7.1.

65. The formulation of any one of paragraphs 1-61, wherein the formulation has a pH of between about 6.5 and 8.0.

66. The formulation of any one of paragraphs 1-61, wherein the formulation is has a pH between about 7.2 and about 7.8.

67. The formulation of any one of paragraphs 1-66, wherein the formulation comprises between about 1.0E+11 genome copy/ mL (GC/mL) and about 1.0E+15 GC/mL rAAV particles.

68. The formulation of paragraph 67, wherein the formulation comprises about 1.0E+11 GC/mL, about 1.0E+12 GC/mL, about 1.0E+13 GC/mL, about 1.0E+14 GC/mL, or about 1.0E+15 GC/mL rAAV particles.

69. The formulation of any one of paragraphs 1-68, wherein the rAAV particles comprise a capsid protein of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

70. The formulation of paragraph 69, wherein the rAAV particles comprise a capsid protein of the AAV-8 or AAV-9 serotype.

71. The formulation of any one of paragraphs 1-70 further comprising a stabilizer selected from the group consisting of glycerol, or sorbitol.

72. The formulation of any one of paragraphs 1-70 further comprising glycerol.

73. The formulation of any one of paragraphs 1-70, comprising between about 0.1% and between about 5% glycerol.

74. The formulation of any one of paragraphs 1-70, comprising between about 0.1% and between about 2% glycerol.

75. The formulation of any one of paragraphs 1-70, comprising between about 0.25% and between about 2% glycerol.

76. The formulation of any one of paragraphs 1-70, the formulation does not comprise mannitol.

77. The formulation of any one of paragraphs 1-70, the formulation comprises less than 10 mM, 20 mM, 50mM, 100mM or 150mM mannitol.

78. The formulation of any one of paragraphs 1-77 that is a pre-lyophilization formulation.

79. The formulation of paragraph having a residual moisture content between about 1% and about 7%.

80. The formulation of paragraph 79, wherein the residual moisture content is between about 1% and about 7%, between about 2% and about 7%, between about 3% and about 7%, between about 4% and about 7%, between about 5% and about 7%, between about 1% and about 6%, between about 1% and about 5%, between about 1% and about 4%, or between about 1% and about 3%.

81. The formulation of paragraph 79 wherein the residual moisture content is between about 3% and about 7%, between about 3% and about 6%, or between about 3% and about 5%.

82. The formulation of paragraph 79, wherein the residual moisture content is about 3%, about 3.5%, about 4%, about 4.5%, about 5%, about 5.5%, or about 6%.

83. The formulation of paragraph 79, wherein the moisture content is between about 1% to about 2%.

84. The formulation of paragraph 79, wherein the moisture content is about 1.5%, about 1.4%, about 1.3%, about 1.2%, or about 1.1%.

85. The formulation of paragraph 79, wherein the moisture content is about 1%.

86. The formulation of any one of paragraphs 1 to 78, wherein the glass transition temperature (Tg) of the lyophilized cakes of the formulation is higher than 35°C.

87. The formulation of any one of paragraphs 1 to 78, wherein the glass transition temperature of the maximally freeze-concentrated solution (Tg') of the formulation is higher than -40 °C.

88. The formulation of any one of paragraphs 1 to 78, wherein the % relative potency of the rAAV particles is at least about 60%, at least about 70%, or at least about 80% after storing the formulation for 3 months at room temperature; wherein the reference rAAV particles are stored at -70°C in DPBS with 0.001% poloxamer 188 buffer.

89. The formulation of any one of paragraphs 1 to 78, wherein the % relative potency of the rAAV particles is at least about 60%, at least about 70%, or at least about 80% after storing the formulation for 6 months at room temperature; wherein the reference rAAV particles are stored at -70°C in Dulbecco's phosphate-buffered saline (DPBS) with 0.001% poloxamer 188 buffer.

90. The formulation of any one of paragraphs 1 to 78, wherein the % relative potency of the rAAV particles is at least about 30%, at least about 40%, at least about 50%, or at least 60% after storing the formulation for 1 week at 35°C, wherein the reference rAAV particles are stored at -70°C in DPBS with 0.001% poloxamer 188 buffer.

91. The formulation of any one of paragraphs 1 to 78, wherein the % relative potency of the rAAV particles is at least about 30%, at least about 40%, at least about 50%, or at least 60% after storing the formulation for 2 weeks at 35°C, wherein the reference rAAV particles are stored at -70°C in DPBS with 0.001% poloxamer 188 buffer.

92. The formulation of any one of paragraphs 1 to 78, wherein the % relative potency of the rAAV particles is at least about 30%, at least about 40%, at least about 50%, or at least 60% after storing the formulation for 4 weeks at 35°C, wherein the reference rAAV particles are stored at -70°C in DPBS with 0.001% poloxamer 188 buffer.

93. The formulation of any one of paragraphs 87 to 92, wherein the formulation is lyophilized prior storing.

94. The formulation of paragraph 94, wherein the lyophilized formulation is reconstituted after storing.

95. The formulation of any one of paragraphs 1 to 78, wherein the % relative potency of the rAAV particles is at least about 25%, at least about 30%, at least about 40%, at least about 50%, at least about 60%, at least about 70%, at least about 80%, at least about 90%, least about 95%, or at least about 99% right after lyophilization.

96. The formulation of any one of paragraphs 1 to 78, wherein the level of rAAV particle aggregation of the formulation is decreased about 10%, about 20%, about 30%, about 40%, about 50%, about 60%, about 70%, about 80%, about 90%, or about 100% as compared to the level of rAAV particle aggregation in a reference formulation.

97. The formulation of any one of paragraphs 1 to 78, wherein the stability of the formulation is assessed by vector genome content or viral titer assay, wherein the formulation has at least about 5%, about 10%, about 20%, about 30%, about 40%, about 50%, about 60%, about 70%, about 80%, about 90%, about 100%, or about 200%, more genome content after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the genome content of a reference formulation stored under the same condition.

98. The formulation of any one of paragraphs 1 to 78, wherein the stability of the formulation is assessed by measuring the relative potency of the formulation, wherein the formulation has at least about 5%, about 10%, about 20%, about 30%, about 40%, about 50%, about 60%, about 70%, about 80%, about 90%, about 100%, or about 200%, more relative potency after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the genome content of a reference formulation stored under the same condition.

99. The formulation of any one of paragraphs 1 to 78, wherein the stability of the formulation is assessed by loss of infectivity, wherein the formulation has at least about 5%, about 10%, about 20%, about 30%, about 40%, about 50%, about 60%, about 70%, about 80%, about 90%, about 100%, or about 200%, less infectivity loss after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the infectivity loss of a reference formulation stored under the same condition.

100. The formulation of any one of paragraphs 1 to 78, wherein the stability of the formulation is assessed by rAAV genome release, wherein the rAAV genome release is determined by measuring relative fluorescence in preference of a DNA specific florescent stain, and wherein the formulation has at least about 5%, about 10%, about 20%, about 30%, about 40%, about 50%, about 60%, about 70%, about 80%, about 90%, about 100%, or about 200%, less relative fluorescence level after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the relative fluorescence level of a reference formulation stored under the same condition.

101. The formulation of any one of paragraphs 97 to100, wherein the formulation is lyophilized prior to storing.

102. The formulation of paragraph 101, wherein the lyophilized formulation is reconstituted after storing.

103. The formulation of any one of paragraphs 97 to 102, wherein the formulation is stored at -80°C, -70°C, -20°C, 4°C, 20°C, 25°C, 30°C, 35°C, or 40 °C.

104. The formulation of any one of paragraphs 96 to 103, wherein the reference formulation is DPBS with 0.001% poloxamer 188 buffer.

105. The formulation of any one of paragraphs 96 to 104, wherein the reference formulation is a formulation not comprising sugar.

106. The formulation of any one of paragraphs 96 to 105, wherein the reference formulation is a formulation not comprising plasticizer.

107. The formulation of any one of paragraphs 1 to 106, wherein the formulation is frozen to a temperature of about - 20 °C in the process of lyophilization.

108. The formulation of any one of paragraphs 1 to 107, wherein the frozen formulation maintains pH between about pH 6 to about pH 9 when freezing down to -20 °C.

109. The formulation of any one of paragraphs 1 to 107, wherein the frozen formulation maintains a pH value within a range of plus or minus 1 unit of the pH value prior to freezing when freezing down to -20 °C.

110. The formulation of any one of paragraphs 1-109 is a stabilized aqueous formulation of rAAV for lyophilization.

111. A method of producing a stable formulation comprising recombinant adeno-associated virus (rAAV) particles, comprising combining rAAV particles with a buffering agent, a sugar, a salt, optionally a plasticizer, and optionally a nonionic surfactant of the formulation according to any one of paragraphs 1 to 108, thereby producing the formulation comprising rAAV.

112. A method of reducing rAAV genome release from rAAV particles, comprising producing a formulation comprising rAAV particles, a buffering agent, a sugar, a salt, and optionally a nonionic surfactant , wherein rAAV genome release from the rAAV particles after three freeze-thaw cycles is reduced compared to rAAV genome release in a formulation not comprising the sugar.

113. A method of reducing rAAV genome release from rAAV particles, comprising producing a formulation comprising rAAV particles, a buffering agent, a sugar, a salt, and optionally a nonionic surfactant, wherein rAAV genome release from the rAAV particles after lyophilization and reconstitution is reduced compared to rAAV genome release in a formulation not comprising the sugar.

114. The method of paragraph 112 or 113, further comprising lyophilizing the formulation to achieve a residual moisture content between about 1% and about 5%.

115. A method of reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar.

116. A method of reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar.

117. Use of a sugar for reducing rAAV genome release from rAAV particles, comprising producing a formulation comprising rAAV particles, a buffering agent, a sugar, a salt, and optionally a nonionic surfactant, wherein rAAV genome release from the rAAV particles after three freeze-thaw cycles is reduced compared to rAAV genome release in a formulation not comprising the sugar.

118. Use of a sugar for reducing rAAV genome release from rAAV particles, comprising producing a formulation comprising rAAV particles, a buffering agent, a sugar, a salt, and optionally a nonionic surfactant, wherein rAAV genome release from the rAAV particles after lyophilization and reconstitution is reduced compared to rAAV genome release in a formulation not comprising the sugar.

119. The use of paragraph 117 or 118, further comprising lyophilizing the formulation to achieve a residual moisture content between about 1% and about 7%.

120. Use of a plasticizer for reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar.

121. Use of a plasticizer for reducing rAAV genome release from rAAV particles, comprising producing a formulation comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar.

122. The method of any one of paragraphs 112-116 or the use of any one of paragraphs 117-121, wherein rAAV genome release is determined by measuring relative fluorescence in the presence of a DNA specific fluorescent stain.

123. The method of any one of paragraphs 112-116 and 122 or the use of any one of paragraphs 117-121, wherein freezing-induced rAAV genome release is reduced by at least about 10%, 20%, 50%, 80%, or 90%.

124. The method of any one of paragraphs 112-116 and 122 or the use of any one of paragraphs 117-121, wherein freezing-induced rAAV genome release is substantially eliminated.

125. The method of any one of paragraphs 112-116 and 122 -124 or the use of any one of paragraphs 117-124, wherein the sugar is a non-reducing sugar.

126. The method of paragraph 125 or the use of paragraph 125, wherein the non-reducing sugar is sucrose, trehalose, or raffinose.

127. The method of paragraph 125 or the use of paragraph 125, wherein the non-reducing sugar is sucrose.

128. The method of any one of paragraphs 112-116 and 122-124 or the use of any one of paragraphs 117-124, wherein the sugar is a reducing sugar.

129. The method of paragraph 122 or the use of paragraph 122, wherein the reducing sugar is glucose, fructose, mannose, galactose, or lactose.

130. The method of paragraph 128 or the use of paragraph 128, wherein the reducing sugar is dextrose.

131. The method of any one of paragraphs 111-117 and 122-130 or the use of any one of paragraphs 82-105, wherein the plasticizer comprises glycerol.

132. The method of any one of paragraphs 111-117 and 122-131 or the use of any one of paragraphs 82-106, wherein the formulation is according to any one of paragraphs claims 1-110.

133. A method of producing a stable lyophilized formulation of an rAAV product, comprising a step of subjecting to lyophilization a pre-lyophilized formulation, wherein pre-lyophilized formulation is according to any one of paragraphs claims 1-110.

134. A method of treating or preventing a disease, the method comprising administering to a subject in need thereof a therapeutically effective dose of an rAAV formulation that is a reconstituted stable lyophilized formulation, wherein the pre-lyophilized formulation of the stable lyophilized formulation is according to any one of paragraphs claims 1-110.

## 5. BRIEF DESCRIPTION OF THE DRAWINGS

Figures 1A and B. Viral particle aggregation is affected by ionic strength. (Figure 1A) Viral particle aggregation observed upon dilution into buffers with different ionic strength. Salt 1 is sodium chloride and Salt 2 is sodium citrate. (Figure 1B) Viral particle aggregation increases over time.

Figure 2. Minimum ionic strength to prevent aggregation. Effective diameter of an AAV8 particle at 1.8E+13 GC/mL prepared at different NaCl concentrations. Ionic strength $\geq$ 90 mM appears to be required to prevent particle aggregation as indicated by the diameters of the particles.

Figure 3. Minimum ionic strength is serotype dependent. Effective diameter of an AAV8 (open square) and an AAV9 (open triangle) prepared at different NaCl concentrations. Concentration of vectors was 6.0E+11 GC/mL.

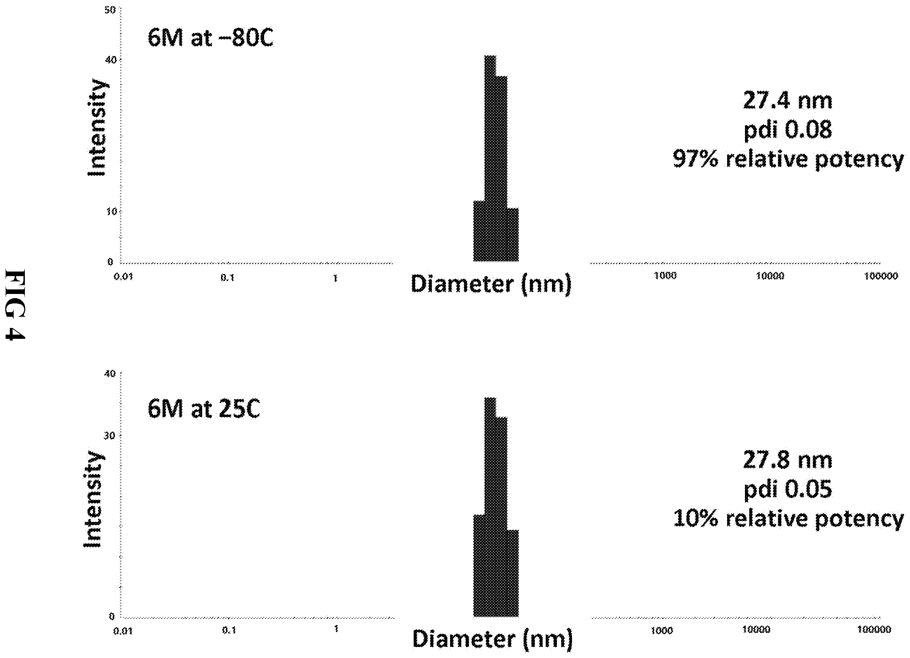

Figure 4. Preventing aggregation is necessary but not sufficient to ensure a stable drug product. Size distribution of viral particles after storage for 6 months at -80°C (6M at -80C) or at 25°C (6M at 25C) shown.

Figures 5A and 5B. Detection of AAV genome release in the presence of SYBR gold, a dye that exhibits significant increase in fluorescent intensity upon binding to DNA. Solutions contain 9.7E+11 GC/mL of an AAV8 particle in Tris buffer containing poloxamer 188 in either (A) NaCl or (B) sodium citrate. Solid line is non-frozen control and dashed line is after 3 freeze-thaw cycles.

Figure 6. Increased DNA release in presence of salt that crystallizes during freezing. Salt 1 is sodium citrate and Salt 2 is sodium chloride. DNA release in control sample and following 3 freeze-thaw cycles (3X F/T) is shown. Buffer 1 is Tris, buffer 2 is Sodium Phosphate and buffer 3 is Histidine.

Figures 7A and 7B. Detection of AAV genome release in the presence of SYBR gold. Solutions contain 1E+12 GC/mL of an AAV8 in either phosphate-buffered saline (PBS) (Figure 7A) or PBS containing 4% sucrose (Figure 7B). Solid line is non-frozen control and dashed line is after 10 freeze-thaw cycles.

Figure 8. Addition of amorphous sugar inhibits co-solute crystallization, exhibits less DNA release. DNA release in control sample and following 3 freeze-thaw cycles (3X F/T) in a buffer comprising different ratios of crystalline mannitol and amorphous sucrose components is shown.

Figure 9. GC loss leads to concurrent loss in relative potency post-lyophilization. Relative potency and viral DNA titer of formulations post-lyophilization is shown. Samples marked with "C" included a crystalline bulking agent.

Figure 10. pH of the frozen solution impacts stability of the lyophilized solid. pH of liquid and frozen solutions is indicated by color. Relative potency of lyophilized formulation after storage for 6 months at 25°C is shown.

Figure 11. Percent relative potency of an AAV8 formulation in a non-lyophilized control ('Liquid'), after lyophilization ('Post Lyo'), and after 3 months and 6 months room temperature storage. Formulations are in either Tris buffer (solid fill) or phosphate buffer (gray fill).

Figure 12. Percent relative potency of a lyophilized AAV8 formulation during storage at 35°C. Residual moisture contents are 5.8% (circles), 4.7% (triangles), 3.6% (diamonds), and 2.1% (squares).

Figure 13. Percent relative potency of a lyophilized AAV8 formulation during storage at 5°C (triangles) or room temperature (squares).

Figure 14. Detection of AAV genome release in the presence of SYBR gold. Solutions contain either formulation buffer alone (thick solid line) or 2.4E+11 GC/mL of an AAV8 prior to lyophilization (solid line) or after lyophilization to different residual moisture levels followed by reconstitution to original volume with water: 4.7% (dotted line), 3.6% (dashed line), and 2.1% (dash-dot line). More AAV genomes are released as residual moisture decreases.

Figures 15A-15C. Comparison of AAV stability in Formulations 1A, 6A and 7A after lyophilization. Figure15A shows free DNA release due to AAV capsid damage. Higher fluorescence intensity indicates more free DNA. Figure 15 B shows the log loss of viral titer. Figure 15 C shows in vitro relative potency.

Figure 16. Thermal transition of Formulation 4A characterized by low temperature DSC.

Figures 17A-17C. Comparison of Formulation 1A, 2A, 3A, 4A and 5A after 35 °C for 2 weeks. Figure 17A shows free DNA release due to AAV capsid damage. Figure 17B shows viral titer. Figure 17C shows in vitro relative potency.

Figures 18A-18C. Particle size of AAV after dilution in NaCl or $Na_2SO4$ solution at different ionic strength, Figure 18A shows diameters at $T_0$. Figure 18B shows diameters after 8 hrs. Figure 18C shows the diameters at room temperature after 24 hours in the presence of 30 mM $Na_2SO4$ at increasing sucrose concentration.

Figure 19 shows the pH shifts of dPBS, Formulation 4A and 5A when freezing down to -20 °C.

Figure 19A and 19B show the pH shifts of dPBS, Formulation 4A and 5A when freezing down to -20 °C.

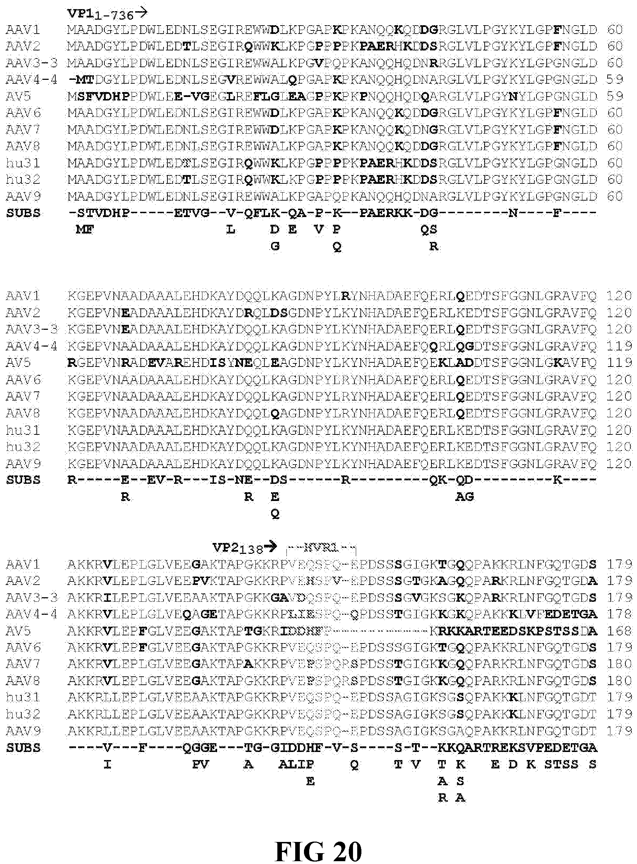

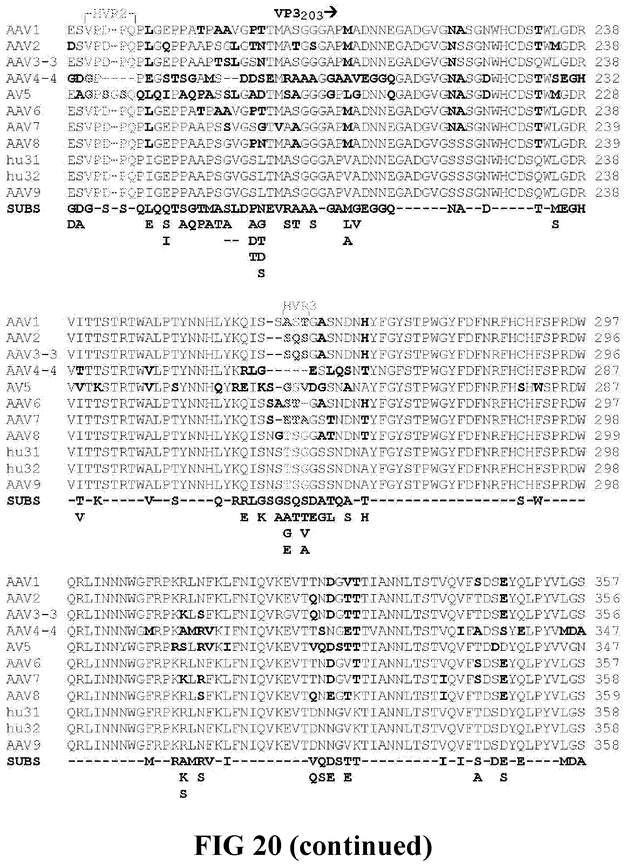

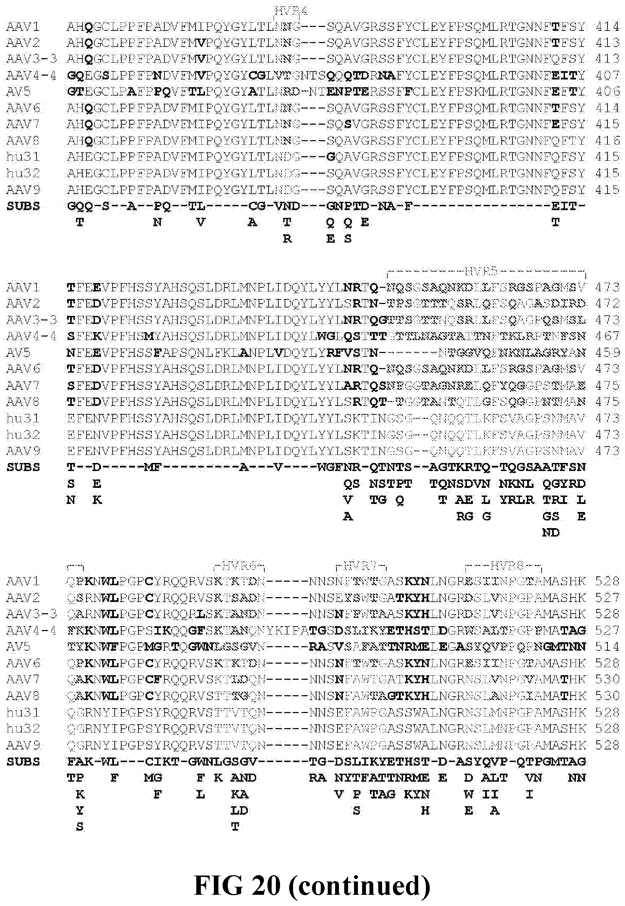

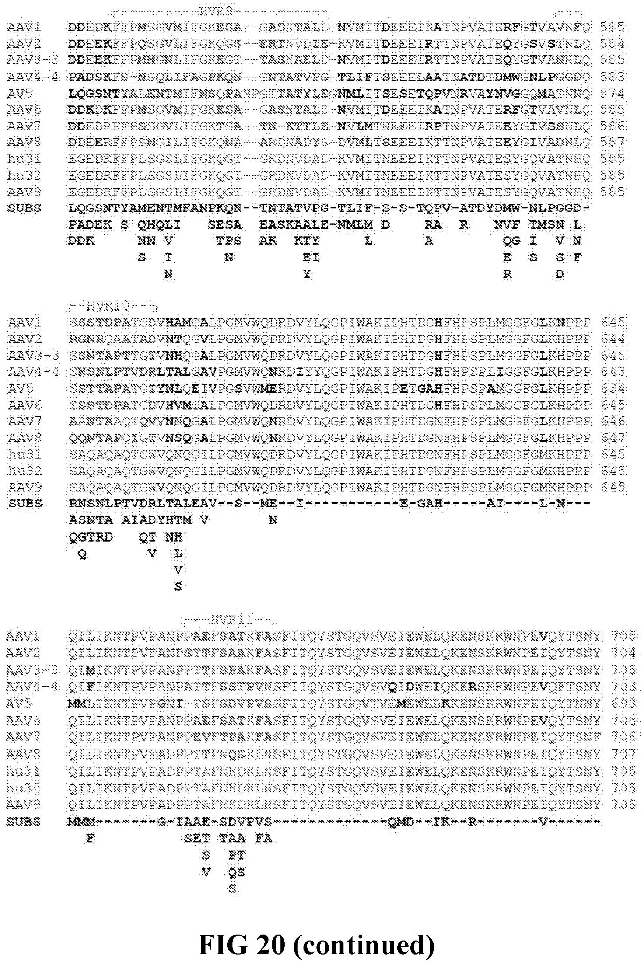

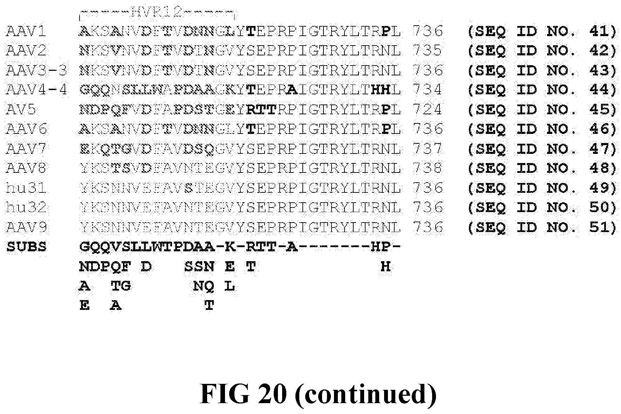

Figure 20. Clustal Multiple Sequence Alignment of AAV capsids 1 - 9 (SEQ ID NOs: 41-51). Amino acid substitutions (shown in bold in the bottom rows) can be made to AAV9 and AAV8 capsids by "recruiting" amino acid residues from the corresponding position of other aligned AAV capsids. Sequence regions designated by "HVR" = hypervariable regions.

## 6. DETAILED DESCRIPTION

Provided herein are formulations comprising rAAV particles. Reference to rAAV particle in the context of the invention and its embodiments means a rAAV particle comprising an AAV8 capsid protein. In some embodiments, a formulation disclosed herein comprises a buffering agent Tris, a sugar as stabilizer, an amorphous salt, and optionally a nonionic surfactant. A formulation disclosed herein may be a frozen formulation or a lyophilized formulation. In some embodiments, a formulation disclosed herein remains amorphous during freezing. In some embodiments, a formulation comprising rAAV particles disclosed herein are stable when stored at room temperature for extended periods of time without significant loss of infectivity.

In some embodiments, provided herein are stable formulations, stable formulations for use in treating diseases, and methods to produce the stable formulations.

In some embodiments, formulations described in Section 6.2 are formulated such that they have one or more functional properties described in Section 6.2. In some embodiments, stable formulations described in Section 6.2 are for formulated such that they have one or more properties described in Section 6.2. In certain embodiments, the formulations provided herein has various advantages, for example, improved stability after lyophilization, and improved long-term stability under various conditions. Also provided herein are assays that may be used in Section 6.8. The inventors surprisingly found that the minimum ionic strength requirement to prevent aggregation is AAV serotype dependent. AAV8 aggregation can be prevented at ionic strengths lower than 200 mM, and less ionic strength is required for AAV9 compared to AAV8. The inventors have further surprisingly found that crystallization of formulation components during freezing promotes the loss of rAAV genome from rAAV particles. To the inventors' knowledge, there are no published reports of this type of genome loss, or methods for preventing the genome loss through formulation development. The inventors have found that the genome loss can be mitigated by either of the following two formulation approaches: (a) formulating with a non-crystallizing salt instead of a salt that crystallizes during freezing, and (b) including a component in the formulation (e.g., a sugar) that inhibits the crystallization. In some embodiments, an additional unique feature of the compositions disclosed herein is the use of sodium citrate in combination with sucrose, which offers unique advantages to the freeze-drying process compared to other salts. In some embodiments, a formulation disclosed herein uses a buffering agent that prevents pH shifting during freezing.

The inventors have further surprisingly found that, in some embodiments, drying a lyophilized rAAV formulation as much as possible does not result in the highest stability in the dried state. Consequently, in some embodiments, a stable rAAV formulation disclosed herein comprises between about 1% and 7% of residual moisture content. Additionally, in some embodiments, without being bound by a particular theory, a stable rAAV formulation disclosed herein comprises a plasticizer (e.g., glycerol) that can act as water substitute in the dried state.

### 6.1 Definitions

Unless defined otherwise, all technical and scientific terms used herein have the same meaning as commonly understood by one of ordinary skill in the art to which this disclosure is related. To facilitate an understanding of the disclosed methods, a number of terms and phrases are defined below.

"About" modifying, for example, the quantity of an ingredient in the compositions, concentration of an ingredient in the compositions, flow rate, rAAV particle yield, feed volume, salt concentration, and like values, and ranges thereof, employed in the methods provided herein, refers to variation in the numerical quantity that can occur, for example, through typical measuring and handling procedures used for making concentrates or use solutions; through inadvertent error in these procedures; through differences in the manufacture, source, or purity of the ingredients employed to make the compositions or carry out the methods; and like considerations. The term "about" also encompasses amounts that differ due to aging of a composition with a particular initial concentration or mixture. The term "about" also encompasses amounts that differ due to mixing or processing a composition with a particular initial concentration or mixture. Whether or not modified by the term "about" the claims include equivalents to the quantities. In some embodiments, the term "about" refers to ranges of approximately 10-20% greater than or less than the indicated number or range. In further embodiments, "about" refers to plus or minus 10% of the indicated number or range. For example, "about 10%" indicates a range of 9% to 11%. In certain embodiments, the term "about" encompasses the exact number recited.

"AAV" is an abbreviation for adeno-associated virus, and may be used to refer to the virus itself or modifications, derivatives, or pseudotypes thereof. The term covers all subtypes and both naturally occurring and recombinant forms, except where required otherwise. The abbreviation "rAAV" refers to recombinant adeno-associated virus. The term "AAV" includes AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), AAV type 9 (AAV-9), avian AAV, bovine AAV, canine AAV, equine AAV, primate AAV, non-primate AAV, and ovine AAV, and modifications, derivatives, or pseudotypes thereof. "Primate AAV" refers to AAV that infect primates, "non-primate AAV" refers to AAV that infect non-primate mammals, "bovine AAV" refers to AAV that infect bovine mammals, etc.

"Recombinant", as applied to an AAV particle means that the AAV particle is the product of one or more procedures that result in an AAV particle construct that is distinct from an AAV particle in nature.

A recombinant Adeno-associated virus particle "rAAV particle" refers to a viral particle composed of at least one AAV capsid protein and an encapsidated polynucleotide rAAV vector comprising a heterologous polynucleotide (i.e. a polynucleotide other than a wild-type AAV genome such as a transgene to be delivered to a mammalian cell). The rAAV particle may be of any AAV serotype, including any modification, derivative or pseudotype (e.g., AAV-1, AAV-2, AAV-3, AAV-4, AAV-5, AAV-6, AAV-7, AAV-8, AAV-9, or AAV-10, or derivatives/modifications/pseudotypes thereof). Such AAV serotypes and derivatives/modifications/pseudotypes, and methods of producing such serotypes/derivatives/modifications/ pseudotypes are known in the art (see, e.g., Asokan et al., Mol. Ther. 20(4):699-708 (2012).

The rAAV particles of the disclosure may be of any serotype, or any combination of serotypes, (e.g., a population of rAAV particles that comprises two or more serotypes, e.g., comprising two or more of rAAV2, rAAV8, and rAAV9 particles). In some embodiments, the rAAV particles are rAAV1, rAAV2, rAAV3, rAAV4, rAAV5, rAAV6, rAAV7, rAAV8, rAAV9, rAAV10, or other rAAV particles, or combinations of two or more thereof). In some examples, the rAAV particles are rAAV8 or rAAV9 particles.

The rAAV particles may have an AAV capsid protein of a serotype selected from the group consisting of AAV-1, AAV-2, AAV-3, AAV-4, AAV-5, AAV-6, AAV-7, AAV-8, AAV-9, AAV-10, AAV-11, AAV-12, AAV-13, AAV-14, AAV-15 and AAV-16 or a derivative, modification, or pseudotype thereof. According to the invention, the rAAV particles have an AAV capsid protein of a serotype of AAV-8, or a derivative, modification, or pseudotype thereof.

The terms "purifying", "purification", "separate", "separating", "separation", "isolate", "isolating", or "isolation", as used herein, refer to increasing the degree of purity of rAAV particles from a sample comprising the target product and one or more impurities. Typically, the degree of purity of the target product is increased by removing (completely or partially) at least one impurity from the sample. In some embodiments, the degree of purity of the rAAV in a sample is increased by removing (completely or partially) one or more impurities from the sample by using a method described herein.

As used in the present disclosure and claims, the singular forms "a", "an" and "the" include plural forms unless the context clearly dictates otherwise.

It is understood that wherever embodiments are described herein with the language "comprising" otherwise analogous embodiments described in terms of "consisting of" and/or "consisting essentially of" are also provided. It is also understood that wherever embodiments are described herein with the language "consisting essentially of" otherwise analogous embodiments described in terms of "consisting of" are also provided.

The term "and/or" as used in a phrase such as "A and/or B" herein is intended to include both A and B; A or B; A (alone); and B (alone). Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

Where embodiments of the disclosure are described in terms of a Markush group or other grouping of alternatives, the disclosed method encompasses not only the entire group listed as a whole, but also each member of the group individually and all possible subgroups of the main group, and also the main group absent one or more of the group members. The disclosed methods also envisage the explicit exclusion of one or more of any of the group members in the disclosed methods.

### 6.2 Formulations comprising rAAV particles

In some embodiments, the disclosure provides formulations comprising Tris recombinant adeno-associated virus (rAAV) particles and a buffering agent Tris, a sugar, and an amorphous salt. In some embodiments, the salt is a pharmaceutically acceptable salt. In some embodiments, the salt is a non-crystallizing, amorphous salt. In some embodiments, the salt is a sodium salt. In some embodiments, the salt is sodium citrate, sodium acetate, or sodium chloride. In some embodiments, the salt is sodium citrate, sodium sulfate, ammonium sulfate or magnesium sulfate. Multivalent salts, which are made up of multiply charged ions having higher ionic strength (per molecule excipient added compared to mono-sodium chloride), may inhibit AAV aggregation while minimizing crystallization. As such, rAAV formulated compositions containing salts other than mono-sodium chloride that maintain the amorphous matrix of the composition are potentially useful.

### 6.2.1 Salt

In some embodiment, the salt excipient is added or adjusted to achieve a desirable ionic strength.

In some embodiments, provided herein is a stable formulation comprising Tris recombinant adeno-associated virus (rAAV) particles and a buffering agent a sugar, and an amorphous salt and having an ionic strength between 60mM and 150 mM, wherein the formulation is suitable for lyophilization. In some embodiments, the stable formulation also comprises a surfactant. In some embodiments, the stable formulation also comprises poloxamer 188.

In some examples, provided herein is a stable formulation comprising recombinant adeno-associated virus (rAAV) particles and a buffering agent, a sugar, and an amorphous salt and having an ionic strength higher than 200mM, wherein the formulation is suitable for lyophilization. In some examples, the stable formulation also comprises a surfactant. In some examples, the stable formulation also comprises poloxamer 188.

In some examples, provided herein is a stable formulation comprising recombinant adeno-associated virus (rAAV) particles and a buffering agent, a sugar, and an amorphous salt and having an ionic strength higher than 200mM, 210mM, 220mM, 230mM, 240mM, 250mM, 260mM, 270mM, 280mM, 290mM, or 300mM, wherein the formulation is suitable for lyophilization. In some examples, the stable formulation also comprises a surfactant. In some examples, the stable formulation also comprises poloxamer 188.

In some embodiments, the salt is a pharmaceutically acceptable salt. In some embodiments, the salt is a non-crystallizing, amorphous salt. In some embodiments, the salt is a sodium salt. In some embodiments, the salt is sodium citrate, sodium acetate, or sodium chloride. In some embodiments, the salt is sodium citrate, sodium sulfate, ammonium sulfate or magnesium sulfate. In some embodiment, the salt is a multivalent salts, which is made up of multiply charged ions having higher ionic strength (per molecule excipient added compared to mono-sodium chloride), and may inhibit AAV aggregation while minimizing crystallization.

In some embodiments, the disclosure provides a stable formulation comprises a recombinant adeno-associated virus (AAV), an amorphous salt excipient, and a sugar. In some embodiments, the stable formulation also comprises a surfactant. In some embodiments, the stable formulation also comprises poloxamer 188. In some embodiments, the stable formulation is suitable for lyophilization.

In some embodiments, provided herein is a stable formulation comprising recombinant adeno-associated virus (rAAV) particles and a buffering agent Tris, a sugar, and an amorphous salt having an ionic strength between 60mM and 150 mM, wherein the formulation is suitable for lyophilization. In some embodiments, the stable formulation also comprises a surfactant. In some embodiments, the stable formulation also comprises poloxamer 188. In some embodiments, the formulation is a pre-lyophilization formulation.

In some embodiments, provided herein is a stable formulation comprising recombinant adeno-associated virus (rAAV) particles and a buffering agent Tris, a sugar, and an amorphous salt having an ionic strength between 30mM and 100 mM, wherein the formulation is suitable for lyophilization. In some embodiments, the stable formulation also comprises a surfactant. In some embodiments, the stable formulation also comprises poloxamer 188. In some embodiments, the formulation is a pre-lyophilization formulation.

In some examples, provided herein is a stable formulation comprising recombinant adeno-associated virus (rAAV) particles and a buffering agent, a sugar, and an amorphous salt having an ionic strength higher than 200mM, wherein the formulation is suitable for lyophilization. In some examples, the stable formulation also comprises a surfactant. In some examples, the stable formulation also comprises poloxamer 188. In some examples, the formulation is a pre-lyophilization formulation.

In some embodiments, provided herein is a stable formulation comprising recombinant adeno-associated virus (rAAV) particles and a buffering agent Tris; a sugar, an amorphous salt having an ionic strength between 60mM and 150 mM, and a surfactant, wherein the formulation is suitable for lyophilization. In some embodiments, the stable formulation also comprises a surfactant. In some embodiments, the stable formulation also comprises poloxamer 188. In some embodiments, the formulation is a pre-lyophilization formulation.

In some embodiments, provided herein is a stable formulation comprising recombinant adeno-associated virus (rAAV) particles and a buffering agent Tris; a sugar, an amorphous salt having an ionic strength between 30mM and 100 mM, and a surfactant, wherein the formulation is suitable for lyophilization. In some embodiments, the stable formulation also comprises a surfactant. In some embodiments, the stable formulation also comprises poloxamer 188. In some embodiments, the formulation is a pre-lyophilization formulation.

In some examples, provided herein is a stable formulation comprising recombinant adeno-associated virus (rAAV) particles and a buffering agent, a sugar, and an amorphous salt having an ionic strength higher than 200mM, and a surfactant wherein the formulation is suitable for lyophilization. In some examples, the stable formulation also comprises a surfactant. In some examples, the stable formulation also comprises poloxamer 188. In some examples, the formulation is a pre-lyophilization formulation.

In some embodiments, a formulation disclosed herein comprises between about 0.0001% and about 0.5% nonionic surfactant. In some embodiments, a formulation disclosed herein comprises between about 0.0005% and about 0.1% nonionic surfactant. In some embodiments, a formulation disclosed herein comprises about 0.001%, about 0.002%, about 0.003%, about 0.004%, about 0.005%, about 0.007%, or about 0.01% nonionic surfactant.

In some embodiments, a formulation disclosed herein comprises between about 0.0001% and about 0.5% poloxamer 188. In some embodiments, a formulation disclosed herein comprises between about 0.0005% and about 0.1% poloxamer 188. In some embodiments, a formulation disclosed herein comprises about 0.001%, about 0.002%, about 0.003%, about 0.004%, about 0.005%, about 0.007%, or about 0.01% poloxamer 188.

In some embodiments, a formulation disclosed herein comprises about 0.0005% poloxamer 188. In some embodiments, a formulation disclosed herein comprises about 0.001% poloxamer 188. In some embodiments, a formulation disclosed herein comprises about 0.002% poloxamer 188. In some embodiments, a formulation disclosed herein comprises about 0.003% poloxamer 188. In some embodiments, a formulation disclosed herein comprises about 0.004% poloxamer 188. In some embodiments, a formulation disclosed herein comprises about 0.005% poloxamer 188. In some embodiments, a formulation disclosed herein comprises about 0.008% poloxamer 188.

In some embodiments, the salt is a pharmaceutically acceptable salt. In some embodiments, the salt is a non-crystallizing, amorphous salt. In some embodiments, the salt is a sodium salt. In some embodiments, the salt is sodium citrate, sodium acetate, or sodium chloride. In some embodiments, the salt is sodium citrate, sodium sulfate, ammonium sulfate or magnesium sulfate. In some embodiment, the salt is a multivalent salt, which is made up of multiply charged ions having higher ionic strength (per molecule excipient added compared to mono-sodium chloride), and may inhibit AAV aggregation while minimizing crystallization.

In some embodiment, the salt excipient is added or adjusted to achieve a desirable ionic strength. In some embodiments, the amorphous salt has an ionic strength no greater than about 150 mM, about 145 mM, about 140 mM, about 135 mM, about 130 mM, about 125 mM, about 120 mM, about 115 mM, about 110 mM, about 110 mM, about 105 mM, or about 100 mM. In certain embodiments, the stable formulation has a buffering agent ionic strength no greater than about 150 mM, about 145 mM, about 140 mM, about 135 mM, about 130 mM, about 125 mM, about 120 mM, about 115 mM, about 110 mM, about 105 mM, or about 100 mM. In some examples, the rAAV particles in the formulation are rAAV8 or rAAV9 particles.

In some embodiments, the stable formulation has an ionic strength no greater than 150 mM, 145 mM, 140 mM, 135 mM, 130 mM, 125 mM, 120 mM, 115 mM, or 110 mM. In certain embodiments, the stable formulation has a buffering agent ionic strength no greater than 150 mM, 145 mM, 140 mM, 135 mM, 130 mM, 125 mM, 120 mM, 115 mM, or 110 mM. According to the invention, the rAAV particles in the formulation have AAV8 capsids.

In certain embodiments, the stable formulation has an ionic greater than 60 mM. In certain embodiments, the stable formulation has an ionic strength about 60 mM to 150 mM. In certain embodiments, the stable formulation has an ionic strength about 60 mM to 115 mM. In certain embodiments, the stable formulation has an ionic strength about 60 mM to 100 mM. In a specific embodiment, the stable formulation has an ionic strength about 60 mM. In a specific embodiment, the stable formulation has an ionic strength about 65 mM. In a specific embodiment, the stable formulation has an ionic strength about 70 mM. In a specific embodiment, the stable formulation has an ionic strength about 75 mM. In a specific embodiment, the stable formulation has an ionic strength about 80 mM. In a specific embodiment, the stable formulation has an ionic strength about 85 mM. In a specific embodiment, the stable formulation has an ionic strength about 90 mM. According to the invention, the rAAV particles in the formulation have AAV8 capsids.

In certain embodiments, the stable formulation has an ionic strength greater than about 30 mM. In certain embodiments, the stable formulation has an ionic strength about 30 mM to 100 mM. In a specific embodiment, the stable formulation has an ionic strength about 30 mM. In a specific embodiment, the stable formulation has an ionic strength about 35 mM. In a specific embodiment, the stable formulation has an ionic strength about 40 mM. In a specific embodiment, the stable formulation has an ionic strength about 45 mM. In a specific embodiment, the stable formulation has an ionic strength about 50 mM. In a specific embodiment, the stable formulation has an ionic strength about 55 mM. In a specific embodiment, the stable formulation has an ionic strength about 60 mM. In a specific embodiment, the stable formulation has an ionic strength about 65 mM. In a specific embodiment, the stable formulation has an ionic strength about 70 mM. In a specific embodiment, the stable formulation has an ionic strength about 75 mM. In a specific embodiment, the stable formulation has an ionic strength about 80 mM. In a specific embodiment, the stable formulation has an ionic strength about 85 mM. In a specific embodiment, the stable formulation has an ionic strength about 90 mM. In a specific embodiment, the stable formulation has an ionic strength about 95 mM. In a specific embodiment, the stable formulation has an ionic strength about 100 mM. In some examples, the rAAV particles in the formulation have AAV9 capsids.

In certain embodiments, the stable formulation has an ionic strength about 60 mM to 115 mM. In certain embodiments, the stable formulation has an ionic strength about 65 mM to 95 mM. In certain embodiments, the stable formulation has an ionic strength about 70 mM to 90 mM. In certain embodiments, the stable formulation has an ionic strength about 75 mM to 85 mM. According to the invention, the rAAV particles in the formulation have AAV8 capsids.

In certain embodiments, the stable formulation has a ionic strength about 30 mM to 100 mM. In certain embodiments, the stable formulation has an ionic strength about 35 mM to 95 mM. In certain embodiments, the stable formulation has an ionic strength about 40 mM to 90 mM. In certain embodiments, the stable formulation has an ionic strength about 45 mM to 85 mM. In certain embodiments, the stable formulation has an ionic strength about 50 mM to 80 mM. In certain embodiments, the stable formulation has an ionic strength about 55 mM to 75 mM. In certain embodiments, the stable formulation has an ionic strength about 60 mM to 70 mM. In some examples, the rAAV particles in the formulation do not have AAV8 capsids. In some examples, the rAAV particles in the formulation have AAV9 capsids.

In some embodiments, the stable formulation has an ionic strength greater than about 200 mM. In some examples, the rAAV particles in the formulation have AAV2 capsids. In some examples, the rAAV particles in the formulation do not have AAV8 or AAV9 capsids.

In some embodiments, the stable formulation has an ionic strength greater than 200 mM, 210 mM, 220 mM, 230 mM, 240 mM, 250 mM, 260 mM, 270 mM, 280 mM, 290mM, or 300mM. In some examples, the rAAV particles in the formulation have AAV2 capsids.

In certain embodiments, the stable formulation has an ionic strength about 200 mM to about 210 mM. In certain embodiments, the stable formulation has an ionic strength about 210 mM to about 220 mM. In certain embodiments, the stable formulation has an ionic strength about 220 mM to about 230 mM. In certain embodiments, the stable formulation has an ionic strength about 230 mM to about 240 mM. In certain embodiments, the stable formulation has an ionic strength about 240 mM to about 250 mM. In certain embodiments, the stable formulation has an ionic strength about 250 mM to about 260 mM. In certain embodiments, the stable formulation has an ionic strength about 260 mM to about 270 mM. In certain embodiments, the stable formulation has an ionic strength about 270 mM to about 280 mM. In certain embodiments, the stable formulation has an ionic strength about 280 mM to about 290 mM. In certain embodiments, the stable formulation has an ionic strength about 290 mM to about 300 mM. In some examples, the rAAV particles in the formulation do not have AAV8 or AAV9 capsids.

In some embodiments, a formulation disclosed herein comprises less than about 100 mM salt. In some embodiments, a formulation disclosed herein comprises between about 10 mM and about 100 mM salt.

In some embodiments, a formulation disclosed herein comprises between about 10 mM and about 100 mM, between about 20 mM and about 100 mM, between about 30 mM and about 100 mM, between about 40 mM and about 100 mM, between about 50 mM and about 100 mM, between about 10 mM and about 80 mM, between about 10 mM and about 60 mM, between about 10 mM and about 50 mM, between about 10 mM and about 40 mM, or between about 10 mM and about 30 mM salt.

In some embodiments, a formulation disclosed herein comprises between about 10 mM and about 50 mM, between about 20 mM and about 60 mM, between about 30 mM and about 70 mM, or between about 10 mM and about 30 mM salt.

In some embodiments, a formulation disclosed herein comprises about 10 mM, about 20 mM, about 30 mM, about 40 mM, about 50 mM, or about 60 mM salt.

In some embodiments, a formulation disclosed herein comprises about 20 mM salt.

In some embodiments, a formulation disclosed herein comprises less than about 100 mM sodium citrate. In some embodiments, a formulation disclosed herein comprises between about 10 mM and about 100 mM sodium citrate.

In some embodiments, a formulation disclosed herein comprises between about 10 mM and about 100 mM sodium citrate. In some embodiments, a formulation disclosed herein comprises between about 20 mM and about 100 mM sodium citrate. In some embodiments, a formulation disclosed herein comprises between about 30 mM and about 100 mM sodium citrate. In some embodiments, a formulation disclosed herein comprises between about 40 mM and about 100 mM sodium citrate. In some embodiments, a formulation disclosed herein comprises between about 50 mM and about 100 mM sodium citrate. In some embodiments, a formulation disclosed herein comprises between about 10 mM and about 80 mM sodium citrate. In some embodiments, a formulation disclosed herein comprises between about 10 mM and about 60 mM sodium citrate. In some embodiments, a formulation disclosed herein comprises between about 10 mM and about 50 mM sodium citrate. In some embodiments, a formulation disclosed herein comprises between about 10 mM and about 40 mM sodium citrate. In some embodiments, a formulation disclosed herein comprises between about 10 mM and about 30 mM sodium citrate.

In some embodiments, a formulation disclosed herein comprises between about 10 mM and about 50 mM sodium citrate. In some embodiments, a formulation disclosed herein comprises between about 20 mM and about 60 mM sodium citrate. In some embodiments, a formulation disclosed herein comprises between about 30 mM and about 70 mM sodium citrate. In some embodiments, a formulation disclosed herein comprises between about 10 mM and about 30 mM sodium citrate.

In some embodiments, a formulation disclosed herein comprises about 10 mM sodium citrate. In some embodiments, a formulation disclosed herein comprises about 20 mM sodium citrate. In some embodiments, a formulation disclosed herein comprises about 30 mM sodium citrate. In some embodiments, a formulation disclosed herein comprises about 40 mM sodium citrate. In some embodiments, a formulation disclosed herein comprises about 50 mM sodium citrate. In some embodiments, a formulation disclosed herein comprises about 60 mM sodium citrate.

In some embodiments, a formulation disclosed herein comprises about 20 mM sodium citrate.

### 6.2.2 Buffering agents

Buffering agents are well known in the art, and include without limitation, phosphate buffers, histidine, sodium citrate, HEPES, Tris, Bicine, glycine, N-glycylglycine, sodium acetate, sodium carbonate, glycyl glycine, lysine, arginine, sodium phosphate, and mixtures thereof. In certain examples, the buffer is histidine (e.g., L-histidine). In some examples, the buffering agent is a pharmaceutically acceptable buffering agent. According to the invention, the buffering agent comprises Tris.

In some embodiments, a formulation disclosed herein comprises between about 1 mM and about 50 mM of a buffering agent. In some embodiments, a formulation disclosed herein comprises between about 1 mM and about 30 mM, between about 1 mM and about 20 mM, between about 5 mM and about 30 mM, between about 5 mM and about 20 mM, between about 10 mM and about 30 mM, between about 10 mM and about 20 mM, or between about 20 mM and about 50 mM of a buffering agent. In some embodiments, a formulation disclosed herein comprises about 1 mM, about 2 mM, about 3 mM, about 5 mM, about 10 mM, about 15 mM, about 20 mM, about 25 mM, about 30 mM, or about 40 mM of a buffering agent. In some embodiments, a formulation disclosed herein comprises about 5 mM of a buffering agent.

In some embodiments, a formulation disclosed herein comprises between about 1 mM and about 50 mM Tris. In some embodiments, a formulation disclosed herein comprises between about 1 mM and about 30 mM, between about 1 mM and about 20 mM, between about 5 mM and about 30 mM, between about 5 mM and about 20 mM, between about 10 mM and about 30 mM, between about 10 mM and about 20 mM, or between about 20 mM and about 50 mM Tris. In some embodiments, a formulation disclosed herein comprises about 1 mM, about 2 mM, about 3 mM, about 5 mM, about 10 mM, about 15 mM, about 20 mM, about 25 mM, about 30 mM, or about 40 mM Tris. In some embodiments, a formulation disclosed herein comprises about 5 mM Tris.

A formulation may comprise a buffering agent capable of providing a pH for a formulation disclosed herein that is substantially the same in the liquid and frozen states.

In some embodiments, a formulation disclosed herein has a pH of between about 6.5 and 8.0. In some embodiments, a formulation disclosed herein has a pH of between about 7.0 and 8.0. In some embodiments, a formulation disclosed herein has a pH of between about 7.2 and 7.8.

In some embodiments, a formulation disclosed herein has a pH of about 6.6. In some embodiments, a formulation disclosed herein has a pH of about 6.8. In some embodiments, a formulation disclosed herein has a pH of about 7.0. In some embodiments, a formulation disclosed herein has a pH of about 7.2. In some embodiments, a formulation disclosed herein has a pH of about 7.3. In some embodiments, a formulation disclosed herein has a pH of about 7.4. In some embodiments, a formulation disclosed herein has a pH of about 7.5. In some embodiments, a formulation disclosed herein has a pH of about 7.6. In some embodiments, a formulation disclosed herein has a pH of about 7.7. In some embodiments, a formulation disclosed herein has a pH of about 7.8.

In some embodiments, a formulation disclosed herein has a pH of about 7.5.

In some embodiments, a formulation disclosed herein has substantially the same pH in liquid and frozen states.

In some embodiments, a formulation disclosed herein has a pH of between about 6.5 and 8.0 in liquid and frozen states. In some embodiments, a formulation disclosed herein has a pH of between about 7.0 and 8.0 in liquid and frozen states. In some embodiments, a formulation disclosed herein has a pH of between about 7.2 and 7.8 in liquid and frozen states. In some embodiments, a formulation disclosed herein has a pH of about 6.6, 6.8, 7.0, or 7.2 in liquid and frozen states. In some embodiments, a formulation disclosed herein has a pH of about 7.2, 7.3, 7.4, 7.5, 7.6, 7.7, or 7.8 in liquid and frozen states. In some embodiments, a formulation disclosed herein has a pH of about 7.5 in liquid and frozen states.

6.2.3 Sugar

In some embodiments, a formulation disclosed herein comprises a non-reducing sugar. In some embodiments, the non-reducing sugar is sucrose, trehalose, raffinose, or a combination thereof. In some embodiments, a formulation disclosed herein comprises sucrose.

In some embodiments, a formulation disclosed herein comprises a reducing sugar. In some embodiments, the reducing sugar is glucose, fructose, mannose, galactose, lactose, or a combination thereof. In some embodiments, a formulation disclosed herein comprises dextrose.

In some embodiments, a formulation disclosed herein comprises between about 50 mM and about 400 mM sugar. In some embodiments, a formulation disclosed herein comprises between about 50 mM and about 350 mM, between about 50 mM and about 300 mM, between about 50 mM and about 250 mM, between about 50 mM and about 200 mM, or between about 50 mM and about 150 mM sugar.

In some embodiments, a formulation disclosed herein comprises between about 100 mM and about 400 mM, between about 150 mM and about 400 mM, between about 200 mM and about 400 mM, between about 250 mM and about 400 mM, or between about 300 mM and about 400 mM sugar.

In some embodiments, a formulation disclosed herein comprises between about 100 mM and about 300 mM, between about 150 mM and about 250 mM, between about 200 mM and about 300 mM, or between about 250 mM and about 350 mM sugar.

In some embodiments, a formulation disclosed herein comprises about 50 mM, about 100 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, or about 350 mM sugar.

In some embodiments, a formulation disclosed herein comprises between about 190 mM and about 230 mM, between about 170 mM and about 250 mM, or between about 150 mM and about 270 mM sugar.

In some embodiments, a formulation disclosed herein comprises about 210 mM sugar.

In some embodiments, a formulation disclosed herein comprises between about 50 mM and about 400 mM sucrose. In some embodiments, a formulation disclosed herein comprises between about 50 mM and about 350 mM sucrose. In some embodiments, a formulation disclosed herein comprises between about 50 mM and about 300 mM sucrose. In some embodiments, a formulation disclosed herein comprises between about 50 mM and about 250 mM sucrose. In some embodiments, a formulation disclosed herein comprises between about 50 mM and about 200 mM sucrose. In some embodiments, a formulation disclosed herein comprises between about 50 mM and about 150 mM sucrose.

In some embodiments, a formulation disclosed herein comprises between about 100 mM and about 400 mM sucrose. In some embodiments, a formulation disclosed herein comprises between about 150 mM and about 400 mM sucrose. In some embodiments, a formulation disclosed herein comprises between about 200 mM and about 400 mM sucrose. In some embodiments, a formulation disclosed herein comprises between about 250 mM and about 400 mM sucrose. In some embodiments, a formulation disclosed herein comprises between about 300 mM and about 400 mM sucrose.

In some embodiments, a formulation disclosed herein comprises between about 100 mM and about 300 mM sucrose. In some embodiments, a formulation disclosed herein comprises between about 150 mM and about 250 mM sucrose. In some embodiments, a formulation disclosed herein comprises between about 200 mM and about 300 mM sucrose. In some embodiments, a formulation disclosed herein comprises between about 250 mM and about 350 mM sucrose.

In some embodiments, a formulation disclosed herein comprises about 50 mM sucrose. In some embodiments, a formulation disclosed herein comprises about 100 mM sucrose. In some embodiments, a formulation disclosed herein comprises about 150 mM sucrose. In some embodiments, a formulation disclosed herein comprises about 160 mM sucrose. In some embodiments, a formulation disclosed herein comprises about 170 mM sucrose. In some embodiments, a formulation disclosed herein comprises about 180 mM sucrose. In some embodiments, a formulation disclosed herein comprises about 190 mM sucrose. In some embodiments, a formulation disclosed herein comprises about 200 mM sucrose. In some embodiments, a formulation disclosed herein comprises about 210 mM sucrose. In some embodiments, a formulation disclosed herein comprises about 220 mM sucrose. In some embodiments, a formulation disclosed herein comprises about 230 mM sucrose. In some embodiments, a formulation disclosed herein comprises about 240 mM sucrose. In some embodiments, a formulation disclosed herein comprises about 250 mM sucrose. In some embodiments, a formulation disclosed herein comprises about 260 mM sucrose. In some embodiments, a formulation disclosed herein comprises about 270 mM sucrose. In some embodiments, a formulation disclosed herein comprises about 280 mM sucrose. In some embodiments, a formulation disclosed herein comprises about 290 mM sucrose. In some embodiments, a formulation disclosed herein comprises about 300 mM sucrose. In some embodiments, a formulation disclosed herein comprises about 350 mM sucrose.

In some embodiments, a formulation disclosed herein comprises between about 190 mM and about 230 mM sucrose. In some embodiments, a formulation disclosed herein comprises between about 170 mM and about 250 mM sucrose. In some embodiments, a formulation disclosed herein comprises between about 150 mM and about 270 mM sucrose.

In some embodiments, a formulation disclosed herein comprises about 210 mM sucrose.

In some embodiments, a formulation disclosed herein comprises a sugar and a salt at a specific ratio. In some embodiments, the ratio of sugar to salt is a molar ratio. In some embodiments, the molar ratio of the sugar to salt is about 1, about 2, about 3, about 5, about 10, about 20, about 40, or about 60. In some embodiments, the molar ratio of the sugar to salt is between about 5 and about 20. In some embodiments, the molar ratio of the sugar to salt is about 10. In some embodiments, the sugar comprises sucrose and the salt comprises sodium citrate.

In some embodiments, a formulation disclosed herein comprises a salt and a sugar at a specific ratio. In some embodiments, the ratio of salt to sugar is a weight to weight (w:w) ratio. In some embodiments, the w:w ratio of the salt to sugar is about 3:1, about 1:1, or about 1:3. In some embodiments, the w:w ratio of the salt to sugar is between about 3:1 and about 1:3. In some embodiments, the w:w ratio of the salt to sugar is about 1:1. In some embodiments, the sugar comprises sucrose and the salt comprises sodium citrate.

### 6.2.4 Surfactants

In some embodiments, a formulation disclosed herein further comprises nonionic surfactant. Pharmaceutically acceptable non-ionic surfactants include, without limitations, poloxamer 188, poloxamer 407, polysorbate 80, polysorbate 20, Pluronic F-68, or BRIJ 35. In some embodiments, a formulation disclosed herein comprises poloxamer 188, poloxamer 407, polysorbate 80, polysorbate 20, Pluronic F-68, BRIJ 35, or a combination thereof. In some embodiments, a formulation disclosed herein comprises poloxamer 188.

In some embodiments, a formulation disclosed herein further comprises between about 0.0001% and about 0.5% nonionic surfactant. In some embodiments, a formulation disclosed herein further comprises between about 0.0005% and about 0.1% nonionic surfactant. In some embodiments, a formulation disclosed herein further comprises about 0.001%, about 0.002%, about 0.003%, about 0.004%, about 0.005%, about 0.007%, or about 0.01% nonionic surfactant.

In some embodiments, a formulation disclosed herein further comprises between about 0.0001% and about 0.5% poloxamer 188. In some embodiments, a formulation disclosed herein further comprises between about 0.0005% and about 0.1% poloxamer 188. In some embodiments, a formulation disclosed herein further comprises about 0.001%, about 0.002%, about 0.003%, about 0.004%, about 0.005%, about 0.007%, or about 0.01% poloxamer 188.

In some embodiments, a formulation disclosed herein further comprises about 0.0005% poloxamer 188. In some embodiments, a formulation disclosed herein further comprises about 0.001% poloxamer 188. In some embodiments, a formulation disclosed herein further comprises about 0.002% poloxamer 188. In some embodiments, a formulation disclosed herein further comprises about 0.003% poloxamer 188. In some embodiments, a formulation disclosed herein further comprises about 0.004% poloxamer 188. In some embodiments, a formulation disclosed herein further comprises about 0.005% poloxamer 188. In some embodiments, a formulation disclosed herein further comprises about 0.008% poloxamer 188.

### 6.2.5 Stabilizers or plasticizers

In some embodiments, a formulation disclosed herein further comprises a plasticizer or a stabilizer. Pharmaceutically acceptable stabilizers or plasticizers include, but are not limited to, glycerol, xylitol, and sorbitol. In some embodiments, a formulation disclosed herein further comprises glycerol, xylitol, sorbitol, or a combination thereof. In some embodiments, a formulation disclosed herein further comprises glycerol.

In some embodiments, a formulation disclosed herein further comprises between about 0.1% and between about 5% plasticizer or a stabilizer. In some embodiments, a formulation disclosed herein further comprises between about 0.1% and between about 2% plasticizer or a stabilizer. In some embodiments, a formulation disclosed herein further comprises between about 0.25% and between about 2% plasticizer.

In some embodiments, a formulation disclosed herein further comprises between about 0.1% and between about 5% glycerol. In some embodiments, a formulation disclosed herein further comprises between about 0.1% and between about 2% glycerol. In some embodiments, a formulation disclosed herein further comprises between about 0.25% and between about 2% glycerol.

### 6.2.6 Formulations Properties

In some embodiments, a formulation disclosed herein has a glass transition temperature that is higher than the glass transition temperature of the corresponding pure sugar and pure salt solutions. In some embodiments, a formulation disclosed herein comprises sucrose and sodium citrate and has a glass transition temperature that is higher than the glass transition temperature of the corresponding pure sucrose and pure salt sodium citrate. In some embodiment, a formulation disclosed herein comprises between about 50 mM and about 400 mM sucrose, and between about 10 mM and 100 mM sodium citrate.

In some embodiments, a formulation disclosed herein comprises between about 1.0E+11 GC/mL and about 1.0E+14 GC/mL rAAV particles. In some embodiments, a formulation disclosed herein comprises between about 1.0E+11 GC/mL and about 1.0E+15 GC/mL rAAV particles. In some embodiments, a formulation disclosed herein comprises about 1.0E+11 GC/mL, about 1.0E+12 GC/mL, about 1.0E+13 GC/mL, about 1.0E+14 GC/mL, or about 1.0E+ 15 GC/mL rAAV particles. The rAAV particles may comprise a capsid protein of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV. HSC12, AAV.HSC13, AAV.HSC14, AAV. HSC15, or AAV.HSC16, or a combination thereof. In some examples, the rAAV particles comprise a capsid protein of the AAV-8 serotype, AAV-9 serotype, or a combination thereof.

In some embodiments, a formulation disclosed herein is a liquid formulation.

In some embodiments, a formulation disclosed herein is a frozen formulation.

In some embodiments, a formulation disclosed herein is a lyophilized formulation lyophilized from a liquid formulation disclosed herein.

In some embodiments, a formulation disclosed herein is a reconstituted lyophilized formulation.

In some embodiments, a formulation disclosed herein is a pre-lyophilization formulation.

In some embodiments, a formulation disclosed herein is a lyophilized formulation having a residual moisture content between about 1% and about 7%. In some embodiments, residual moisture content is determined using Karl Fischer titration. In some embodiments, the residual moisture content is between about 1% and about 7%. In some embodiments, the residual moisture content is between about 2% and about 7%. In some embodiments, the residual moisture content is between about 3% and about 7%. In some embodiments, the residual moisture content is between about 4% and about 7%. In some embodiments, the residual moisture content is between about 5% and about 7%. In some embodiments, the residual moisture content is between about 1% and about 6%. In some embodiments, the residual moisture content is between about 1% and about 5%. In some embodiments, the residual moisture content is between about 1% and about 4%. In some embodiments, the residual moisture content is between about 1% and about 3%.

In some embodiments, a formulation disclosed herein is a lyophilized formulation having a residual moisture content between about 1% and about 7%. In some embodiments, the residual moisture content is between about 3% and about 7%. In some embodiments, the residual moisture content is between about 3% and about 6%. In some embodiments, the residual moisture content is between about 3% and about 5%.

In some embodiments, a formulation disclosed herein is a lyophilized formulation having a residual moisture content between about 1% and about 7%. In some embodiments, the residual moisture content is about 3%. In some embodiments, the residual moisture content is about 3.5%. In some embodiments, the residual moisture content is about 4%. In some embodiments, the residual moisture content is about 4.5%. In some embodiments, the residual moisture content is about 5%. In some embodiments, the residual moisture content is about 5.5%. In some embodiments, the residual moisture content is about 6%.

In some embodiments of a formulation disclosed herein, the % relative potency of the rAAV particles is at least about 60%, at least about 70%, or at least about 80% after storing the formulation for 3 months at room temperature. In some embodiments of a formulation disclosed herein, the % relative potency of the rAAV particles is at least about 60% after storing the formulation for 3 months at room temperature. In some embodiments of a formulation disclosed herein, the % relative potency of the rAAV particles is at least about 70% after storing the formulation for 3 months at room temperature. In some embodiments of a formulation disclosed herein, the % relative potency of the rAAV particles is at least about 60%, at least about 70%, or at least about 80% after storing the formulation for 6 months at room temperature. In some embodiments of a formulation disclosed herein, the % relative potency of the rAAV particles is at least about 60%, after storing the formulation for 6 months at room temperature. In some embodiments of a formulation disclosed herein, the % relative potency of the rAAV particles is at least about 70%, after storing the formulation for 6 months at room temperature. In some embodiments of a formulation disclosed herein, the % relative potency of the rAAV particles is at least about 30%, at least about 40%, at least about 50%, or at least 60% after storing the formulation for 1 week at 35°C. In some embodiments of a formulation disclosed herein, the % relative potency of the rAAV particles is at least about 30% after storing the formulation for 1 week at 35°C. In some embodiments of a formulation disclosed herein, the % relative potency of the rAAV particles is at least about 50% after storing the formulation for 1 week at 35°C. In some embodiments of a formulation disclosed herein, the % relative potency of the rAAV particles is at least about 30%, at least about 40%, at least about 50%, or at least 60% after storing the formulation for 2 weeks at 35°C. In some embodiments of a formulation disclosed herein, the % relative potency of the rAAV particles is at least about 30% after storing the formulation for 2 weeks at 35°C. In some embodiments of a formulation disclosed herein, the % relative potency of the rAAV particles is at least about 50% after storing the formulation for 2 weeks at 35°C. In some embodiments of a formulation disclosed herein, the % relative potency of the rAAV particles is at least about 30%, at least about 40%, at least about 50%, or at least 60% after storing the formulation for 4 weeks at 35°C. In some embodiments of a formulation disclosed herein, the % relative potency of the rAAV particles is at least about 30% after storing the formulation for 4 weeks at 35°C. In some embodiments of a formulation disclosed herein, the % relative potency of the rAAV particles is at least about 50% after storing the formulation for 4 weeks at 35°C. In some embodiments, the reference rAAV particles are stored at -70°C in DPBS with 0.001% poloxamer 188 buffer. In some embodiments, % relative potency is determined as disclosed in PCT International Application No. PCT/US19/56042, filed on October 14, 2019, titled "METHODS FOR MEASURING THE INFECTIVITY OF REPLICATION DEFECTIVE VIRAL VECTORS AND VIRUSES". In some embodiments, the formulation is a frozen formulation. In some embodiments, the formulation is a lyophilized formulation. In some embodiments, the formulation is a pre-lyophilized formulation. In some embodiments, the formulation is a liquid formulation. In some embodiments, the comprises rAAV particles and between about 1 mM and about 25 mM of Tris, between about 50 mM and about 400 mM sucrose, between about 10 mM and about 100 mM sodium citrate, and between about 0.0005% and about 0.01 % of a non-ionic surfactant, wherein the formulation has a pH of between about 7.2 and about 7.8.

In some embodiments, a formulation disclosed herein comprises rAAV particles and between about 1 mM and about 25 mM of a buffering agent, between about 50 mM and about 400 mM sugar, between about 10 mM and about 100 mM sodium citrate, and between about 0.0005% and about 0.01 % non-ionic surfactant, wherein the formulation has a pH of between about 7.2 and about 7.8. In some embodiments, the buffering agent comprises Tris, and the sugar comprises sucrose. In some embodiments, the buffering agent comprises Tris, the sugar comprises sucrose, and the non-ionic surfactant comprises poloxamer 188. In some embodiments, the buffering agent comprises Tris, the sugar comprises sucrose, the non-ionic surfactant comprises poloxamer 188, and the formulation further comprises a plasticizer (e.g., between about 0.1% and between about 2% glycerol). In some embodiments, the formulation is a frozen formulation. In some embodiments, the formulation is a lyophilized formulation. In some embodiments, the formulation is a liquid formulation. In some examples the rAAV particles comprise rAAV-8, rAAV-9, or a combination thereof. In some embodiments, the formulation comprises between about 1.0E+11 GC/mL and about 1.0E+14 GC/mL rAAV particles. In some embodiments, the formulation comprises between about 1.0E+11 GC/mL and about 1.0E+15 GC/mL rAAV particles.

In some embodiments, a formulation disclosed herein comprises rAAV particles and between about 2 mM and about 10 mM of a buffering agent, between about 150 mM and about 250 mM sugar, between about 10 mM and about 20 mM sodium citrate, and between about 0.001% and about 0.005 % non-ionic surfactant, wherein the formulation has a pH of between about 7.2 and about 7.8. In some embodiments, the buffering agent comprises Tris, and the sugar comprises sucrose. In some embodiments, the buffering agent comprises Tris, the sugar comprises sucrose, and the non-ionic surfactant comprises poloxamer 188. In some embodiments, the buffering agent comprises Tris, the sugar comprises sucrose, the non-ionic surfactant comprises poloxamer 188, and the formulation further comprises a plasticizer (e.g., between about 0.1% and between about 2% glycerol). In some embodiments, the formulation is a frozen formulation. In some embodiments, the formulation is a lyophilized formulation. In some embodiments, the formulation is a liquid formulation. In some examples, the rAAV particles comprise rAAV-8, rAAV-9, or a combination thereof. In some embodiments, the formulation comprises between about 1.0E+11 GC/mL and about 1.0E+14 GC/mL rAAV particles. In some embodiments, the formulation comprises between about 1.0E+11 GC/mL and about 1.0E+15 GC/mL rAAV particles.

In some embodiments, a formulation disclosed herein comprises rAAV particles and about 5 mM of a buffering agent, about 210 mM sugar, about 20 mM sodium citrate, and about 0.002 % non-ionic surfactant, wherein the formulation has a pH of about 7.5. In some embodiments, the buffering agent comprises Tris, and the sugar comprises sucrose. In some embodiments, the buffering agent comprises Tris, the sugar comprises sucrose, and the non-ionic surfactant comprises poloxamer 188. In some embodiments, the buffering agent comprises Tris, the sugar comprises sucrose, the non-ionic surfactant comprises poloxamer 188, and the formulation further comprises a plasticizer (e.g., between about 0.1% and between about 2% glycerol). In some embodiments, the formulation is a frozen formulation. In some embodiments, the formulation is a lyophilized formulation. In some embodiments, the formulation is a liquid formulation. In some examples, the rAAV particles comprise rAAV-8, rAAV-9, or a combination thereof. In some embodiments, the formulation comprises between about 1.0E+11 GC/mL and about 1.0E+14 GC/mL rAAV particles. In some embodiments, the formulation comprises between about 1.0E+11 GC/mL and about 1.0E+15 GC/mL rAAV particles.

Formulations disclosed herein can comprise rAAV particles comprising a capsid protein from any AAV capsid serotype. In some examples, the rAAV particles comprise a capsid protein from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16. In some examples, the rAAV particles comprise a capsid protein that is a derivative, modification, or pseudotype of AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16 capsid protein.

In some examples, the rAAV particles comprise a capsid protein from an AAV capsid serotype selected from AAV-8 and AAV-9. According to the invention, the rAAV particles have an AAV capsid serotype of AAV-8. In some examples, the rAAV particles have an AAV capsid serotype of AAV-9.

In some examples, the rAAV particles comprise a capsid protein that is a derivative, modification, or pseudotype of AAV-8 or AAV-9 capsid protein. In some embodiments, the rAAV particles comprise a capsid protein that has an AAV-8 capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of AAV-8 capsid protein.

In some examples, the rAAV particles composition comprise a capsid protein that is a derivative, modification, or pseudotype of AAV-9 capsid protein. In some examples, rAAV particles in the feed composition comprise a capsid protein that has an AAV-9 capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of AAV-9 capsid protein.

In additional embodiments, the rAAV particles comprise a mosaic capsid. In additional embodiments, the rAAV particles comprise a pseudotyped rAAV particle. In additional embodiments, the rAAV particles comprise a capsid containing a capsid protein chimera of two or more AAV capsid serotypes.

In some embodiments, a formulation disclosed herein is a stable formulation comprising a pharmaceutically acceptable carrier.

As used herein the term "pharmaceutically acceptable means a biologically acceptable formulation, gaseous, liquid or solid, or mixture thereof, which is suitable for one or more routes of administration, in vivo delivery or contact. A "pharmaceutically acceptable" composition is a material that is not biologically or otherwise undesirable, e.g., the material may be administered to a subject without causing substantial undesirable biological effects. Thus, such a stable formulation may be used, for example in administering rAAV isolated according to the disclosed methods to a subject. Supplementary active compounds (e.g., preservatives, antibacterial, antiviral and antifungal agents) can be incorporated into the compositions. Pharmaceutical compositions can be formulated to be compatible with a particular route of administration or delivery, as set forth herein or known to one of skill in the art. Thus, pharmaceutical compositions include carriers, diluents, or excipients suitable for administration by various routes. Pharmaceutical compositions and delivery systems appropriate for rAAV particles and methods and uses of the invention are known in the art (see, e.g., Remington: The Science and Practice of Pharmacy (2003) 20th ed., Mack Publishing Co., Easton, Pa.; Remington's Pharmaceutical Sciences (1990) 18th ed., Mack Publishing Co., Easton, Pa.; The Merck Index (1996) 12th ed., Merck Publishing Group, Whitehouse, N.J.; Pharmaceutical Principles of Solid Dosage Forms (1993), Technonic Publishing Co., Inc., Lancaster, Pa.; Ansel and Stoklosa, Pharmaceutical Calculations (2001) 11th ed., Lippincott Williams & Wilkins, Baltimore, Md.; and Poznansky et al., Drug Delivery Systems (1980), R. L. Juliano, ed., Oxford, N.Y., pp. 253-315).

In some embodiments, a formulation disclosed herein is a pharmaceutically acceptable composition that is suitable for administration to a patient in need thereof for administration via known methods, such as intravenous administration (e.g., as a bolus or by continuous infusion over a period of time), or by intramuscular, intraperitoneal, intracerobrospinal, subcutaneous, intra-articular, intrasynovial, intrathecal, oral, topical, or inhalation routes. In some embodiments, a formulation disclosed herein is suitable for systemic or local administration. Systemic administration includes, without limitation: oral, subdermal, intraperitoneal, subcutaneous, transnasal, sublingual, or rectal routes of administration. Local administration includes, without limitation: topical, subcutaneous, intramuscular, subretinal, intrathecal and intraperitoneal routes of administration.

In some embodiments, a formulation disclosed herein is a pharmaceutical unit dose. A "unit dose" refers to a physically discrete unit suited as a unitary dosage for the subject to be treated; each unit containing a predetermined quantity optionally in association with a pharmaceutical carrier (excipient, diluent, vehicle or filling agent) which, when administered in one or more doses, is calculated to produce a desired effect (e.g., prophylactic or therapeutic effect). Unit dose forms may be within, for example, ampules and vials, which may include a liquid composition, or a composition in a freeze-dried or lyophilized state; a sterile liquid carrier, for example, can be added prior to administration or delivery in vivo. Individual unit dose forms can be included in multidose kits or containers. Recombinant vector (e.g., AAV) sequences, plasmids, vector genomes, and recombinant virus particles, and pharmaceutical compositions thereof can be packaged in single or multiple unit dose form for ease of administration and uniformity of dosage. In some examples, the composition comprises rAAV particles comprising an AAV capsid protein from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16. In According to the invention, the AAV capsid serotype is AAV-8. In some examples, the AAV capsid serotype is AAV-9.

In some embodiments, the disclosure provides methods of producing a formulation comprising recombinant adeno-associated virus (rAAV) particles disclosed herein, comprising combining rAAV particles with a buffering agent This, a sugar, an amorphous salt, optionally a plasticizer, and optionally a nonionic surfactant of a formulation disclosed herein, thereby producing the formulation comprising rAAV. Methods for combining the components of a formulation to produce the formulation are known to one of skill in the art.

In certain embodiments, provided herein is a stable formulation comprising recombinant adeno-associated virus (rAAV) particles and

    a) about 5 mM Tris,
    b) about 210 mM sucrose,
    c) about 20 mM sodium citrate, and
    d) about 0.002 % (w/v) poloxamer 188.

In certain embodiments, provided herein is a stable formulation comprising recombinant adeno-associated virus (rAAV) particles and

    a) about 5 mM Tris,
    b) about 210 mM sucrose,
    c) about 20 mM sodium citrate,
    d) about 0.002 % (w/v) poloxamer 188, and
    e) about 0.25% (w/v) glycerol.

In certain embodiments, provided herein is a stable formulation comprising recombinant adeno-associated virus (rAAV) particles and

    a) about 5 mM Tris,
    b) about 210 mM sucrose,
    c) about 20 mM sodium citrate,
    d) about 0.002 % (w/v) poloxamer 188, and
    e) about 0.5% (w/v) sorbitol.

In certain embodiments, provided herein is a stable formulation comprising recombinant adeno-associated virus (rAAV) particles and

a) about 5 mM Tris,
b) about 30 mM sodium sulfate,
c) about 263 mM sucrose, and
d) about 0.005 % (w/v) poloxamer 188.

In certain embodiments, the stable formulation does not comprise mannitol.

In certain embodiments, the stable formulation comprises less than 10 mM, 20 mM, 30mM, 40mM, 50mM, 60mM, 70mM, 80mM, 90mM, 100mM, 110mM, 120mM, 130mM, 140mM, or 150mM mannitol.

In certain embodiments, the stable formulation comprises sucrose at a concentration of about 210 mM. In certain embodiments, the stable formulation comprises sucrose at a concentration of about 263 mM. In certain embodiments, the stable formulation comprises sucrose at a concentration of about 409 mM. In certain embodiments, the stable formulation comprises sucrose at a concentration of about 14.6 mM. In certain embodiments, the stable formulation comprises sucrose at a concentration of about 45 mM. In certain embodiments, the stable formulation comprises sucrose at a concentration between about 0 mM and about 20 mM. In certain embodiments, the stable formulation comprises sucrose at a concentration between about 20 mM and about 50 mM. In certain embodiments, the stable formulation comprises sucrose at a concentration between about 50 mM and about 100 mM. In certain embodiments, the stable formulation comprises sucrose at a concentration between about 100 mM and about 200 mM. In certain embodiments, the stable formulation comprises sucrose at a concentration between about 200 mM and about 300 mM. In certain embodiments, the stable formulation comprises sucrose at a concentration between about 300 mM and about 400 mM. In certain embodiments, the stable formulation comprises sucrose at a concentration between about 400 mM and about 500 mM. In certain embodiments, the stable formulation comprises sucrose at a concentration between about 500 mM and about 600 mM. In certain embodiments, the stable formulation comprises sucrose at a concentration between about 600 mM and about 700 mM.

In certain embodiments, the stable formulation comprises sorbitol at a concentration of about 0.5%. In certain embodiments, the stable formulation comprises sorbitol at a concentration of about 0.25%. In certain embodiments, the stable formulation comprises sorbitol at a concentration of about 0.10%.

In certain embodiments, the stable formulation comprises sorbitol at a concentration of about 0% to about 0.10%. In certain embodiments, the stable formulation comprises sorbitol at a concentration of about 0.10% to about 0.20%. In certain embodiments, the stable formulation comprises sorbitol at a concentration of about 0.20% to about 0.30%. In certain embodiments, the stable formulation comprises sorbitol at a concentration of about 0.30% to about 0.40%. In certain embodiments, the stable formulation comprises sorbitol at a concentration of about 0.40% to about 0.50%. In certain embodiments, the stable formulation comprises sorbitol at a concentration of about 0.50% to about 0.60%. In certain embodiments, the stable formulation comprises sorbitol at a concentration of about 0.60% to about 0.70%. In certain embodiments, the stable formulation comprises sorbitol at a concentration of about 0.70% to about 0.80%. In certain embodiments, the stable formulation comprises sorbitol at a concentration of about 0.80% to about 0.90%. In certain embodiments, the stable formulation comprises sorbitol at a concentration of about 0.90% to about 1.00%.

In certain embodiments, the stable formulation comprises glycerol at a concentration of about 0.5%. In certain embodiments, the stable formulation comprises glycerol at a concentration of about 0.25%. In certain embodiments, the stable formulation comprises glycerol at a concentration of about 0.10%.

In certain embodiments, the stable formulation comprises glycerol at a concentration of about 0% to about 0.10%. In certain embodiments, the stable formulation comprises glycerol at a concentration of about 0.10% to about 0.20%. In certain embodiments, the stable formulation comprises glycerol at a concentration of about 0.20% to about 0.30%. In certain embodiments, the stable formulation comprises glycerol at a concentration of about 0.30% to about 0.40%. In certain embodiments, the stable formulation comprises glycerol at a concentration of about 0.40% to about 0.50%. In certain embodiments, the stable formulation comprises glycerol at a concentration of about 0.50% to about 0.60%. In certain embodiments, the stable formulation comprises glycerol at a concentration of about 0.60% to about 0.70%. In certain embodiments, the stable formulation comprises glycerol at a concentration of about 0.70% to about 0.80%. In certain embodiments, the stable formulation comprises glycerol at a concentration of about 0.80% to about 0.90%. In certain embodiments, the stable formulation comprises glycerol at a concentration of about 0.90% to about 1.00%.

In certain embodiments, the stable formulation comprises poloxamer 188 at a concentration of about 0.001% (weight/volume, 0.01 g/L). In certain embodiments, the stable formulation comprises poloxamer 188 at a concentration of about 0.002% (weight/volume, 0.02 g/L). In certain embodiments, the stable formulation comprises poloxamer 188 at a concentration of about 0.005% (weight/volume, 0.05 g/L).

In certain embodiments, the stable formulation comprises poloxamer 188 at a concentration of about 0.0005% (weight/volume, 0.005 g/L) to about 0.05% (weight/volume, 0.5 g/L). In certain embodiments, the stable formulation comprises poloxamer 188 at a concentration of about 0.0001% (weight/volume, 0.001 g/L) to about 0.01% (weight/volume, 0.1 g/L). In certain embodiments, the stable formulation comprises poloxamer 188 at a concentration of about 0.0005% (weight/volume, 0.005 g/L) to about 0.001% (weight/volume, 0.01 g/L). In certain embodiments, the stable formulation comprises poloxamer 188 at a concentration of about 0.001% (weight/volume, 0.01 g/L) to about 0.05% (weight/volume, 0.5 g/L). In certain embodiments, the stable formulation comprises poloxamer 188 at a concentration of about 0.0005% (weight/volume, 0.005 g/L). In certain embodiments, the stable formulation comprises poloxamer 188 at a concentration of about 0.0006% (weight/volume, 0.006 g/L). In certain embodiments, the stable formulation comprises poloxamer 188 at a concentration of about 0.0007% (weight/volume, 0.007 g/L). In certain embodiments, the stable formulation comprises poloxamer 188 at a concentration of about 0.0008% (weight/volume, 0.008 g/L). In certain embodiments, the stable formulation comprises poloxamer 188 at a concentration of about 0.0009% (weight/volume, 0.009 g/L). In certain embodiments, the stable formulation comprises poloxamer 188 at a concentration of about 0.001% (weight/volume, 0.01 g/L). In certain embodiments, the stable formulation comprises poloxamer 188 at a concentration of about 0.002% (weight/volume, 0.02 g/L). In certain embodiments, the stable formulation comprises poloxamer 188 at a concentration of about 0.003% (weight/volume, 0.03 g/L). In certain embodiments, the stable formulation comprises poloxamer 188 at a concentration of about 0.004% (weight/volume, 0.04 g/L). In certain embodiments, the stable formulation comprises poloxamer 188 at a concentration of about 0.005% (weight/volume, 0.05 g/L). In certain embodiments, the stable formulation comprises poloxamer 188 at a concentration of about 0.01% (weight/volume, 0.1 g/L). In certain embodiments, the stable formulation comprises poloxamer 188 at a concentration of about 0.05% (weight/volume, 0.5 g/L).

In certain embodiments, the pH of the pharmaceutical composition is about 7.4.

In certain embodiments, the pH of the pharmaceutical composition is about 6.0 to 8.8. In certain embodiments, the pH of the pharmaceutical composition is about 6.0 to 9.0. In certain embodiments, the pH of the pharmaceutical composition is about 6.0. In certain embodiments, the pH of the pharmaceutical composition is about 6.1. In certain embodiments, the pH of the pharmaceutical composition is about 6.2. In certain embodiments, the pH of the pharmaceutical composition is about 6.3. In certain embodiments, the pH of the pharmaceutical composition is about 6.4. In certain embodiments, the pH of the pharmaceutical composition is about 6.5. In certain embodiments, the pH of the pharmaceutical composition is about 6.6. In certain embodiments, the pH of the pharmaceutical composition is about 6.7. In certain embodiments, the pH of the pharmaceutical composition is about 6.8. In certain embodiments, the pH of the pharmaceutical composition is about 6.9. In certain embodiments, the pH of the pharmaceutical composition is about 7.0. In certain embodiments, the pH of the pharmaceutical composition is about 7.1. In certain embodiments, the pH of the pharmaceutical composition is about 7.2. In certain embodiments, the pH of the pharmaceutical composition is about 7.3. In certain embodiments, the pH of the pharmaceutical composition is about 7.4. In certain embodiments, the pH of the pharmaceutical composition is about 7.5. In certain embodiments, the pH of the pharmaceutical composition is about 7.6. In certain embodiments, the pH of the pharmaceutical composition is about 7.7. In certain embodiments, the pH of the pharmaceutical composition is about 7.8. In certain embodiments, the pH of the pharmaceutical composition is about 7.9. In certain embodiments, the pH of the pharmaceutical composition is about 8.0. In certain embodiments, the pH of the pharmaceutical composition is about 8.1. In certain embodiments, the pH of the pharmaceutical composition is about 8.2. In certain embodiments, the pH of the pharmaceutical composition is about 8.3. In certain embodiments, the pH of the pharmaceutical composition is about 8.4. In certain embodiments, the pH of the pharmaceutical composition is about 8.5. In certain embodiments, the pH of the pharmaceutical composition is about 8.6. In certain embodiments, the pH of the pharmaceutical composition is about 8.7. In certain embodiments, the pH of the pharmaceutical composition is about 8.8. In certain embodiments, the pH of the pharmaceutical composition is about 8.9. In certain embodiments, the pH of the pharmaceutical composition is about 9.0.

In certain embodiments, the vector genome concentration (VGC) of the stable formulation is about $3 \times 10^9$ GC/mL, $4 \times 10^9$ GC/mL, $5 \times 10^9$ GC/mL, $6 \times 10^9$ GC/mL, $7 \times 10^9$ GC/mL, $8 \times 10^9$ GC/mL, $9 \times 10^9$ GC/mL, about $1 \times 10^{10}$ GC/mL, about $2 \times 10^{10}$ GC/mL, about $3 \times 10^{10}$ GC/mL, about $4 \times 10^{10}$ GC/mL, about $5 \times 10^{10}$ GC/mL, about $6 \times 10^{10}$ GC/mL, about $7 \times 10^{10}$ GC/mL, about $8 \times 10^{10}$ GC/mL, about $9 \times 10^{10}$ GC/mL, about $1 \times 10^{11}$ GC/mL, about $2 \times 10^{11}$ GC/mL, about $3 \times 10^{11}$ GC/mL, about $4 \times 10^{11}$ GC/mL, about $5 \times 10^{11}$ GC/mL, about $6 \times 10^{11}$ GC/mL, about $7 \times 10^{11}$ GC/mL, about $8 \times 10^{11}$ GC/mL, about $9 \times 10^{11}$ GC/mL, about $1 \times 10^{12}$ GC/mL, about $2 \times 10^{12}$ GC/mL, about $3 \times 10^{12}$ GC/mL, about $4 \times 10^{12}$ GC/mL, about $5 \times 10^{12}$ GC/mL, about $6 \times 10^{12}$ GC/mL, about $7 \times 10^{12}$ GC/mL, about $8 \times 10^{12}$ GC/mL, about $9 \times 10^{12}$ GC/mL, about $1 \times 10^{13}$ GC/mL, about $1 \times 10^{13}$ GC/mL, about $2 \times 10^{13}$ GC/mL, or about $3 \times 10^{13}$ GC/mL.

### 6.3 Methods for reducing rAAV genome release from rAAV particles

In some embodiments, the disclosure provides methods of reducing rAAV genome release from rAAV particles, comprising producing a formulation disclosed herein comprising rAAV particles, a buffering agent, a sugar, a salt, and optionally a nonionic surfactant, wherein rAAV genome release from the rAAV particles after three freeze-thaw cycles is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, a method of reducing rAAV genome release disclosed herein further comprises lyophilizing the formulation to achieve a residual moisture content between about 1% and about 5%.

In some embodiments, the disclosure provides methods of reducing rAAV genome release from rAAV particles, comprising producing a formulation disclosed herein comprising **rAAV** particles, a buffering agent, a sugar, a salt, and optionally a nonionic surfactant, wherein rAAV genome release from the rAAV particles after lyophilization and reconstitution is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, a method of reducing rAAV genome release disclosed herein further comprises lyophilizing the formulation to achieve a residual moisture content between about 1% and about 5%.

In some embodiments, the disclosure provides methods of reducing rAAV genome release from rAAV particles, comprising producing a formulation disclosed herein comprising rAAV particles, a buffering agent, a sugar, a salt, a plasticizer, and optionally a nonionic surfactant, wherein rAAV genome release from the rAAV particles after three freeze-thaw cycles is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, the salt comprises sodium citrate, the sugar comprises sucrose, and the plasticizer comprises glycerol. In some embodiments, a method of reducing rAAV genome release disclosed herein further comprises lyophilizing the formulation.

In some embodiments, the disclosure provides methods of reducing rAAV genome release from rAAV particles, comprising producing a formulation disclosed herein comprising rAAV particles, a buffering agent, a sugar, a salt, a plasticizer, and optionally a nonionic surfactant, wherein rAAV genome release from the rAAV particles after lyophilization and reconstitution is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, the salt comprises sodium citrate, the sugar comprises sucrose, and the plasticizer comprises glycerol. In some embodiments, a method of reducing rAAV genome release disclosed herein further comprises lyophilizing the formulation.

In some embodiments, the disclosure provides a use of a sugar for reducing rAAV genome release from rAAV particles, comprising producing a formulation disclosed herein comprising rAAV particles, a buffering agent, the sugar, a salt, and optionally a nonionic surfactant, wherein rAAV genome release from the rAAV particles after three freeze-thaw cycles is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, a use of a sugar for reducing rAAV genome release disclosed herein further comprises lyophilizing the formulation to achieve a residual moisture content between about 1% and about 7%.

In some embodiments, the disclosure provides a use of a sugar for reducing rAAV genome release from rAAV particles, comprising producing a formulation disclosed herein comprising rAAV particles, a buffering agent, the sugar, a salt, and optionally a nonionic surfactant, wherein rAAV genome release from the rAAV particles after lyophilization and reconstitution is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, a use of a sugar for reducing rAAV genome release disclosed herein further comprises lyophilizing the formulation to achieve a residual moisture content between about 1% and about 7%.

In some embodiments, the disclosure provides a use of a plasticizer for reducing rAAV genome release from rAAV particles, comprising producing a formulation disclosed herein comprising rAAV particles, a buffering agent, a sugar, a salt, the plasticizer, and optionally a nonionic surfactant, wherein rAAV genome release from the rAAV particles after three freeze-thaw cycles is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, the salt comprises sodium citrate, the sugar comprises sucrose, and the plasticizer comprises glycerol. In some embodiments, a use of a sugar for reducing rAAV genome release disclosed herein further comprises lyophilizing the formulation.

In some embodiments, the disclosure provides a use of a plasticizer for reducing rAAV genome release from rAAV particles, comprising producing a formulation disclosed herein comprising rAAV particles, a buffering agent, a sugar, a salt, the plasticizer, and optionally a nonionic surfactant, wherein rAAV genome release from the rAAV particles after lyophilization and reconstitution is reduced compared to rAAV genome release in a formulation not comprising the sugar. In some embodiments, the formulation is a frozen formulation. In some embodiments, the formulation is a lyophilized formulation. In some embodiments, the salt comprises sodium citrate. In some embodiments, the salt comprises sodium citrate and the sugar comprises sucrose. In some embodiments, the salt comprises sodium citrate, the sugar comprises sucrose, and the plasticizer comprises glycerol. In some embodiments, a use of a sugar for reducing rAAV genome release disclosed herein further comprises lyophilizing the formulation.

In some embodiments of a method of reducing rAAV genome release or a use of a compound for reducing rAAV genome release disclosed herein, rAAV genome release is determined by measuring relative fluorescence in the presence of a DNA specific fluorescent stain. In some embodiments, the DNA specific fluorescent stain is SYBR Gold.

In some embodiments of a method of reducing rAAV genome release or a use of a compound for reducing rAAV genome release disclosed herein, freezing-induced rAAV genome release is reduced by at least about 10%, 20%, 50%, 80%, or 90%. In some embodiments, freezing-induced rAAV genome release is reduced by at least about 20%. In some embodiments, freezing-induced rAAV genome release is reduced by at least about 50%. In some embodiments, freezing-induced rAAV genome release is reduced by at least about 80%. In some embodiments, freezing-induced rAAV genome release is substantially eliminated.

In some embodiments of a method of reducing rAAV genome release or a use of a compound for reducing rAAV genome release disclosed herein, the sugar is a non-reducing sugar. In some embodiments, the non-reducing sugar is sucrose, trehalose, raffinose, or a combination thereof. In some embodiments, the non-reducing sugar is sucrose.

In some embodiments of a method of reducing rAAV genome release or a use of a compound for reducing rAAV genome release disclosed herein, the sugar is a reducing sugar. In some embodiments, the reducing sugar is glucose, fructose, mannose, galactose, lactose, or a combination thereof. In some embodiments, the reducing sugar is dextrose.

In some embodiments of a method of reducing rAAV genome release or a use of a compound for reducing rAAV genome release disclosed herein, the plasticizer comprises glycerol, xylitol, sorbitol, mannitol, or a combination thereof. In some embodiments, the plasticizer is glycerol.

## 6.4 rAAV **Particles**

The provided formulations are suitable to comprise any isolated recombinant AAV particles, and for use in a method of treating a disease or disorder in a subject in need thereof comprising the administration of any isolated recombinant AAV particles. Additionally, the provided methods are suitable to formulate any isolated recombinant AAV particles, and to reduce rAAV genome release from any isolated rAAV particles. The rAAV may be of any serotype, modification, or derivative, known in the art, or any combination thereof (e.g., a population of rAAV particles that comprises two or more serotypes, e.g., comprising two or more of rAAV2, rAAV8, and rAAV9 particles) known in the art. In some examples, the rAAV particles are AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16 or other rAAV particles, or combinations of two or more thereof.

In some examples, rAAV particles have a capsid protein from an AAV serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16 or a derivative, modification, or pseudotype thereof. In some examples, rAAV particles comprise a capsid protein at least 80% or more identical, *e.g.,* 85%, 85%, 87%, 88%, 89%, 90%, 91%, 92%, 93%, 94%, 95%, 96%, 97%, 98%, 99%, 99.5%, etc., i.e. up to 100% identical, to *e.g.,* VP1, VP2 and/or VP3 sequence of an AAV capsid serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

In some examples, rAAV particles comprise a capsid protein from an AAV capsid serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16, or a derivative, modification, or pseudotype thereof. In some examples, rAAV particles comprise a capsid protein at least 80% or more identical, *e.g.,* 85%, 85%, 87%, 88%, 89%, 90%, 91%, 92%, 93%, 94%, 95%, 96%, 97%, 98%, 99%, 99.5%, etc., i.e. up to 100% identical, to *e.g.,* VP1, VP2 and/or VP3 sequence of an AAV capsid serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

In certain examples, rAAV particles comprise Anc80 or Anc80L65, as described in Zinn et al., 2015, Cell Rep. 12(6): 1056-1068. In certain examples, the rAAV particles comprise one of the following amino acid insertions: LGETTRP or LALGETTRP, as described in United States Patent Nos. 9,193,956; 9458517; and 9,587,282 and US patent application publication no. 2016/0376323. In certain examples, the rAAV particles comprise AAV.7m8, as described in United States Patent Nos. 9,193,956; 9,458,517; and 9,587,282 and US patent application publication no. 2016/0376323. In certain examples, the rAAV particles comprise any AAV disclosed in United States Patent No. 9,585,971, such as AAV-PHP.B. In certain examples, the rAAV particles comprise any AAV disclosed in United States Patent No. 9,840,719 and WO 2015/013313, such as AAV.Rh74 and RHM4-1. In certain examples, the rAAV particles comprise any AAV disclosed in WO 2014/172669, such as AAV rh.74, which is. In certain examples, the rAAV particles comprise AAV2/5, as described in Georgiadis et al., 2016, Gene Therapy 23: 857-862 and Georgiadis et al., 2018, Gene Therapy 25: 450. In certain examples, the rAAV particles comprise any AAV disclosed in WO 2017/070491, such as AAV2tYF. In certain examples, the rAAV particles comprise AAVLK03 or AAV3B, as described in Puzzo et al., 2017, Sci. Transl. Med. 29(9): 418. In certain examples examples, the rAAV particles comprise any AAV disclosed in US Pat Nos. 8,628,966; US 8,927,514; US 9,923,120 and WO 2016/049230, such as HSC1, HSC2, HSC3, HSC4, HSC5, HSC6, HSC7, HSC8, HSC9, HSC10 , HSC11, HSC12, HSC13, HSC14, HSC15, or HSC16.

In certain examples, the rAAV particles comprise an AAV disclosed in any of the following patents and patent applications: United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335. In some examples, the rAAV particles have a capsid protein at least 80% or more identical, *e.g.,* 85%, 85%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of an AAV capsid disclosed in any of the following patents and patent applications: United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335.

In some examples, rAAV particles have a capsid protein disclosed in Intl. Appl. Publ. No. WO 2003/052051 (see, *e.g.,* SEQ ID NO: 2), WO 2005/033321 (see, *e.g.,* SEQ ID NOs: 123 and 88), WO 03/042397 (see, *e.g.,* SEQ ID NOs: 2, 81, 85, and 97), WO 2006/068888 (see, *e.g.,* SEQ ID NOs: 1 and 3-6), WO 2006/110689, (see, *e.g.,* SEQ ID NOs: 5-38) WO2009/104964 (see, *e.g.,* SEQ ID NOs: 1-5, 7, 9, 20, 22, 24 and 31), WO 2010/127097 (see, *e.g.,* SEQ ID NOs: 5-38), and WO 2015/191508 (see, *e.g.,* SEQ ID NOs: 80-294), and U.S. Appl. Publ. No. 20150023924 (see, *e.g.,* SEQ ID NOs: 1, 5-10). In some examples, rAAV particles have a capsid protein at least 80% or more identical, *e.g.,* 85%, 85%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of an AAV capsid disclosed in Intl. Appl. Publ. No. WO 2003/052051 (see, *e.g.,* SEQ ID NO: 2), WO 2005/033321 (see, *e.g.,* SEQ ID NOs: 123 and 88), WO 03/042397 (see, *e.g.,* SEQ ID NOs: 2, 81, 85, and 97), WO 2006/068888 (see, *e.g.,* SEQ ID NOs: 1 and 3-6), WO 2006/110689 (see, *e.g.,* SEQ ID NOs: 5-38) WO2009/104964 (see, *e.g.,* SEQ ID NOs: 1-5, 7, 9, 20, 22, 24 and 31), WO 2010/127097 (see, *e.g.,* SEQ ID NOs: 5-38), and WO 2015/191508 (see, *e.g.,* SEQ ID NOs: 80-294), and U.S. Appl. Publ. No. 20150023924 (see, *e.g.,* SEQ ID NOs: 1, 5-10).

Nucleic acid sequences of AAV based viral vectors and methods of making recombinant AAV and AAV capsids are taught, for example, in United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; International Patent Application Nos. PCT/US2015/034799; PCT/EP2015/053335; WO 2003/052051, WO 2005/033321, WO 03/042397, WO 2006/068888, WO 2006/110689, WO2009/104964, WO 2010/127097, and WO 2015/191508, and U.S. Appl. Publ. No. 20150023924.

The provided methods are suitable for used in the production of recombinant AAV encoding a transgene. In some embodiments, provided herein are AAV viral vectors encoding an anti-VEGF Fab. In specific embodiments, provided herein are AAV8-based viral vectors encoding an anti-VEGF Fab. In more specific embodiments, provided herein are AAV8-based viral vectors encoding ranibizumab. In some embodiments, provided herein are AAV viral vectors encoding Iduronidase (IDUA). In specific examples, provided herein are AAV9-based viral vectors encoding IDUA. In some embodiments, provided herein are AAV viral vectors encoding Iduronate 2-Sulfatase (IDS). In specific examples, provided herein are AAV9-based viral vectors encoding IDS. In some embodiments, provided herein are AAV viral vectors encoding a low-density lipoprotein receptor (LDLR) . In specific embodiments, provided herein are AAV8-based viral vectors encoding LDLR. In some embodiments, provided herein are AAV viral vectors encoding tripeptidyl peptidase 1 (TPP1) protein In specific examples, provided herein are AAV9-based viral vectors encoding TPP.

In additional embodiments, rAAV particles comprise a pseudotyped rAAV particle. In some examples, the pseudotyped AAV are rAAV2/8 or rAAV2/9 pseudotyped AAV. Methods for producing and using pseudotyped rAAV particles are known in the art (see, *e.g.,* Duan et al., J. Virol., 75:7662-7671 (2001); Halbert et al., J. Virol., 74:1524-1532 (2000); Zolotukhin et al., Methods 28:158-167 (2002); and Auricchio et al., Hum. Molec. Genet. 10:3075-3081, (2001).

In additional embodiments, rAAV particles comprise a capsid containing a capsid protein chimeric of two or more AAV capsid serotypes. In some examples, the capsid protein is a chimeric of 2 or more AAV capsid proteins from AAV serotypes selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

In certain embodiments, a single-stranded AAV (ssAAV) can be used. In certain embodiments, a self-complementary vector, e.g., scAAV, can be used (see, e.g., Wu, 2007, Human Gene Therapy, 18(2):171-82, McCarty et al, 2001, Gene Therapy, Vol. 8, Number 16, Pages 1248-1254; and U.S. Patent Nos. 6,596,535; 7,125,717; and 7,456,683).

In some examples, the rAAV particles have a capsid protein from an AAV serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16, or a derivative, modification, or pseudotype thereof. In some examples, the rAAV particles comprise a capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to e.g., VP1, VP2 and/or VP3 sequence of an AAV capsid serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, or AAV.HSC16.

In some examples, the rAAV particles comprise a capsid protein from an AAV capsid serotype selected from AAV-8 or AAV-9. According to the invention, the rAAV particles have an AAV capsid serotype of AAV-8. In some examples, the rAAV particles have an AAV capsid serotype of AAV-9.

In some examples, the rAAV particles comprise a capsid protein that is a derivative, modification, or pseudotype of AAV-8 or AAV-9 capsid protein. In some embodiments, the rAAV particles comprise a capsid protein that has an AAV-8 capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of AAV-8 capsid protein.

In some examples, the rAAV particles comprise a capsid protein that is a derivative, modification, or pseudotype of AAV-9 capsid protein. In some examples, the rAAV particles comprise a capsid protein that has an AAV-9 capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to the VP1, VP2 and/or VP3 sequence of AAV-9 capsid protein.

In additional embodiments, the rAAV particles comprise a mosaic capsid. Mosaic AAV particles are composed of a mixture of viral capsid proteins from different serotypes of AAV. In some examples, the rAAV particles comprise a mosaic capsid containing capsid proteins of a serotype selected from AAV-1, AAV-2, AAV-3, AAV-4, AAV-5, AAV-6, AAV-7, AAV-8, AAV-9, AAV-10, rAAVrh10, AAV-11, AAV-12, AAV-13, AAV-14, AAV-15 and AAV-16. In some examples, the rAAV particles comprise a mosaic capsid containing capsid proteins of a serotype selected from AAV-1, AAV-2, AAV-5, AAV-6, AAV-7, AAV-8, AAV-9, AAV-10, AAVrh.8, and AAVrh.10.

In additional embodiments, the rAAV particles comprise a pseudotyped rAAV particle. In some examples, the pseudotyped rAAV particle comprises (a) a nucleic acid vector comprising AAV ITRs and (b) a capsid comprised of capsid proteins derived from AAVx (e.g., AAV-1, AAV-3, AAV-4, AAV-5, AAV-6, AAV-7, AAV-8, AAV-9, AAV-10 AAV-11, AAV-12, AAV-13, AAV-14, AAV-15 and AAV-16). In additional examples, the rAAV particles comprise a pseudotyped rAAV particle comprised of a capsid protein of an AAV serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, and AAV.HSC16. In additional embodiments, the rAAV particles comprise a pseudotyped rAAV particle containing AAV-8 capsid protein. In additional examples, the rAAV particles comprise a pseudotyped rAAV particle is comprised of AAV-9 capsid protein. In some examples, the pseudotyped rAAV8 or rAAV9 particles are rAAV2/8 or rAAV2/9 pseudotyped particles. Methods for producing and using pseudotyped rAAV particles are known in the art (see, e.g., Duan et al., J. Virol., 75:7662-7671 (2001); Halbert et al., J. Virol., 74:1524-1532 (2000); Zolotukhin et al., Methods 28:158-167 (2002); and Auricchio et al., Hum. Molec. Genet. 10:3075-3081, (2001).

In additional embodiments, the rAAV particles comprise a capsid containing a capsid protein chimeric of two or more AAV capsid serotypes. In some embodiments, the rAAV particles comprise an AAV capsid protein chimeric of AAV-8 capsid protein and one or more AAV capsid proteins from an AAV serotype selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, and AAV.HSC16. In some embodiments, the rAAV particles comprise an AAV capsid protein chimeric of AAV-8 capsid protein and one or more AAV capsid proteins from an AAV serotype selected from AAV-1, AAV-2, AAV-5, AAV-6, AAV-7, AAV-9, AAV-10, rAAVrh10, AAVrh.8, and AAVrh.10. In some examples, the rAAV particles comprise an AAV capsid protein chimeric of AAV-9 capsid protein the capsid protein of one or more AAV capsid serotypes selected from AAV1, AAV2, AAV3, AAV4, AAV5, AAV6, AAV7, AAV8, AAV9, AAV10, AAV-11, AAV-12, AAV-13, AAV-14, AAV-15 and AAV-16, rAAV.rh8, rAAV.rh10, rAAV.rh20, rAAV.rh39, rAAV.Rh74, rAAV.RHM4-1, AAV.hu37, rAAV.Anc80, rAAV.Anc80L65, rAAV.7m8, rAAV.PHP.B, rAAV2.5, rAAV2tYF, rAAV3B, rAAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10 , AAV.HSC11, AAV.HSC12, AAV.HSC13, AAV.HSC14, AAV.HSC15, and AAV.HSC16. In some examples, the rAAV particles comprise an AAV capsid protein chimeric of AAV-9 capsid protein the capsid protein of one or more AAV capsid serotypes selected from AAV1, AAV2, AAV3, AAV4, AAV5, AA6, AAV7, AAV8, AAV9, AAVrh.8, and AAVrh.10.

## 6.5 Methods for Isolating rAAV particles

**In** some embodiments, the disclosure provides methods for producing a formulation comprising isolated recombinant adeno-associated virus (rAAV) particles disclosed herein, comprising (a) isolating rAAV particles from a feed comprising an impurity (for example, rAAV production culture), and (b) formulating the isolated rAAV particles using a method disclosed herein to produce the formulation. In some embodiments, a method for producing a formulation comprising isolated recombinant adeno-associated virus (rAAV) particles disclosed herein comprises (a) isolating rAAV particles from a feed comprising an impurity (for example, rAAV production culture), (b) determining the genome titer of the isolated rAAV particles, and (c) formulating the isolated rAAV particles using a method disclosed herein to produce the formulation.

In some embodiments, the disclosure further provides methods for producing a pharmaceutical unit dosage of a formulation comprising isolated recombinant adeno-associated virus (rAAV) particles disclosed herein, comprising isolating rAAV particles from a feed comprising an impurity (for example, rAAV production culture), determining the genome titer of the isolated rAAV particles, and formulating the isolated rAAV particles using a method disclosed herein. In some embodiments, a method for producing a pharmaceutical unit dosage of a formulation comprising isolated recombinant adeno-associated virus (rAAV) particles disclosed herein comprises isolating rAAV particles from a feed comprising an impurity (for example, rAAV production culture), and formulating the isolated rAAV particles using a method disclosed herein.

Isolated rAAV particles can be isolated using methods known in the art. In some embodiments, methods of isolating rAAV particles comprises downstream processing such as, for example, harvest of a cell culture, clarification of the harvested cell culture (e.g., by centrifugation or depth filtration), tangential flow filtration, affinity chromatography, anion exchange chromatography, cation exchange chromatography, size exclusion chromatography, hydrophobic interaction chromatography, hydroxylapatite chromatography, sterile filtration, or any combination(s) thereof. In some embodiments, downstream processing includes at least 2, at least 3, at least 4, at least 5 or at least 6 of: harvest of a cell culture, clarification of the harvested cell culture (e.g., by centrifugation or depth filtration), tangential flow filtration, affinity chromatography, anion exchange chromatography, cation exchange chromatography, size exclusion chromatography, hydrophobic interaction chromatography, hydroxylapatite chromatography, and sterile filtration. In some embodiments, downstream processing comprises harvest of a cell culture, clarification of the harvested cell culture (e.g., by depth filtration), sterile filtration, tangential flow filtration, affinity chromatography, and anion exchange chromatography. In some embodiments, downstream processing comprises clarification of a harvested cell culture, sterile filtration, tangential flow filtration, affinity chromatography, and anion exchange chromatography. In some embodiments, downstream processing comprises clarification of a harvested cell culture by depth filtration, sterile filtration, tangential flow filtration, affinity chromatography, and anion exchange chromatography. In some embodiments, clarification of the harvested cell culture comprises sterile filtration. In some embodiments, downstream processing does not include centrifugation. According to the invention, the **rAAV** particles comprise a capsid protein of the AAV-8 serotype. In some examples, the rAAV particles comprise a capsid protein of the AAV-9 serotype.

In some embodiments, a method of isolating rAAV particles comprises harvest of a cell culture, clarification of the harvested cell culture (e.g., by depth filtration), a first sterile filtration, a first tangential flow filtration, affinity chromatography, monolith anion exchange chromatography, a second tangential flow filtration, and a second sterile filtration. In some embodiments, a method of isolating rAAV particles comprises clarification of a harvested cell culture, a first sterile filtration, a first tangential flow filtration, affinity chromatography, monolith anion exchange chromatography, a second tangential flow filtration, and a second sterile filtration. In some embodiments, a method of isolating rAAV particles comprises clarification of a harvested cell culture by depth filtration, a first sterile filtration, a first tangential flow filtration, affinity chromatography, monolith anion exchange chromatography, a second tangential flow filtration, and a second sterile filtration. In some embodiments, the method does not include centrifugation. In some embodiments, clarification of the harvested cell culture comprises sterile filtration. According to the invention, the rAAV particles comprise a capsid protein of the AAV-8 serotype. In some examples, the rAAV particles comprise a capsid protein of the AAV-9 serotype.

Numerous methods are known in the art for production of rAAV particles, including transfection, stable cell line production, and infectious hybrid virus production systems which include Adenovirus-AAV hybrids, herpesvirus-AAV hybrids and baculovirus-AAV hybrids. rAAV production cultures for the production of rAAV virus particles all require; (1) suitable host cells, including, for example, human-derived cell lines such as HeLa, A549, or HEK293 cells and their derivatives (HEK293T cells, HEK293F cells), mammalian cell lines such as Vero, or insect-derived cell lines such as SF-9 in the case of baculovirus production systems; (2) suitable helper virus function, provided by wild type or mutant adenovirus (such as temperature sensitive adenovirus), herpes virus, baculovirus, or a plasmid construct providing helper functions; (3) AAV rep and cap genes and gene products; (4) a transgene (such as a therapeutic transgene) flanked by AAV ITR sequences; and (5) suitable media and media components to support rAAV production. Suitable media known in the art may be used for the production of rAAV vectors. These media include, without limitation, media produced by Hyclone Laboratories and JRH including Modified Eagle Medium (MEM), Dulbecco's Modified Eagle Medium (DMEM), and Sf-900 II SFM media as described in U.S. Pat. No. 6,723,551.

rAAV production cultures can routinely be grown under a variety of conditions (over a wide temperature range, for varying lengths of time, and the like) suitable to the particular host cell being utilized. As is known in the art, rAAV production cultures include attachment-dependent cultures which can be cultured in suitable attachment-dependent vessels such as, for example, roller bottles, hollow fiber filters, microcarriers, and packed-bed or fluidized-bed bioreactors. rAAV vector production cultures may also include suspension-adapted host cells such as HeLa, HEK293, Vero, and its derivatives, and SF-9 cells which can be cultured in a variety of ways including, for example, spinner flasks, stirred tank bioreactors, and disposable systems such as the Wave bag system. Numerous suspension cultures are known in the art for production of rAAV particles, including for example, the cultures disclosed in U.S. Patent Nos. 6,995,006, 9,783,826, and in U.S. Pat. Appl. Pub. No. 20120122155

In some embodiments, methods for the production of rAAV particles encompasses providing a cell culture comprising a cell capable of producing rAAV; adding to the cell culture a histone deacetylase (HDAC) inhibitor to a final concentration between about 0.1 mM and about 20 mM; and maintaining the cell culture under conditions that allows production of the rAAV particles. In some embodiments, the HDAC inhibitor comprises a short-chain fatty acid or salt thereof. In some embodiments, the HDAC inhibitor comprises butyrate (e.g., sodium butyrate), valproate (e.g., sodium valproate), propionate (e.g., sodium propionate), or a combination thereof.

In some embodiments, rAAV particles are produced as disclosed in U.S. PCT International Publication No. WO2020/033842, published on February 13, 2020, titled "SCALABLE METHOD FOR RECOMBINANT AAV PRODUCTION"

Recombinant AAV particles can be harvested from rAAV production cultures by harvest of the production culture comprising host cells or by harvest of the spent media from the production culture, provided the cells are cultured under conditions known in the art to cause release of rAAV particles into the media from intact host cells. Recombinant AAV particles can also be harvested from rAAV production cultures by lysis of the host cells of the production culture. Suitable methods of lysing cells are also known in the art and include for example multiple freeze/thaw cycles, sonication, microfluidization, and treatment with chemicals, such as detergents and/or proteases.

At harvest, rAAV production cultures can contain one or more of the following: (1) host cell proteins; (2) host cell DNA; (3) plasmid DNA; (4) helper virus; (5) helper virus proteins; (6) helper virus DNA; and (7) media components including, for example, serum proteins, amino acids, transferrins and other low molecular weight proteins. rAAV production cultures can further contain product-related impurities, for example, inactive vector forms, empty viral capsids, aggregated viral particles or capsids, mis-folded viral capsids, degraded viral particle.

In some embodiments, the rAAV production culture harvest is clarified to remove host cell debris. In some embodiments, the production culture harvest is clarified by filtration through a series of depth filters. Clarification can also be achieved by a variety of other standard techniques known in the art, such as, centrifugation or filtration through any cellulose acetate filter of 0.2 mm or greater pore size known in the art. In some embodiments, clarification of the harvested cell culture comprises sterile filtration. In some embodiments, the production culture harvest is clarified by centrifugation.

In some embodiments, clarification of the production culture harvest does not included centrifugation.

In some embodiments, harvested cell culture is clarified using filtration. In some embodiments, clarification of the harvested cell culture comprises depth filtration. In some embodiments, clarification of the harvested cell culture further comprises depth filtration and sterile filtration. In some embodiments, harvested cell culture is clarified using a filter train comprising one or more different filtration media. In some embodiments, the filter train comprises a depth filtration media. In some embodiments, the filter train comprises one or more depth filtration media. In some embodiments, the filter train comprises two depth filtration media. In some embodiments, the filter train comprises a sterile filtration media. In some embodiments, the filter train comprises 2 depth filtration media and a sterile filtration media. In some embodiments, the depth filter media is a porous depth filter. In some embodiments, the filter train comprises Clarisolve® 20MS, Millistak+® C0HC, and a sterilizing grade filter media. In some embodiments, the filter train comprises Clarisolve® 20MS, Millistak+® C0HC, and Sartopore® 2 XLG 0.2 $\mu$m. In some embodiments, the harvested cell culture is pretreated before contacting it with the depth filter. In some embodiments, the pretreating comprises adding a salt to the harvested cell culture. In some embodiments, the pretreating comprises adding a chemical flocculent to the harvested cell culture. In some embodiments, the harvested cell culture is not pre-treated before contacting it with the depth filter.

In some embodiments, the production culture harvest is clarified by filtration are disclosed in PCT International Publication No. WO2019212921A1, published on November 8, 2019, titled "SCALABLE CLARIFICATION PROCESS FOR RECOMBINANT AAV PRODUCTION".

In some embodiments, the rAAV production culture harvest is treated with a nuclease (e.g., Benzonase®) or endonuclease (e.g., endonuclease from Serratia marcescens) to digest high molecular weight DNA present in the production culture. The nuclease or endonuclease digestion can routinely be performed under standard conditions known in the art. For example, nuclease digestion is performed at a final concentration of 1-2.5 units/ml of Benzonase® at a temperature ranging from ambient to 37°C for a period of 30 minutes to several hours.

Sterile filtration encompasses filtration using a sterilizing grade filter media. In some embodiments, the sterilizing grade filter media is a 0.2 or 0.22 $\mu$m pore filter. In some embodiments, the sterilizing grade filter media comprises polyethersulfone (PES). In some embodiments, the sterilizing grade filter media comprises polyvinylidene fluoride (PVDF). In some embodiments, the sterilizing grade filter media has a hydrophilic heterogeneous double layer design. In some embodiments, the sterilizing grade filter media has a hydrophilic heterogeneous double layer design of a 0.8 $\mu$m pre-filter and 0.2 $\mu$m final filter membrane. In some embodiments, the sterilizing grade filter media has a hydrophilic heterogeneous double layer design of a 1.2 $\mu$m pre-filter and 0.2 $\mu$m final filter membrane. In some embodiments, the sterilizing grade filter media is a 0.2 or 0.22 $\mu$m pore filter. In further embodiments, the sterilizing grade filter media is a 0.2 $\mu$m pore filter. In some embodiments, the sterilizing grade filter media is a Sartopore® 2 XLG 0.2 $\mu$m, Durapore™ PVDF Membranes 0.45$\mu$m, or Sartoguard® PES 1.2 $\mu$m + 0.2 $\mu$m nominal pore size combination. In some embodiments, the sterilizing grade filter media is a Sartopore® 2 XLG 0.2 $\mu$m.

In some embodiments, the clarified feed is concentrated via tangential flow filtration ("TFF") before being applied to a chromatographic medium, for example, affinity chromatography medium. Large scale concentration of viruses using TFF ultrafiltration has been described by Paul et al., Human Gene Therapy 4:609-615 (1993). TFF concentration of the clarified feed enables a technically manageable volume of clarified feed to be subjected to chromatography and allows for more reasonable sizing of columns without the need for lengthy recirculation times. In some embodiments, the clarified feed is concentrated between at least two-fold and at least ten-fold. In some embodiments, the clarified feed is concentrated between at least ten-fold and at least twenty-fold. In some embodiments, the clarified feed is concentrated between at least twenty-fold and at least fifty-fold. In some embodiments, the clarified feed is concentrated about twenty-fold. One of ordinary skill in the art will also recognize that TFF can also be used to remove small molecule impurities (e.g., cell culture contaminants comprising media components, serum albumin, or other serum proteins) form the clarified feed via diafiltration. In some embodiments, the clarified feed is subjected to diafiltration to remove small molecule impurities. In some embodiments, the diafiltration comprises the use of between about 3 and about 10 diafiltration volume of buffer. In some embodiments, the diafiltration comprises the use of about 5 diafiltration volume of buffer. One of ordinary skill in the art will also recognize that TFF can also be used at any step in the purification process where it is desirable to exchange buffers before performing the next step in the purification process. In some embodiments, the methods for isolating rAAV from the clarified feed disclosed herein comprise the use of TFF to exchange buffers.

Affinity chromatography can be used to isolate rAAV particles from a composition. In some embodiments, affinity chromatography is used to isolate rAAV particles from the clarified feed. In some embodiments, affinity chromatography is used to isolate rAAV particles from the clarified feed that has been subjected to tangential flow filtration. Suitable affinity chromatography media are known in the art and include without limitation, AVB Sepharose™, POROS™ CaptureSelect™ AAVX affinity resin, POROS™ CaptureSelect™ AAV9 affinity resin, and POROS™ CaptureSelect™ AAV8 affinity resin. In some embodiments, the affinity chromatography media is POROS™ CaptureSelect™ AAV9 affinity resin. In some embodiments, the affinity chromatography media is POROS™ CaptureSelect™ AAV8 affinity resin. In some embodiments, the affinity chromatography media is POROS™ CaptureSelect™ AAVX affinity resin.

Anion exchange chromatography can be used to isolate rAAV particles from a composition. In some embodiments, anion exchange chromatography is used after affinity chromatography as a final concentration and polish step. Suitable anion exchange chromatography media are known in the art and include without limitation, Unosphere Q (Biorad, Hercules, Calif.), and N-charged amino or imino resins such as e.g., POROS 50 PI, or any DEAE, TMAE, tertiary or quaternary amine, or PEI-based resins known in the art (U.S. Pat. No. 6,989,264; Brument et al., Mol. Therapy 6(5):678-686 (2002); Gao et al., Hum. Gene Therapy 11:2079-2091 (2000)). In some embodiments, the anion exchange chromatography media comprises a quaternary amine. In some embodiments, the anion exchange media is a monolith anion exchange chromatography resin. In some embodiments, the monolith anion exchange chromatography media comprises glycidylmethacrylate-ethylenedimethacrylate or styrene-divinylbenzene polymers. In some embodiments, the monolith anion exchange chromatography media is selected from the group consisting of CIMmultus™ QA-1 Advanced Composite Column (Quaternary amine), CIMmultus™ DEAE-1 Advanced Composite Column (Diethylamino), CIM® QA Disk (Quaternary amine), CIM® DEAE, and CIM® EDA Disk (Ethylene diamino). In some embodiments, the monolith anion exchange chromatography media is CIMmultus™ QA-1 Advanced Composite Column (Quaternary amine). In some embodiments, the monolith anion exchange chromatography media is CIM® QA Disk (Quaternary amine). In some embodiments, the anion exchange chromatography media is CIM QA (BIA Separations, Slovenia). In some embodiments, the anion exchange chromatography media is BIA CIM® QA-80 (Column volume is 80mL). One of ordinary skill in the art can appreciate that wash buffers of suitable ionic strength can be identified such that the rAAV remains bound to the resin while impurities, including without limitation impurities which may be introduced by upstream purification steps are stripped away.

In some embodiments, anion exchange chromatography is performed according to a method disclosed in PCT International Publication Application No. WO2019/241535, published on December 19, 2019, titled "Anion Exchange Chromatography for Recombinant AAV production".

In some embodiments, a method of isolating rAAV particles comprises determining the vector genome titer, capsid titer, and/or the ratio of full to empty capsids in a composition comprising the isolated rAAV particles. In some embodiments, the vector genome titer is determined by quantitative PCR (qPCR) or digital PCR (dPCR) or droplet digital PCR (ddPCR). In some embodiments, the capsid titer is determined by serotype-specific ELISA. In some embodiments, the ratio of full to empty capsids is determined by Analytical Ultracentrifugation (AUC) or Transmission Electron Microscopy (TEM).

In some embodiments, the vector genome titer, capsid titer, and/or the ratio of full to empty capsids is determined by spectrophotometry, for example, by measuring the absorbance of the composition at 260 nm; and measuring the absorbance of the composition at 280 nm. In some embodiments, the rAAV particles are not denatured prior to measuring the absorbance of the composition. In some embodiments, the rAAV particles are denatured prior to measuring the absorbance of the composition. In some embodiments, the absorbance of the composition at 260 nm and 280 nm is determined using a spectrophotometer. In some embodiments, the absorbance of the composition at 260 nm and 280 nm is determined using a HPLC. In some embodiments, the absorbance is peak absorbance. Several methods for measuring the absorbance of a composition at 260 nm and 280 nm are known in the art. Methods of determining vector genome titer and capsid titer of a composition comprising the isolated recombinant rAAV particles are disclosed in PCT International Publication No. WO2019212922A1, published on November 7, 2019, titled "Systems and methods of spectrophotometry for the determination of genome content, capsid content and full/empty ratios of adeno-associated virus particles".

### 6.6 Kits

In some embodiments, the disclosure provides kits comprising a formulation disclosed herein that can be used to perform a method of treating a disease or disorder in a subject in need thereof comprising the administration of isolated recombinant AAV particles. In some embodiments, the kit comprises at least one formulation comprising a formulation comprising rAAV particles disclosed herein in one or more containers. In some embodiments, the kit contains all of the components necessary and/or sufficient to perform a method of treating a disease or disorder in a subject in need thereof comprising the administration of isolated recombinant AAV particles. One skilled in the art will readily recognize that a formulation comprising a formulation comprising rAAV particles disclosed herein can be readily incorporated into one of the established kit formats which are well known in the art.

In order that the disclosure provided herein may be readily understood and put into practical effect, some embodiments will now be described by way of the following non-limiting examples.

### 6.7 Functional Properties

In certain embodiments, the formulation or stable formulation described herein is suitable for intravenous administration, subcutaneous administration, intramuscular injection, suprachoroidal injection (for example, via a suprachoroidal drug delivery device such as a microinjector with a microneedle), subretinal injection via transvitreal approach (a surgical procedure), juxtascleral administration, intravitreal administration, subconjunctival administration, intraretinal administration, subretinal administration via the suprachoroidal space (for example, a surgical procedure via a subretinal drug delivery device comprising a catheter that can be inserted and tunneled through the suprachoroidal space toward the posterior pole, where a small needle injects into the subretinal space), and/or a posterior juxtascleral depot procedure (for example, via a juxtascleral drug delivery device comprising a cannula whose tip can be inserted and kept in direct apposition to the scleral surface)).

In certain embodiments, the pharmaceutical composition has a desired density that is suitable for intravenous administration, subcutaneous administration, intramuscular injection, suprachoroidal injection (for example, via a suprachoroidal drug delivery device such as a microinjector with a microneedle), subretinal injection via transvitreal approach (a surgical procedure), juxtascleral administration, intravitreal administration, subconjunctival administration, intraretinal administration, subretinal administration via the suprachoroidal space (for example, a surgical procedure via a subretinal drug delivery device comprising a catheter that can be inserted and tunneled through the suprachoroidal space toward the posterior pole, where a small needle injects into the subretinal space), and/or a posterior juxtascleral depot procedure (for example, via a juxtascleral drug delivery device comprising a cannula whose tip can be inserted and kept in direct apposition to the scleral surface)).

In certain embodiments, the pharmaceutical composition has a desired viscosity that is suitable for intravenous administration, subcutaneous administration, intramuscular injection, suprachoroidal injection (for example, via a suprachoroidal drug delivery device such as a microinjector with a microneedle), juxtascleral administration, intravitreal administration, subconjunctival administration, intraretinal administration, subretinal injection via transvitreal approach (a surgical procedure), subretinal administration via the suprachoroidal space (for example, a surgical procedure via a subretinal drug delivery device comprising a catheter that can be inserted and tunneled through the suprachoroidal space toward the posterior pole, where a small needle injects into the subretinal space), and/or a posterior juxtascleral depot procedure (for example, via a juxtascleral drug delivery device comprising a cannula whose tip can be inserted and kept in direct apposition to the scleral surface)).

In certain embodiments, the recombinant adeno-associated virus (rAAV) particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times more stable to lyophilization or reconstitution process, than compared to the same rAAV particles in a reference formulation. In certain embodiments, the stability of the rAAV particles is determined by an assay or assays disclosed in Section 6.8.

In certain embodiments, the rAAV particles in the stable formulation has at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times more infectivity, than compared to the same rAAV particles in a reference formulation. In certain embodiments, the virus infectivity of the rAAV particles is determined by an assay or assays disclosed in Section 6.8. In certain embodiments, the infectivity is measured prior to or after lyophilization. In certain embodiments, the infectivity is measured prior to or after reconstitution of the lyophilized formulation.

In certain embodiments, the rAAV particles in the stable formulation has at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times less aggregation, than compared to the same rAAV particles in a reference formulation. In certain embodiments, the aggregation of the rAAV particles is determined by an assay or assays disclosed in Section 6.8. In certain embodiments, the aggregation is measured prior to or after lyophilization. In certain embodiments, the aggregation is measured prior to or after reconstitution of the lyophilized formulation.

In certain embodiments, the stable formulation is lyophilized prior to storing.

In certain embodiment, the stable formulation reconstituted after storing.

In certain embodiments, the formulation is stored at -80°C, -70°C, -20°C, 4°C, 20°C, 25°C, 30°C, 35°C, 37°C or 40 °C.

In certain embodiments, the formulation is stored about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, about 4 years, or about 5 years. In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 more stable after storing the formulation over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, about 4 years or about 5 years, than compared to the same rAAV particles in a reference formulation stored under the same condition. In certain embodiments, the stability over a period of time of the rAAV particles is determined by an assay or assays disclosed in Section 6.8.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 more stable after storing the formulation over a period of time, at least for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, 12 months, about 15 months, about 18 months, about 24 months, about 2 years, about 3 years, about 4 years, or about 5 years, than compared to the same rAAV particles in a reference formulation. In certain embodiments, the stability over a period of time of the rAAV particles is determined by an assay or assays disclosed in Section 6.8.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times higher in vitro relative potency (IVRP), than compared to the same rAAV particles in a reference formulation. In certain embodiments, the in vitro relative potency (IVRP) of the rAAV particles is determined by an assay or assays disclosed in Section 6.8. In certain embodiments, the IVRP is measured prior to or after lyophilization. In certain embodiments, the IVRP is measured prior to or after reconstitution of the lyophilized formulation.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about1000 times less free DNA, than compared to the same rAAV particles in a reference formulation. In certain embodiments, the free DNA of the rAAV particles is determined by an assay or assays disclosed in Section 6.8. In certain embodiments, the free DNA is measured prior to or after lyophilization. In certain embodiments, the free DNA is measured prior to or after reconstitution of the lyophilized formulation.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about1000 times less rAAV genome release, than compared to the same rAAV particles in a reference formulation. In certain embodiments, the rAAV genome release is determined by measuring relative fluorescence in the presence of a DNA specific fluorescent stain. In certain embodiments, the rAAV genome release is determined by an assay or assays disclosed in Section 6.8. In certain embodiments, the rAAV genome release is measured prior to or after lyophilization. In certain embodiments, the rAAV genome release is measured prior to or after reconstitution of the lyophilized formulation.

In certain embodiments, the rAAV particles in the stable formulation has at most about 20%, about 15%, about 10%, about 8%, about 5%, about 4%, about 3%, about 2%, or about 1% change in size after storing the formulation over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the size of the rAAV particles is determined by an assay or assays disclosed in Section 6.8. In certain embodiments, the size is measured prior to or after freeze/thaw cycles.

In certain embodiments, the rAAV particles in the stable formulation has at most 20%, 15%, 10%, 8%, 5%, 4%, 3%, 2%, or 1% change in size after storing the formulation over a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the size of the rAAV particles is determined by an assay or assays disclosed in Section 6.8. In certain embodiments, the size is measured prior to or after lyophilization. In certain embodiments, the size is measured prior to or after reconstitution of the lyophilized formulation.

In certain embodiments, the rAAV particles in the stable formulation is at least about 2%, about 5%, about 7%, about 10%, about 12%, about 15%, about 17%, about 20%, about 25%, about 30%, about 35%, about 40%, about 45%, about 50%, about 100%, about 2 times, about 3 times, about 5 times, about 10 times, about 100 times, or about 1000 times more stable, than compared to the same rAAV particles in a reference formulation when stored at -80°C, -70°C, -20°C, 4°C, 20°C, 25°C, 30°C, 35°C, 37°C or 40 °C. In certain embodiments, the stability of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more infectivity, than compared to the same rAAV particles in a reference formulation when stored at -80°C, - 70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the virus infectivity of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more infectivity than compared to the same rAAV particles in a reference formulation when stored at -80°C, - 70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the virus infectivity of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less aggregation, than compared to the same rAAV particles in a reference formulation when stored at -80°C, - 70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the aggregation of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less aggregation, than compared to the same rAAV particles in a reference formulation when stored at -80°C, - 70°C, -20°C, 4°C, 20°C, 25°C, 30°C, 35°C, 37°C or 40 °C for a period of time, at least for example, at least about 1 week, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the aggregation of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more stable, than compared to the same rAAV particles in a reference formulation when stored at -80°C, -70°C, - 20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, and about 4 years, or about 5 years. In certain embodiments, the stability over a period of time of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times more stable, than compared to the same rAAV particles in a reference formulation when stored at -80°C, -70°C, - 20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the stability over a period of time of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation is at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times higher in vitro relative potency (IVRP), than compared to the same rAAV particles in a reference formulation when stored at -80°C, -70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the in vitro relative potency (IVRP) of the rAAV particles is determined by an assay or assays disclosed in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less free DNA, than compared to the same rAAV particles in a reference formulation when stored at -80°C, - 70°C, -20°C, 4°C, 20°C, 25°C, 30°C, 35°C, 37°C or 40 °C for a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the free DNA of the rAAV particles is determined by an assay or assays disclosed in in Section 6.8.

In certain embodiments, the rAAV particles in the stable formulation has at least 2%, 5%, 7%, 10%, 12%, 15%, 17%, 20%, 25%, 30%, 35%, 40%, 45%, 50%, 100%, 2 times, 3 times, 5 times, 10 times, 100 times, or 1000 times less free DNA, than compared to the same rAAV particles in a reference formulation when stored at -80°C, - 70°C, -20°C, 4°C, 20°C, 25°C, 30°C, 35°C, 37°C or 40 °C for a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the free DNA of the rAAV particles is determined by an assay or assays disclosed in in Section 6.8.

In certain embodiments, the rAAV particles in the stable formulation has at most 20%, 15%, 10%, 8%, 5%, 4%, 3%, 2%, or 1% change in particle size when stored at -80°C, -70°C, -20°C, 4°C, 20°C, 25°C, 30°C, 35°C, 37°C or 40 °C over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years. In certain embodiments, the size of the rAAV particles is determined by an assay or assays disclosed in Section 6.8.

In certain embodiments, the rAAV particles in the stable formulation has at most 20%, 15%, 10%, 8%, 5%, 4%, 3%, 2%, or 1% change in particle size when stored at -80°C, -70°C, -20°C, 4°C, 20°C, 25°C, 30°C, 35°C, 37°C or 40 °C over a period of time, for example, at least about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, about 4 years, or about 5 years, when compared to the same rAAV particles in a reference formulation. In certain embodiments, the size of the rAAV particles is determined by an assay or assays disclosed in Section 6.8.

In certain embodiments, the size of the rAAV particles is determined by an assay or assays disclosed in Section 6.8. In certain embodiments, the reference formulation is DPBS with 0.001% poloxamer 188 buffer.

In certain embodiments, the reference formulation is a formulation not comprising sugar. In certain embodiments, the reference formulation is a formulation not comprising plasticizer.

In certain embodiments, the formulation is frozen to a temperature of about - 20 °C in the process of lyophilization. In certain embodiments, the frozen formulation maintains pH between about pH 6 to about pH 9 when freezing down to -20 °C. In certain embodiments, the frozen formulation maintains a pH value within a range of plus or minus 1 unit of the pH value prior to freezing when freezing down to -20 °C.

In certain embodiments, the glass transition temperature (Tg) of the lyophilized cakes of the formulation is higher than 35 °C.

In certain embodiments, the glass transition temperature of the maximally freeze-concentrated solution (Tg') of the formulation is higher than -40 °C.

In certain embodiments, the moisture content is between about 0.5% and about 1%. In certain embodiments, the moisture content is between about 1% and about 2%. In certain embodiments, the moisture content is between about 2% and about 3%. In certain embodiments, the moisture content is between about 3% and about 4%. In certain embodiments, the moisture content is between about 4% and about 5%.

In certain embodiments, the moisture content is about 0.1%, about 0.2%, about 0.3%, about 0.4%, about 0.5%, about 0.6%, about 0.8%, about 1.0%, about 1.2%, about 1.2%, about 1.3%, about 1.4%, about 1.5%, about 1.6%, about 1.7%, about 1.8%, about 1.9%, about 2.0%, about 3.0%, about 4.0%, or about 5.0%.

As used herein and unless otherwise specified, the term "about" means within plus or minus 10% of a given value or range. In certain embodiments, the term "about" encompasses the exact number recited.

6.8 ASSAYS

The skilled artesian may use the assays as described herein and/or techniques known in the art to study the composition and methods described herein, for example to test the formulations provided herein. The examples provided in Section 8 also demonstrate in more detail how such assays can be used to test the formulations provided herein.

As described in Li et al., 2019 Cell & Gene Therapy Insights, 5(4):537-547, exemplary assays include but are not limited the following: (1) Digital Droplet PCR (ddPCR) for Genome Copy Determinations; (2) Genome Content and % Full Capsid Analysis of AAV by Spectrophotometry; (3) Size Exclusion Chromatography to Determine DNA Distribution and Purity in Capsid; (4) Assessing Capsid Viral Protein Purity Using Capillary Electrophoresis; (5) In Vitro Potency Methods-Relative Infectivity as a Reliable Method for Quantifying Differences in the Infectivity of AAV Vectors in vitro; and (6) Analytical Ultracentrifugation (AUC) to Determine Capsid Empty/Full Ratios and Size Distributions.

### 6.8.1 Temperature Stress Assay

Stable lyophilized formulation of an rAAV product can be produced by lyophilize a pre-lyophilized formulation, wherein the pre-lyophilized formulation is according to any of the formulations provided in Section 6.2. A temperature stress development stability study can be conducted at a selected concentration for the stable lyophilized formulation over a period of time, for example, about 1 weeks, about 2 weeks, about 3 weeks, about 4 weeks, about 1 month, about 2 months, about 3 months, about 4 months, about 5 months, about 6 months, about 7 months, about 8 months, about 9 months, about 10 months, about 11 months, about 12 months, about 15 months, about 18 months, about 24 months, about 2 years, about 3 years, or about 4 years, at -80°C, - 70°C, -20°C, 4°C, 20°C, 25°C, 30°C, 35°C, 37°C or 40 °C to evaluate the relative stability of formulations provided herein. The stable lyophilized formulation is reconstituted after storing the formulation over a period of time.

Assays can be used to assess stability of the stable formulation include but are not limited to in vitro relative potency (IVRP), vector genome concentration (VGC by ddPCR), free DNA by dye fluorescence, dynamic light scattering, appearance, infectivity, potency, and pH.

### 6.8.2 Long-Term Stability Assay

Stable lyophilized formulation of an rAAV product can be produced by lyophilize a pre-lyophilized formulation, wherein the pre-lyophilized formulation is according to any of the formulations provided in Section 6.2. Long-term development stability studies can be carried out for a period of time, for example, about 12 months, about 13 months, about 14 months, about 15 months, about 18 months, about 24 months, about 2 years, about 3 years, or about 4 years, to demonstrate maintenance of in-vitro relative potency and other quality at -80 °C (≤-60 °C) and -20°C (- 25 °C to - 15 °C) in the formulations provided herein.

Assays can be used to assess stability after long-term storage include but are not limited to in vitro relative potency (IVRP), vector genome concentration (VGC by ddPCR), free DNA by dye fluorescence, dynamic light scattering, appearance, infectivity, potency, and pH.

### 6.8.3 In Vitro Relative Potency (IVRP) Assay

To relate the ddPCR GC titer to gene expression, an in vitro bioassay may be performed by transducing HEK293 cells and assaying the cell culture supernatant for anti-VEGF Fab protein levels. HEK293 cells are plated onto three poly-D-lysine-coated 96-well tissue culture plates overnight. The cells are then pre-infected with wild-type human Ad5 virus followed by transduction with three independently prepared serial dilutions of rAAV reference standard and test article, with each preparation plated onto separate plates at different positions. On the third day following transduction, the cell culture media is collected from the plates and measured for VEGF-binding Fab protein levels via ELISA. For the ELISA, 96-well ELISA plates coated with VEGF are blocked and then incubated with the collected cell culture media to capture anti-VEGF Fab produced by HEK293 cells. Fab-specific anti-human IgG antibody is used to detect the VEGF-captured Fab protein. After washing, horseradish peroxidase (HRP) substrate solution is added, allowed to develop, stopped with stop buffer, and the plates are read in a plate reader. The absorbance or OD of the HRP product is plotted versus log dilution, and the relative potency of each test article is calculated relative to the reference standard on the same plate fitted with a four-parameter logistic regression model after passing the parallelism similarity test, using the formula: EC50 reference ÷ EC50 test article. The potency of the test article is reported as a percentage of the reference standard potency, calculated from the weighted average of the three plates.

To relate the ddPCR GC titer to functional gene expression, an in vitro bioassay may be performed by transducing HEK293 cells and assaying for transgene (e.g. enzyme) activity. HEK293 cells are plated onto three 96-well tissue culture plates overnight. The cells are then pre-infected with wild-type human adenovirus serotype 5 virus followed by transduction with three independently prepared serial dilutions of enzyme reference standard and test article, with each preparation plated onto separate plates at different positions. On the second day following transduction, the cells are lysed, treated with low pH to activate the enzyme, and assayed for enzyme activity using a peptide substrate that yields increased fluorescence signal upon cleavage by transgene (enzyme). The fluorescence or RFU is plotted versus log dilution, and the relative potency of each test article is calculated relative to the reference standard on the same plate fitted with a four-parameter logistic regression model after passing the parallelism similarity test, using the formula: EC50 reference ÷ EC50 test article. The potency of the test article is reported as a percentage of the reference standard potency, calculated from the weighted average of the three plates.

IVRP can be measured prior to lyophilization of the pre-lyophilization formulation. IVRP can be measured after reconstitution of a lyophilized formulation. Stability of the formulation can be assessed by comparing the IVRP of the rAAV particles prior to lyophilization with the IVRP of the rAAV particles after reconstitution of the lyophilized formulation. Stability of the formulation can also be assessed by comparing the IVRP of the rAAV particles after reconstitution of the lyophilized formulation after storing the lyophilized formulation over a period of time with the IVRP of the rAAV particles in a reference formulation stored and reconstituted at the same condition.

### 6.8.4 Vector Genome Concentration Assay

Vector genome concentration (GC) can also be evaluated using ddPCR. GC can be measured prior to lyophilization of the pre-lyophilization formulation. GC can be measured after reconstitution of a lyophilized formulation. Stability of the formulation can be assessed by comparing the GC value of the formulation prior to lyophilization with the GC value after reconstitution of the lyophilized formulation. Stability of the formulation can also be assessed by comparing the GC value of the reconstituted formulation after storing the lyophilized formulation over a period of time with the GC value of a reference formulation stored and reconstituted at the same condition.

### 6.8.5 Free DNA Analysis Using Dye Fluorescence Assay

The stability of the formulation can be assessed by rAAV genome release, wherein the rAAV genome release is determined by measuring relative fluorescence in preference of a DNA specific florescent stain. Free DNA can be determined by fluorescence of SYBR® Gold nucleic acid gel stain ('SYBR Gold dye') that is bound to DNA. The fluorescence can be measured using a microplate reader and quantitated with a DNA standard. The results in ng/$\mu$L can be reported.

Two approaches can be used to estimate the total DNA in order to convert the measured free DNA in ng/$\mu$L to a percentage of free DNA. In the first approach the GC/mL (OD) determined by UV-visible spectroscopy was used to estimate the total DNA in the sample, where M is the molecular weight of the DNA and 1E6 is a unit conversion factor:

$$\text{Total DNA (ng/}\mu\text{L) estimated} = 1E6 \times \text{GC/mL (OD)} \times M \text{ (g/mol)}/6.02E23$$

In the second approach, the sample can be heated to 85°C for 20 min with e.g. 0.05% poloxamer 188 and the actual DNA measured in the heated sample by the SYBR Gold dye assay can be used as the total. This therefore has the assumption that all the DNA was recovered and quantitated. Any variation in the conversion of ng/$\mu$L to percentage of free DNA can be captured as a range in the reported results). For trending, either the raw ng/$\mu$L can be used or the percentage determined by a consistent method can be used.

The relative fluorescence level can be measured prior to lyophilization of the pre-lyophilization formulation. The relative fluorescence level can be measured after reconstitution of a lyophilized formulation. Stability of the formulation can be assessed by comparing the relative fluorescence level of the formulation prior to lyophilization with the relative fluorescence level value after reconstitution of the lyophilized formulation. Stability of the formulation can also be assessed by comparing the relative fluorescence level of the reconstituted formulation after storing the lyophilized formulation over a period of time with the relative fluorescence level of a reference formulation stored and reconstituted at the same condition.

### 6.8.6 Size Exclusion Chromatography (SEC)

The stability of the formulation can be assessed by measuring the size of the rAAV particles, wherein the size of the rAAV particles is determined by SEC. SEC can be performed using a Sepax SRT SEC-1000 Peek column (PN 215950P-4630, SN: 8A11982, LN: BT090, 5 $\mu$m 1000A, 4.6x300mm) on Waters Acquity Arc Equipment ID 0447 (C3PO), with a 25 mm pathlength flowcell. The mobile phase can be, for example, 20 mM sodium phosphate, 300 mM NaCl, 0.005% poloxamer 188, pH 6.5, with a flow rate of 0.35 mL/minute for 20 minutes, with the column at ambient temperature. Data collection can be performed with 2 point/second sampling rate and 1.2 nm resolution with 25 point mean smoothing at 214, 260, and 280 nm. The ideal target load can be 1.5E11 GC. The samples can be injected with 50 $\mu$L, about 1/3 of the ideal target or injected with 5 $\mu$L. The size of the rAAV particles can be measured prior to lyophilization of the pre-lyophilization formulation. The size of the rAAV particles can be measured after reconstitution of a lyophilized formulation. Stability of the formulation can be assessed by comparing the size of the rAAV particles of a formulation prior to lyophilization with the size of the rAAV particles of the same formulation after lyophilization and reconsitution. Stability of the formulation can also be assessed by comparing the size of the rAAV particles of the reconstituted formulation after storing the lyophilized formulation over a period of time with the size of the rAAV particles of a reference formulation stored and reconstituted at the same condition.

### 6.8.7 Dynamic Light Scattering (DLS) Assay

The stability of the formulation can be assessed by measuring the level of aggregation of the rAAV particles, wherein the level of aggregation of the rAAV particles is determined by dynamic light scattering (DLS). DLS can be performed on a Wyatt DynaProIII using Corning 3540 384 well plates with a 30 $\mu$L sample volume. Ten acquisitions each for 10 s can be collected per replicate and there were three replicate measurements per sample. The solvent can be set according to the solvent used in the samples, for example 'PBS' for rAAV in dPBS and '4% sucrose' for the rAAV in modified dPBS with sucrose samples. Results not meeting data quality criteria (baseline, SOS, noise, fit) can be 'marked' and excluded from the analysis. The low delay time cutoff can be changed from 1.4 $\mu$s to 10 $\mu$s for the modified dPBS with sucrose samples to eliminate the impact of the sucrose excipient peak at about 1 nm on causing artifactually low cumulants analysis diameter results. The aggregation level of the rAAV particles can be measured prior to lyophilization of the pre-lyophilization formulation. The aggregation levels of the rAAV particles can be measured after reconstitution of a lyophilized formulation.

Exemplary methods are described in Wright et al., 2005, Molecular Therapy 12(1): 171-189

### 6.8.8 Differential Scanning Calorimetry

Differential scanning calorimetry (DSC) is a frequently used thermal analysis technique. DSC measures enthalpy changes in samples due to changes in their physical and chemical properties as a function of temperature or time. Low temperature Differential Scanning Calorimetry (low-temp DSC) can be run using a TA Instruments DSC250. About 20 $\mu$L of sample can be loaded into a Tzero pan and crimped with a Tzero™ Hermetic lid. Samples can be equilibrated at 25 °C for 2 min, then cooled at 5 °C/min to - 60 °C, equilibrated for 2 min, then heated at 5 °C/min to 25 °C. Heat flow data can be collected in conventional mode.

### 6.8.9 Real-time Buffer pH Tracking

The pH of different formulation buffers was monitored with INLAB COOL PRO-ISM low temperature pH probe, which can detect pH down to - 30 °C. One milliliter buffer was placed in 15 mL Falcon tube and then the pH probe was submerged in the buffer. A piece of parafilm was used to seal the gap between Falcon tube and pH probe to avoid contamination and evaporation. The probe along with the Falcon tube was placed in -20 AD freezer. The pH and temperature of the buffer were recorded every 2.5 min for around 20 hour or until the pH versus temperature behavior achieved repeating pattern. The temperature change caused by the automatic defrosting process created a stress condition for buffer pH stability. The pH of the formulation can be measured prior to lyophilization of the pre-lyophilization formulation. The pH of the formulation can be measured after reconstitution of a lyophilized formulation.

### 6.8.10 Density Measurement

The density can be measured with Anton Paar DMA500 densitometer, using water as reference. The densitometer can be washed with water and then methanol, followed by air-drying between samples. The density of the formulation can be measured prior to lyophilization of the pre-lyophilization formulation. The density of the formulation can be measured after reconstitution of a lyophilized formulation.

### 6.8.11 Viscosity Measurement

Viscosity can be measured using methods known in the art, for example methods provide in the United States Pharmacopeia (USP) published in 2019 and previous versions thereof The viscosity of the formulation can be measured prior to lyophilization of the pre-lyophilization formulation. The viscosity of the formulation can be measured after reconstitution of a lyophilized formulation.

### 6.8.12 Virus Infectivity Assay

$TCID_{50}$ infectious titer assay as described in Frangois, et al. Molecular Therapy Methods & Clinical Development (2018) Vol. 10, pp. 223-236 can be used. Relative infectivity assay as described in PCT International Application No. PCT/US19/56042, filed October 14, 2019) can be used. The virus infectivity of the formulation can be measured prior to lyophilization of the pre-lyophilization formulation. The virus infection of the formulation can be measured after reconstitution of a lyophilized formulation.

### 6.8.13 Crystallization and Glass Transition Temperatures

Exemplary methods are described in Croyle et al., 2001, Gene Ther. 8(17): 1281-90
The stability of the formulation can be assessed by measuring the glass transition temperature (Tg) of the lyophilized cakes.
The Tg of a stable lyophilized cakes of the formulation is higher than 35 °C.
Alternatively, the stability of the formulation can be assessed by measuring the glass transition temperature of the maximally freeze-concentrated solution (Tg').
The Tg' of a stable formulation is higher than -40 °C.

## 7. TABLE OF SEQUENCES

| SEQ ID NO: | Description | Sequence |
|---|---|---|
| 1 | AAV1 | MAADGYLPDWLEDNLSEGIREWWDLKPGAPKPKANQQKQD DGRGLVLPGYKYLGPFNGLDKGEPVNAADAAALEHDKAYD QQLKAGDNPYLRYNHADAEFQERLQEDTSFGGNLGRAVFQA KKRVLEPLGLVEEGAKTAPGKKRPVEQSPQEPDSSSGIGKTG QQPAKKRLNFGQTGDSESVPDPQPLGEPPATPAAVGPTTMAS GGGAPMADNNEGADGVGNASGNWHCDSTWLGDRVITTSTR TWALPTYNNHLYKQISSASTGASNDNHYFGYSTPWGYFDFN RFHCHFSPRDWQRLINNNWGFRPKRLNFKLFNIQVKEVTTND GVTTIANNLTSTVQVFSDSEYQLPYVLGSAHQGCLPPFPADVF MIPQYGYLTLNNGSQAVGRSSFYCLEYFPSQMLRTGNNFTFS YTFEEVPFHSSYAHSQSLDRLMNPLIDQYLYYLNRTQNQSGS AQNKDLLFSRGSPAGMSVQPKNWLPGPCYRQQRVSKTKTDN NNSNFTWTGASKYNLNGRESIINPGTAMASHKDDEDKFFPMS GVMIFGKESAGASNTALDNVMITDEEEIKATNPVATERFGTV AVNFQSSSTDPATGDVHAMGALPGMVWQDRDVYLQGPIWA KIPHTDGHFHPSPLMGGFGLKNPPPQILIKNTPVPANPPAEFSA TKFASFITQYSTGQVSVEIEWELQKENSKRWNPEVQYTSNYA KSANVDFTVDNNGLYTEPRPIGTRYLTRPL |

(continued)

| SEQ ID NO: | Description | Sequence |
|---|---|---|
| 2 | AAV2 | MAADGYLPDWLEDTLSEGIRQWWKLKPGPPPPKPAERHKDDSRGLVLPGYKYLGPFNGLDKGEPVNEADAAALEHDKAYDRQLDSGDNPYLKYNHADAEFQERLKEDTSFGGNLGRAVFQAKKRVLEPLGLVEEPVKTAPGKKRPVEHSPVEPDSSSGTGKAGQQPARKRLNFGQTGDADSVPDPQPLGQPPAAPSGLGTNTMATGSGAPMADNNEGADGVGNSSGNWHCDSTWMGDRVITTSTRTWALPTYNNHLYKQISSQSGASNDNHYFGYSTPWGYFDFNRFHCHFSPRDWQRLINNNWGFRPKRLNFKLFNIQVKEVTQNDGTTTIANNLTSTVQVFTDSEYQLPYVLGSAHQGCLPPFPADVFMVPQYGYLTLNNGSQAVGRSSFYCLEYFPSQMLRTGNNFTFSYTFEDVPFHSSYAHSQSLDRLMNPLIDQYLYYLSRTNTPSGTTTQSRLQFSQAGASDIRDQSRNWLPGPCYRQQRVSKTSADNNNSEYSWTGATKYHLNGRDSLVNPGPAMASHKDDEEKFFPQSGVLIFGKQGSEKTNVDIEKVMITDEEEIRTTNPVATEQYGSVSTNLQRGNRQAATADVNTQGVLPGMVWQDRDVYLQGPIWAKIPHTDGHFHPSPLMGGFGLKHPPPQILIKNTPVPANPSTTFSAAKFASFITQYSTGQVSVEIEWELQKENSKRWNPEIQYTSNYNKSVNVDFTVDTNGVYSEPRPIGTRYLTRNL |
| 3 | AAV3-3 | MAADGYLPDWLEDNLSEGIREWWALKPGVPQPKANQQHQDNRRGLVLPGYKYLGPGNGLDKGEPVNEADAAALEHDKAYDQQLKAGDNPYLKYNHADAEFQERLQEDTSFGGNLGRAVFQAKKRILEPLGLVEEAAKTAPGKKGAVDQSPQEPDSSSGVGKSGKQPARKRLNFGQTGDSESVPDPQPLGEPPAAPTSLGSNTMASGGGAPMADNNEGADGVGNSSGNWHCDSQWLGDRVITTSTRTWALPTYNNHLYKQISSQSGASNDNHYFGYSTPWGYFDFNRFHCHFSPRDWQRLINNNWGFRPKKLSFKLFNIQVRGVTQNDGTTTIANNLTSTVQVFTDSEYQLPYVLGSAHQGCLPPFPADVFMVPQYGYLTLNNGSQAVGRSSFYCLEYFPSQMLRTGNNFQFSYTFEDVPFHSSYAHSQSLDRLMNPLIDQYLYYLNRTQGTTSGTTNQSRLLFSQAGPQSMSLQARNWLPGPCYRQQRLSKTANDNNNSNFPWTAASKYHLNGRDSLVNPGPAMASHKDDEEKFFPMHGNLIFGKEGTTASNAELDNVMITDEEEIRTTNPVATEQYGTVANNLQSSNTAPTTGTVNHQGALPGMVWQDRDVYLQGPIWAKIPHTDGHFHPSPLMGGFGLKHPPPQIMIKNTPVPANPPTTFSPAKFASFITQYSTGQVSVEIEWELQKENSKRWNPEIQYTSNYNKSVNVDFTVDTNGVYSEPRPIGTRYLTRNL |

(continued)

| SEQ ID NO: | Description | Sequence |
|---|---|---|
| 4 | AAV4-4 | MTDGYLPDWLEDNLSEGVREWWALQPGAPKPKANQQHQDN ARGLVLPGYKYLGPGNGLDKGEPVNAADAAALEHDKAYDQ QLKAGDNPYLKYNHADAEFQQRLQGDTSFGGNLGRAVFQA KKRVLEPLGLVEQAGETAPGKKRPLIESPQQPDSSTGIGKKGK QPAKKKLVFEDETGAGDGPPEGSTSGAMSDDSEMRAAAGGA AVEGGQGADGVGNASGDWHCDSTWSEGHVTTTSTRTWVLP TYNNHLYKRLGESLQSNTYNGFSTPWGYFDFNRFHCHFSPRD WQRLINNNWGMRPKAMRVKIFNIQVKEVTTSNGETTVANNL TSTVQIFADSSYELPYVMDAGQEGSLPPFPNDVFMVPQYGYC GLVTGNTSQQQTDRNAFYCLEYFPSQMLRTGNNFEITYSFEK VPFHSMYAHSQSLDRLMNPLIDQYLWGLQSTTTGTTLNAGT ATTNFTKLRPTNFSNFKKNWLPGPSIKQQGFSKTANQNYKIPA TGSDSLIKYETHSTLDGRWSALTPGPPMATAGPADSKFSNSQ LIFAGPKQNGNTATVPGTLIFTSEEELAATNATDTDMWGNLP GGDQSNSNLPTVDRLTALGAVPGMVWQNRDIYYQGPIWAKI PHTDGHFHPSPLIGGFGLKHPPPQIFIKNTPVPANPATTFSSTPV NSFITQYSTGQVSVQIDWEIQKERSKRWNPEVQFTSNYGQQN SLLWAPDAAGKYTEPRAIGTRYLTHHL |
| 5 | AAV5 | MSFVDHPPDWLEEVGEGLREFLGLEAGPPKPKPNQQHQDQA RGLVLPGYNYLGPGNGLDRGEPVNRADEVAREHDISYNEQL EAGDNPYLKYNHADAEFQEKLADDTSFGGNLGKAVFQAKK RVLEPFGLVEEGAKTAPTGKRIDDHFPKRKKARTEEDSKPSTS SDAEAGPSGSQQLQIPAQPASSLGADTMSAGGGGPLGDNNQ GADGVGNASGDWHCDSTWMGDRVVTKSTRTWVLPSYNNH QYREIKSGSVDGSNANAYFGYSTPWGYFDFNRFHSHWSPRD WQRLINNYWGFRPRSLRVKIFNIQVKEVTVQDSTTTIANNLTS TVQVFTDDDYQLPYVVGNGTEGCLPAFPPQVFTLPQYGYATL NRDNTENPTERSSFFCLEYFPSKMLRTGNNFEFTYNFEEVPFH SSFAPSQNLFKLANPLVDQYLYRFVSTNNTGGVQFNKNLAGR YANTYKNWFPGPMGRTQGWNLGSGVNRASVSAFATTNRME LEGASYQVPPQPNGMTNNLQGSNTYALENTMIFNSQPANPGT TATYLEGNMLITSESETQPVNRVAYNVGGQMATNNQSSTTA PATGTYNLQEIVPGSVWMERDVYLQGPIWAKIPETGAHFHPS PAMGGFGLKHPPPMMLIKNTPVPGNITSFSDVPVSSFITQYST GQVTVEMEWELKKENSKRWNPEIQYTNNYNDPQFVDFAPDS TGEYRTTRPIGTRYLTRPL |

(continued)

| SEQ ID NO: | Description | Sequence |
|---|---|---|
| 6 | AAV6 | MAADGYLPDWLEDNLSEGIREWWDLKPGAPKPKANQQKQD DGRGLVLPGYKYLGPFNGLDKGEPVNAADAAALEHDKAYD QQLKAGDNPYLRYNHADAEFQERLQEDTSFGGNLGRAVFQA KKRVLEPFGLVEEGAKTAPGKKRPVEQSPQEPDSSSGIGKTG QQPAKKRLNFGQTGDSESVPDPQPLGEPPATPAAVGPTTMAS GGGAPMADNNEGADGVGNASGNWHCDSTWLGDRVITTSTR TWALPTYNNHLYKQISSASTGASNDNHYFGYSTPWGYFDFN RFHCHFSPRDWQRLINNNWGFRPKRLNFKLFNIQVKEVTTND GVTTIANNLTSTVQFSDSEYQLPYVLGSAHQGCLPPFPADVF MIPQYGYLTLNNGSQAVGRSSFYCLEYFPSQMLRTGNNFTFS YTFEDVPFHSSYAHSQSLDRLMNPLIDQYLYYLNRTQNSGS AQNKDLLFSRGSPAGMSVQPKNWLPGPCYRQQRVSKTKTDN NNSNFTWTGASKYNLNGRESIINPGTAMASHKDDKDKFFPMS GVMIFGKESAGASNTALDNVMITDEEEIKATNPVATERFGTV AVNLQSSSTDPATGDVHVMGALPGMVWQDRDVYLQGPIWA KIPHTDGHFHPSPLMGGFGLKHPPPQILIKNTPVPANPPAEFSA TKFASFITQYSTGQVSVEIEWELQKENSKRWNPEVQYTSNYA KSANVDFTVDNNGLYTEPRPIGTRYLTRPL |
| 7 | AAV7 | MAADGYLPDWLEDNLSEGIREWWDLKPGAPKPKANQQKQD NGRGLVLPGYKYLGPFNGLDKGEPVNAADAAALEHDKAYD QQLKAGDNPYLRYNHADAEFQERLQEDTSFGGNLGRAVFQA KKRVLEPLGLVEEGAKTAPAKKRPVEPSPQRSPDSSTGIGKKG QQPARKRLNFGQTGDSESVPDPQPLGEPPAAPSSVGSGTVAA GGGAPMADNNEGADGVGNASGNWHCDSTWLGDRVITTSTR TWALPTYNNHLYKQISSETAGSTNDNTYFGYSTPWGYFDFNR FHCHFSPRDWQRLINNNWGFRPKKLRFKLFNIQVKEVTTNDG VTTIANNLTSTIQFSDSEYQLPYVLGSAHQGCLPPFPADVFM IPQYGYLTLNNGSQSVGRSSFYCLEYFPSQMLRTGNNFEFSYS FEDVPFHSSYAHSQSLDRLMNPLIDQYLYYLARTQSNPGGTA GNRELQFYQGGPSTMAEQAKNWLPGPCFRQQRVSKTLDQNN NSNFAWTGATKYHLNGRNSLVNPGVAMATHKDDEDRFFPSS GVLIFGKTGATNKTTLENVLMTNEEEIRPTNPVATEEYGIVSS NLQAANTAAQTQVVNNQGALPGMVWQNRDVYLQGPIWAKI PHTDGNFHPSPLMGGFGLKHPPPQILIKNTPVPANPPEVFTPA KFASFITQYSTGQVSVEIEWELQKENSKRWNPEIQYTSNFEKQ TGVDFAVDSQGVYSEPRPIGTRYLTRNL |

(continued)

| SEQ ID NO: | Description | Sequence |
|---|---|---|
| 8 | AAV8 | MAADGYLPDWLEDNLSEGIREWWALKPGAPKPKANQQKQD DGRGLVLPGYKYLGPFNGLDKGEPVNAADAAALEHDKAYD QQLQAGDNPYLRYNHADAEFQERLQEDTSFGGNLGRAVFQA KKRVLEPLGLVEEGAKTAPGKKRPVEPSPQRSPDSSTGIGKKG QQPARKRLNFGQTGDSESVPDPQPLGEPPAAPSGVGPNTMAA GGGAPMADNNEGADGVGSSSGNWHCDSTWLGDRVITTSTR TWALPTYNNHLYKQISNGTSGGATNDNTYFGYSTPWGYFDF NRFHCHFSPRDWQRLINNNWGFRPKRLSFKLFNIQVKEVTQN EGTKTIANNLTSTIQVFTDSEYQLPYVLGSAHQGCLPPFPADV FMIPQYGYLTLNNGSQAVGRSSFYCLEYFPSQMLRTGNNFQF TYTFEDVPFHSSYAHSQSLDRLMNPLIDQYLYYLSRTQTTGG TANTQTLGFSQGGPNTMANQAKNWLPGCYRQQRVSTTTGQ NNNSNFAWTAGTKYHLNGRNSLANPGIAMATHKDDEERFFP SNGILIFGKQNAARDNADYSDVMLTSEEEIKTTNPVATEEYGI VADNLQQQNTAPQIGTVNSQGALPGMVWQNRDVYLQGPIW AKIPHTDGNFHPSPLMGGFGLKHPPPQILIKNTPVPADPPTTFN QSKLNSFITQYSTGQVSVEIEWELQKENSKRWNPEIQYTSNYY KSTSVDFAVNTEGVYSEPRPIGTRYLTRNL |
| 9 | hu31 | MAADGYLPDWLEDTLSEGIRQWWKLKPGPPPPKPAERHKDD SRGLVLPGYKYLGPGNGLDKGEPVNAADAAALEHDKAYDQ QLKAGDNPYLKYNHADAEFQERLKEDTSFGGNLGRAVFQAK KRLLEPLGLVEEAAKTAPGKKRPVEQSPQEPDSSAGIGKSGSQ PAKKKLNFGQTGDTESVPDPQPIGEPPAAPSGVGSLTMASGG GAPVADNNEGADGVGSSSGNWHCDSQWLGDRVITTSTRTW ALPTYNNHLYKQISNSTSGGSSNDNAYFGYSTPWGYFDFNRF HCHFSPRDWQRLINNNWGFRPKRLNFKLFNIQVKEVTDNNG VKTIANNLTSTVQVFTDSDYQLPYVLGSAHEGCLPPFPADVF MIPQYGYLTLNDGGQAVGRSSFYCLEYFPSQMLRTGNNFQFS YEFENVPFHSSYAHSQSLDRLMNPLIDQYLYYLSKTINGSGQN QQTLKFSVAGPSNMAVQGRNYIPGPSYRQQRVSTTVTQNNN SEFAWPGASSWALNGRNSLMNPGPAMASHKEGEDRFFPLSG SLIFGKQGTGRDNVDADKVMITNEEEIKTTNPVATESYGQVA TNHQSAQAQAQTGWVQNQGILPGMVWQDRDVYLQGPIWA KIPHTDGNFHPSPLMGGFGMKHPPPQILIKNTPVPADPPTAFN KDKLNSFITQYSTGQVSVEIEWELQKENSKRWNPEIQYTSNY YKSNNVEFAVSTEGVYSEPRPIGTRYLTRNL |
| | | |

(continued)

| SEQ ID NO: | Description | Sequence |
|---|---|---|
| 10 | hu32 | MAADGYLPDWLEDTLSEGIRQWWKLKPGPPPPKPAERHKDD SRGLVLPGYKYLGPGNGLDKGEPVNAADAAALEHDKAYDQ QLKAGDNPYLKYNHADAEFQERLKEDTSFGGNLGRAVFQAK KRLLEPLGLVEEAAKTAPGKKRPVEQSPQEPDSSAGIGKSGSQ PAKKKLNFGQTGDTESVPDPQPIGEPPAAPSGVGSLTMASGG GAPVADNNEGADGVGSSSGNWHCDSQWLGDRVITTSTRTW ALPTYNNHLYKQISNSTSGGSSNDNAYFGYSTPWGYFDFNRF HCHFSPRDWQRLINNNWGFRPKRLNFKLFNIQVKEVTDNNG VKTIANNLTSTVQVFTDSDYQLPYVLGSAHEGCLPPFPADVF MIPQYGYLTLNDGSQAVGRSSFYCLEYFPSQMLRTGNNFQFS YEFENVPFHSSYAHSQSLDRLMNPLIDQYLYYLSKTINGSGQN QQTLKFSVAGPSNMAVQGRNYIPGPSYRQQRVSTTVTQNNN SEFAWPGASSWALNGRNSLMNPGPAMASHKEGEDRFFPLSG SLIFGKQGTGRDNVDADKVMITNEEEIKTTNPVATESYGQVA TNHQSAQAQAQTGWVQNQGILPGMVWQDRDVYLQGPIWA KIPHTDGNFHPSPLMGGFGMKHPPPQILIKNTPVPADPPTAFN KDKLNSFITQYSTGQVSVEIEWELQKENSKRWNPEIQYTSNY YKSNNVEFAVNTEGVYSEPRPIGTRYLTRNL |
| 11 | AAV9 | MAADGYLPDWLEDNLSEGIREWWALKPGAPQPKANQQHQD NARGLVLPGYKYLGPGNGLDKGEPVNAADAAALEHDKAYD QQLKAGDNPYLKYNHADAEFQERLKEDTSFGGNLGRAVFQA KKRLLEPLGLVEEAAKTAPGKKRPVEQSPQEPDSSAGIGKSG AQPAKKRLNFGQTGDTESVPDPQPIGEPPAAPSGVGSLTMAS GGGAPVADNNEGADGVGSSSGNWHCDSQWLGDRVITTSTRT WALPTYNNHLYKQISNSTSGGSSNDNAYFGYSTPWGYFDFN RFHCHFSPRDWQRLINNNWGFRPKRLNFKLFNIQVKEVTDNN GVKTIANNLTSTVQVFTDSDYQLPYVLGSAHEGCLPPFPADV FMIPQYGYLTLNDGSQAVGRSSFYCLEYFPSQMLRTGNNFQF SYEFENVPFHSSYAHSQSLDRLMNPLIDQYLYYLSKTINGSGQ NQQTLKFSVAGPSNMAVQGRNYIPGPSYRQQRVSTTVTQNN NSEFAWPGASSWALNGRNSLMNPGPAMASHKEGEDRFFPLS GSLIFGKQGTGRDNVDADKVMITNEEEIKTTNPVATESYGQV ATNHQSAQAQAQTGWVQNQGILPGMVWQDRDVYLQGPIW AKIPHTDGNFHPSPLMGGFGMKHPPPQILIKNTPVPADPPTAF NKDKLNSFITQYSTGQVSVEIEWELQKENSKRWNPEIQYTSN YYKSNNVEFAVNTEGVYSEPRPIGTRYLTRNL |

## 8. EXAMPLES

### 8.1 EXAMPLE 1: Formulation and delivery challenges for AAV gene therapy products.

Adeno-associated viruses (AAV) are among the most actively investigated gene therapy vectors, boosted by encouraging clinical results and the promise of increasing commercial approvals. In addition to demonstrating safety and efficacy, an AAV product must remain stable and potent during manufacture, shipping, storage, and administration. For the commercialization of AAV, it is necessary to identify formulations that offer stability for extended periods of time. It would be advantageous to identify formulations that are stable under refrigerated conditions or at room temperature in order to avoid the need to ship and store under frozen conditions. For products that must be frozen, the formulation must be able to withstand the stresses introduced by freezing.

Dried formulations offer the potential to improve stability by significantly reducing the rate of degradation pathways that occur in solution. Lyophilized, or freeze-dried, formulations must be stable during the freezing and drying steps. Further, formulations undergoing lyophilization should consistently yield elegant cakes; formulation components must be chosen carefully to mitigate the risk of product collapse from the lyophilization process.

AAVs present unique formulation challenges compared to traditional protein therapeutics. These include the complex nature of AAV particles, analytics, fill/finish challenges for large molecules, lack of published stability/formulation information, and limitations posed by routes of administration.

AAV particles are complex macromolecular assemblies of different proteins and nucleic acids that are susceptible to different chemical and physical degradation pathways. Information regarding formulations developed for a single macromolecular entity (e.g., a polypeptide such as an antibody, or a nucleic acid) does not provide a reasonable expectation that AAV particles comprising both polypeptide and nucleic acid components will also be stable in the same formulation. The lack of GMP compliant analytics suitable for industrial use also hampers AAV formulation development. There is a heavy reliance on imprecise bioassays in the field. AAV specific degradation pathways need to be identified. Viral particle aggregation prose challenges for the fill/finish processes as they can lead to losses after 0.2 $\mu$m filtration. There is a shortage of published stability/formulation information on AAV particles. This is in part due to the heavy reliance on the common default of frozen storage ($\leq$ -60°C) in PBS based buffers. There is also very limited information on formulations for certain routes of administration, such as subretinal and intrathecal, which are planned for some rAAV products. In sum, these and other challenges make the development of a novel stable AAV formulation a highly unpredictable venture.

### 8.2 Example 2. Aggregation is an AAV formulation challenge.

AAV particle aggregation has been described, with a solution ionic strength of at least 200 mM reported to be required to prevent this aggregation. U.S. Patent 9,051,542.

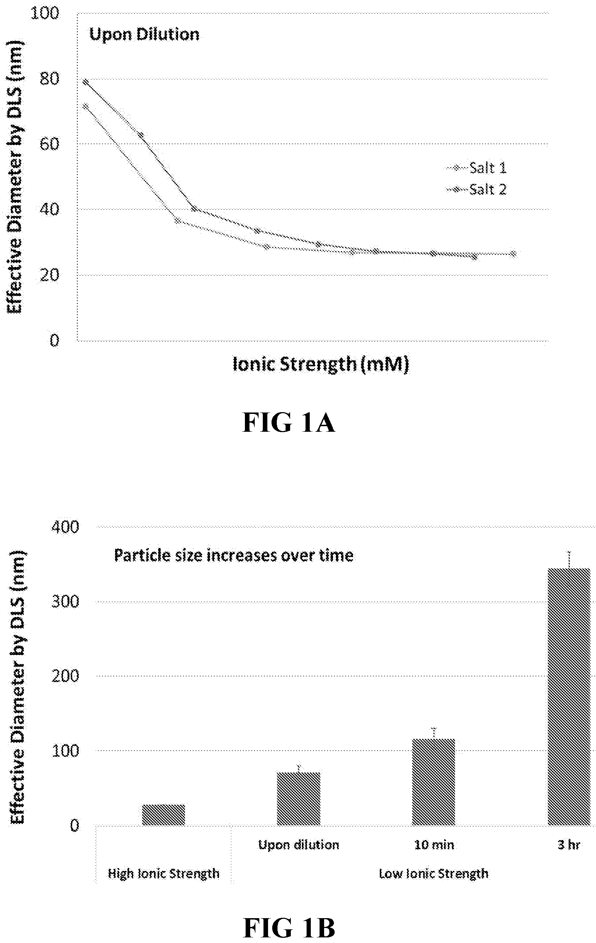

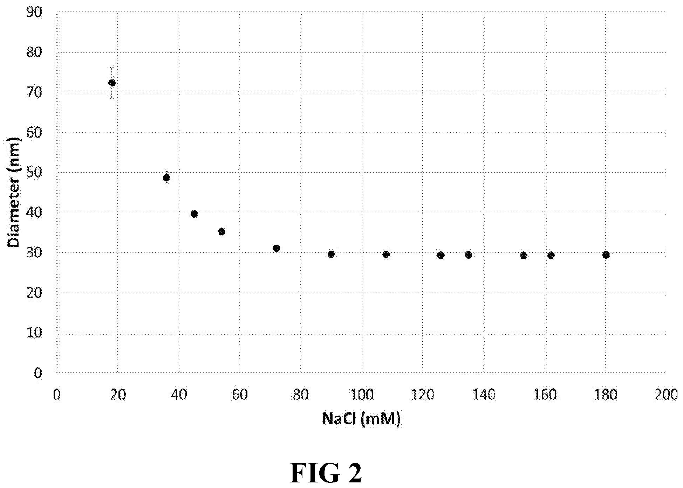

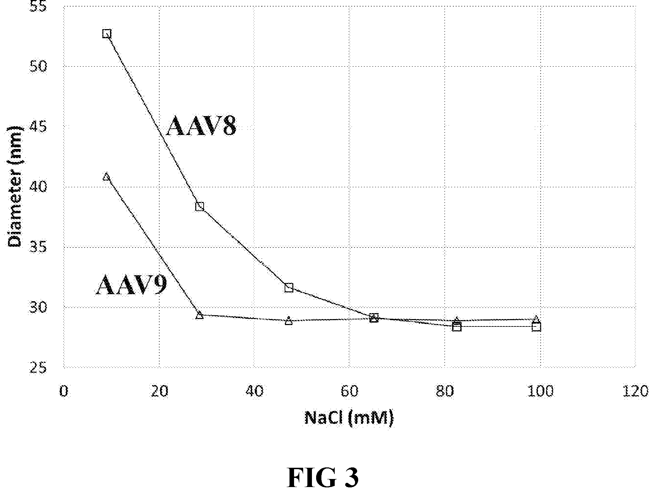

A minimum ionic strength is required to prevent aggregation or self-association of AAV particles. (Figure 1A and 1B). It was found that the minimum ionic strength required to prevent particle aggregation or self-association is AAV serotype dependent. AAV8 aggregation could be prevented at ionic strengths lower than 200 mM (Figure 2), and lower ionic strength is required for AAV9 compared to AAV8 (Figure 3). The ability to formulate with less salt is advantageous for frozen and dried formulations. However, the serotype specific differences in particle aggregation indicate that different formulations may be needed for different serotypes.

### 8.3 Example 3. rAAV genome release in frozen formulations is a formulation challenge.

Preventing aggregation was necessary but not sufficient to ensure a stable drug product. The relative potency of viral particles stored at 25°C in a formulation capable of preventing particle aggregation was significantly lower than the relative potency of viral particles stored in the same formulation at -80°C. This indicates the existence of additional degradation pathways for viral particles in solution.

It was found that crystallization of formulation components during freezing promotes loss of vector DNA from AAV vector particles. This type of vector DNA loss and its application for formulation development has not been reported previously. This DNA loss could be mitigated by either of the following two formulation approaches: (1) formulating with a non-crystallizing salt instead of a salt that crystallizes during freezing and (2) including a component in the formulation (e.g., a sugar) that inhibits the crystallization.

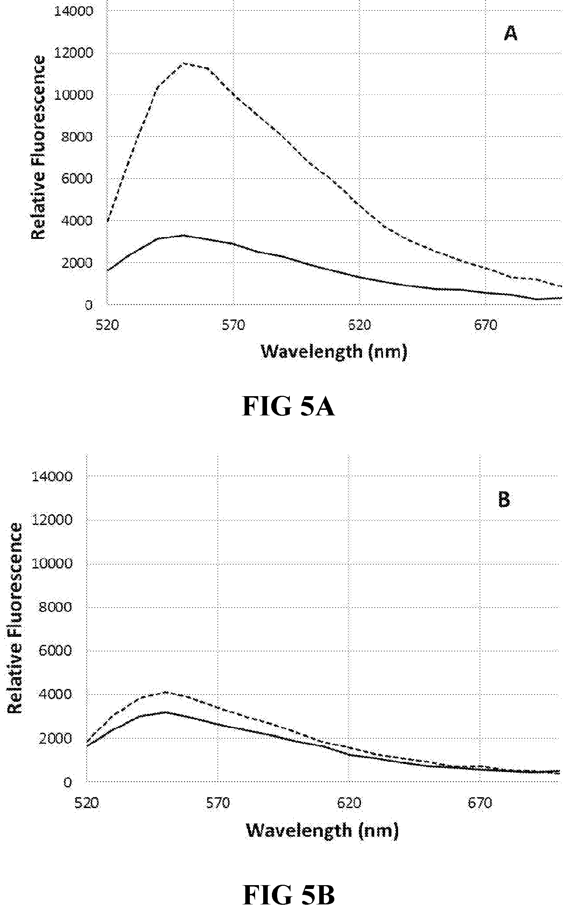

Increased viral genome release was detected after 3 freeze-thaw cycles compared to the control. Figure 5. The extent of genome release was affected by the type of salt in the formulation. Freeze-thaw cycles in the presence of NaCl led to more genome release than freeze-thaw cycles in the presence of sodium citrate. Figure 5 shows binding of SYBR gold to the DNA that is released in the presence of NaCl (A), which crystallizes during freezing, but is minimal in sodium citrate (B), which remains amorphous.

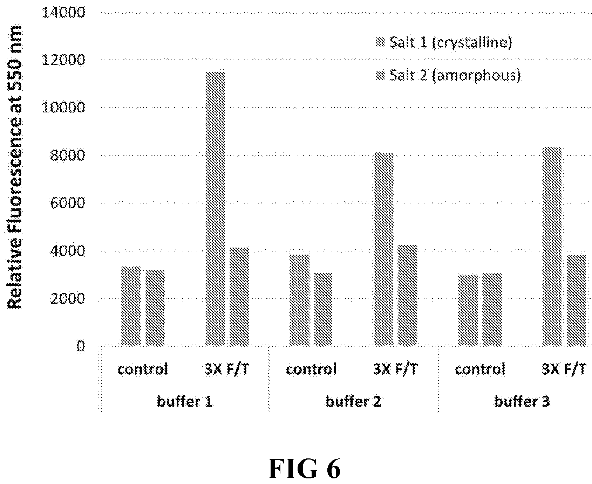

Crystalline salt induced viral genome release upon freezing was examined in Tris, sodium phosphate, and histidine buffers. Figure 6. In each of these buffers the presence of NaCl lead to more viral genome release was detected in the presence of NaCl than in the presence of amorphous sodium citrate.

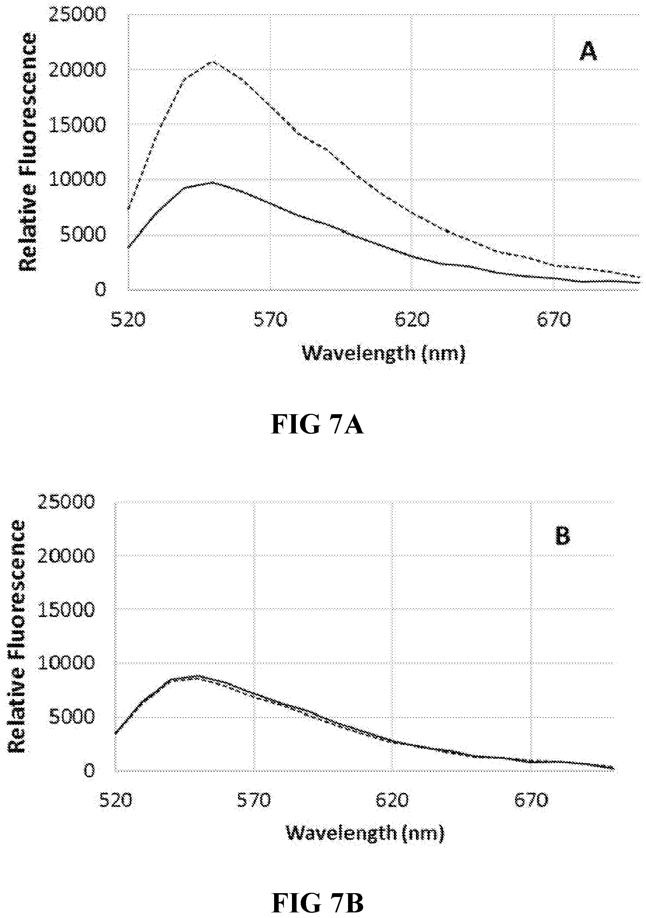

Inclusion of a component in the formulation (e.g., a sugar) that inhibits crystallization reduces viral genome release upon freezing. Viral genome release was also detected after 10 freeze-thaw cycles in PBS. Figure 7A. The inclusion of 4% sucrose in the formulation prevented viral genome release upon freezing. Figure 7B.

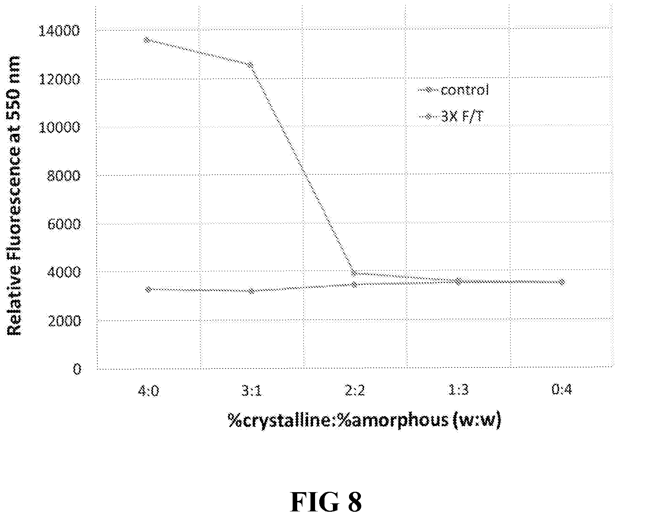

Addition of amorphous sugar inhibited co-solute crystallization leading to less DNA release. Figure 8. AAV was formulated using different mass ratios of mannitol and sucrose, including 3 parts mannitol to 1 part sucrose, 2 part mannitol to 2 part sucrose, 1 part sucrose to 3 parts mannitol and 4 parts sucrose with no mannitol and 4 parts mannitol with no sucrose. After three freeze/thaws, DNA release was reduced as the ratio of sucrose (which is amorphous) to mannitol (which is crystalline) increased. At higher ratios of sucrose to mannitol, free DNA release was completely inhibited as evidenced by equivalence to an unfrozen control.

### 8.4 Example 4. Lyophilization of AAVs presents unique challenges.

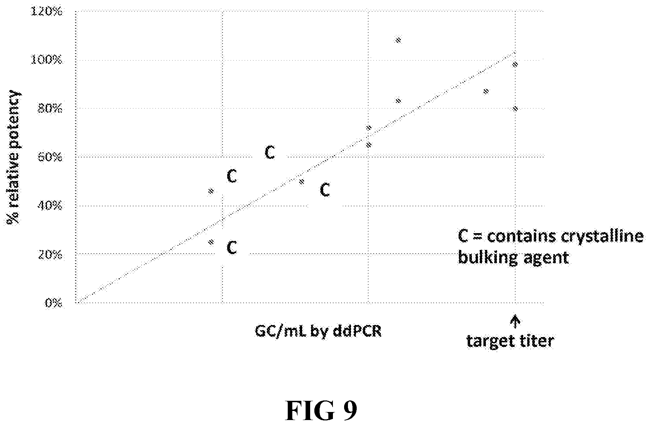

As discussed above, a minimum solution ionic strength is required to prevent particle aggregation. During lyophilization, salts generally decrease collapse temperature unless they are completely crystallized. Crystallization, however, favors DNA release upon freezing. And genome copy (GC) loss leads to concurrent loss in relative potency post-lyophilization. Figure 9. A lyophilization process for AAV particles must find a way to prevent crystallization of formulation components without decreasing collapse temperature to unacceptable levels. A number of different formulations comprising a buffer (Tris, phosphate, and histidine), salt (NaCl and sodium citrate), and excipient (mannitol, sucrose and glycerol) were tested. All formulations tested comprised Pluronic F-68.

Glass transition temperature of the maximally freeze-concentrated solution ($T_g'$) is a parameter that closely correlates with collapse temperature. The $T_g'$ in sucrose/citrate formulations was found to be higher than the individual $T_g'$ values of pure sucrose (-32°C) (Chang and Randall, Cryobiology, 29: 632-656 (1992)) and sodium citrate (-43°C) (Izutsu et al, Chem Pharm Bull, 57: 1231-1236 (2009)). This result was presumably attributable to an interaction between the two molecules. Kets et al, Cryobiology, 48: 46-54 (2004). This property of sucrose/citrate formulations makes them more advantageous for lyophilization process development while also providing the required ionic strength to prevent AAV aggregation.

### 8.5 Example 5. Lyophilization of AAVs present unique challenges.

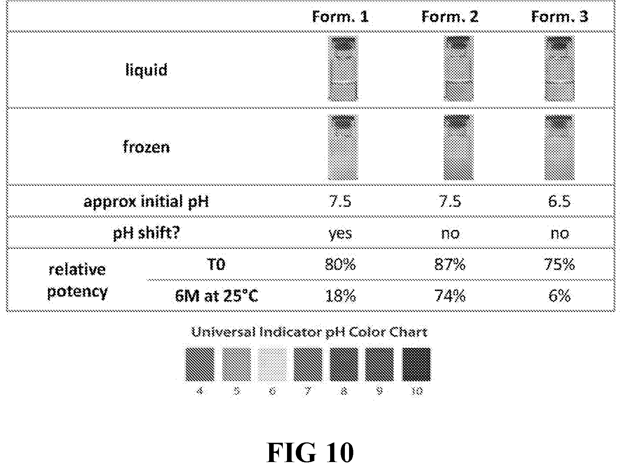

pH of the frozen solutions impacts stability of the lyophilized formulations. Stability of 3 lyophilized rAAV formulations was assessed by measuring relative potency after 6 months storage at 25°C. Figure 10. Formulations 1, 2, and 3 comprised phosphate buffer, Tris buffer, and histidine buffer, respectively. Formulation 1 underwent a pH shift from pH 7.5 to a more acidic pH upon freezing, as indicated by the color difference between the liquid and frozen formulations. The pH of Formulations 2 (pH 7.5) and 3 (pH 6.5) remained stable. Relative potency of Formulations 1 and 3 decreased significantly more after 6 months of storage at 25°C than the relative potency of Formulation 2.

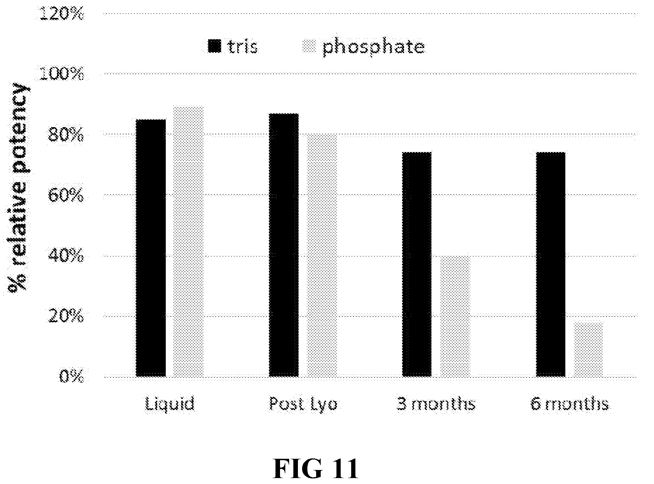

The stability of lyophilized AAV8 formulations comprising Tris or phosphate buffer was compared. Percent relative potency of the formulations in a non-lyophilized control ('Liquid'), after lyophilization ('Post Lyo'), and after 3 months and 6 months room temperature storage is shown in Figure 11. The relative potency of the phosphate buffer formulation declined significantly more the relative potency of the Tris formulation. Phosphate buffers undergo acidic pH shifts during freezing.

### 8.6 Example 6. Residual moisture content affects rAAV stability.

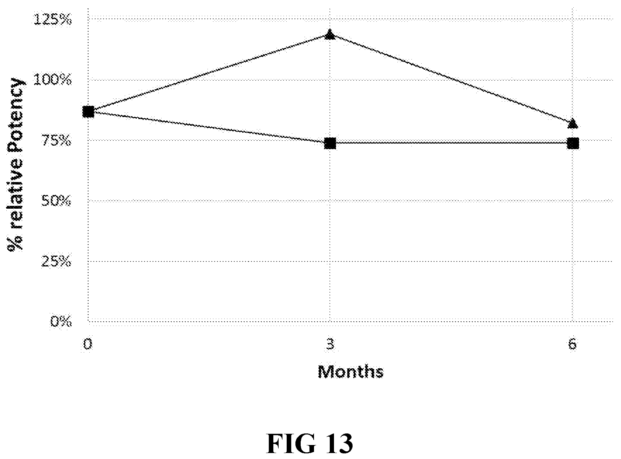

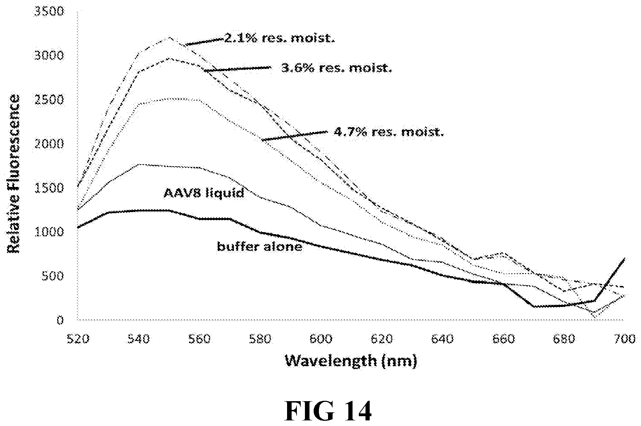

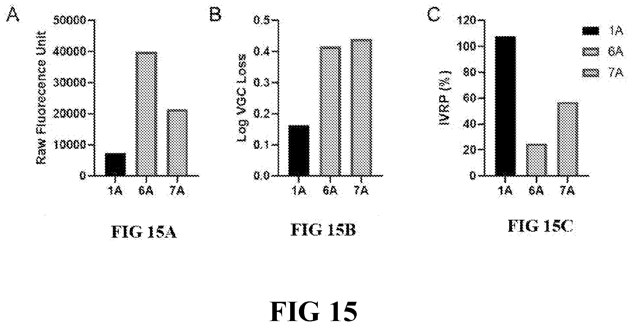

Stability data for lyophilized formulations comprising different residual moisture levels was generated. Figures 13 and 14. Highest stability was found at intermediate moisture levels. Drying as much as possible does not necessarily result in the best stability. Over-drying leads to increased rAAV genome release from the rAAV particles. Figure 15.

Excess residual moisture can stabilize the rAAV particles via a plasticization mechanism of the lyophilized cake. Therefore, plasticizers or stabilizers such as glycerol can be used in the formulation as a substitute for higher residual moisture. Use of a plasticizer or stabilizer, for example glycerol, provides the benefits of improved stability while also allowing for a drying process that simply targets the removal of the most amount of water.

### 8.7 Example 7. Unique excipient composition is required to stabilize AAV in lyophilized formulation.

To evaluate the impact of the buffer system, plasticizer, and salt/bulking agent, five different formulations were compared for Lyophilization Study 1 (Table 1). The vector genome content (VGC)/viral titer and potency data after one month at 40 °C stress study, and 12 months at 25 °C and 5 °C were compared in Table 2. The formulations and their concentrations listed in the Table 2 are pre-lyophilization excipient concentrations. All formulations buffered with sodium phosphate (Formulations 1, 2, 3, and 4) showed a decrease in potency after 1 month at 40 °C (<5% remaining) and 12 months at 25 °C (<80% remaining). Formulation 5 (buffered with TRIS) showed much higher potency after 1 month at 40°C (35%) and 12 months at 5°C (90%) when compared to Formulation 1 and the only difference between the two formulations was the buffer system, suggesting tris buffer is superior over phosphate buffer for lyophilization of AAV. Based on this study, Tris was selected as the base buffer system.

In addition, the mannitol containing formulations (Formulation 3 and 4) showed much lower VGC despite lower moisture content. The use of the crystalline bulking agent, mannitol, an excipient generally used for improving the cake stability and structure and the lyophilization process design (i.e. ability to perform primary drying at a higher temperature without collapse) was destabilizing to the AAV in this study. Considering the moisture content of Formulation 1 (2.9%) was higher than Formulation 3 and 4 (1.2% and 1.4%), lyophilized cakes of different formulations with similar moisture content was generated in Lyophilization Study 2 to further investigate the mechanism of mannitol destabilizing effect on AAV.

Table 1. Formulation compositions examined in Lyophilization Study 1

| | Formulation# | | | | |
|---|---|---|---|---|---|
| Component | 1 | 2 | 3 | 4 | 5 |
| Buffer | 5 mM NaPi pH 7.1 | | | 10 mM NaPi pH 7.1 | 5 mM tris pH 7.5 |
| Salt | 20 mM Na citrate | | | 50 mM NaCl | 20 mM Na citrate |
| Cryo/ Lyoprotectant | 210 mM sucrose | | 14.6 mM sucrose | | 210 mM sucrose |
| Surfactant | 0.002% (w/v) P188 | | | | |
| Bulking Agent | NA | NA | 210 mM mannitol | | NA |
| Plasticizer | NA | 0.25% (w/v) glycerol | NA | NA | NA |

Table 2. Lyophilization Study 1 stability data summary

| mulation# | Moisture (%) | VGC (GC/mL) | Potency | | | | | |
|---|---|---|---|---|---|---|---|---|
| | | | T0 | 1M at 40 °C | 6M at 25 °C | 12M at 25 °C | 9M at 5 °C | 12M at 5 °C |
| 1 | 2.9% | 1.5e11 | 80% | 3% | 18% | 5% | 72% | 78% |
| 2 | 2.5% | 1.5e11 | 98% | 2% | 34% | 10% | NT | 72% |
| 3 | 1.2% | 4.6e10 | 25% | 0% | NT | NT | NT | NT |
| 4 | 1.4% | 7.7e10 | 50% | 0% | NT | NT | NT | NT |
| 5 | 2.7% | 1.4e11 | 87% | 35% | 74% | 38% | 75% | 90% |

The formulation compositions of Lyophilization Study 2 are listed in Table 3. The formulations and their concentrations listed in the Table 3 are pre-lyophilization excipient concentrations. In addition to the mannitol impact, the effect of sucrose concentration, plasticizer, and alternative multivalent salts was examined in Lyophilization Study 2. In biologics formulation, sucrose can function as both a lyoprotectant/cytoprotectant and a stabilizer, while mannitol provides additional function as a bulking agent for easier reconstitution, elegant cake appearance, and potentially efficient and robust lyophilization cycle. (Shreya, 2018) Differing from traditional large molecule therapy such as antibodies and enzymes, AAV is a complex entity that needs capsid integrity to protect the viral genome as well as mediate cellular uptake and intracellular trafficking. (Giles, 2018) SYBR gold fluorescence assay was used to assess AAV capsid integrity, where the SYBR gold dye bind to trace amount of free DNA molecules and indirectly detect capsid damage (i.e. it detects DNA released from damaged capsids). As shown in Table 4, similar moisture contents (~ 1%) for pure sucrose-containing formulation (1A) and mannitol-containing formulations (6A and 7A) were achieved in Lyophilization Study 2, lower than the moisture content in Lyophilization Study 1. When mannitol was added to AAV formulation at 1: 14 and 1:4 sucrose to mannitol molar ratio (Formulation 6A and 7A), much higher fluorescence intensity was detected in the mannitol-containing formulations, suggesting the crystalline matrix formed by mannitol caused more viral genome release by damaging the AAV capsid (Figure 15A). Consistent with capsid damage, Formulation 6A and 7A showed around 0.4 log loss of titer where 1A only had 0.16 log loss of titer (Figure 15B). Also, right after lyophilization, the potency of 6A and 7A decreased to 25% and 57%, respectively while 1A maintained 108% potency (Table 4). These data together showed that the loss of titer and potency in mannitol-containing formulations were due to the crystalline matrix but not lower moisture content. A previous formulation study for adenovirus showed better titer recovery post lyophilization as moisture content increasing from 0.6% to 1.4% along with mannitol to sucrose molar ratio decreasing from 8:1 to 2:1. They concluded the worse titer at low moisture content is due to the low moisture content or 'over-drying'. (Croyle, 2001) However, that is not a sound conclusion since the moisture content in their study changed along with the formulation compositions. By achieving similar moisture content in the mannitol-containing formulations and sucrose-containing formulation, we proved that amorphous matrix is better than crystalline matrix at stabilizing AAV in lyophilization formulation.

Table 3. Formulation compositions examined in Lyophilization Study 2

| Component | Formulation# | | | | | | |
|---|---|---|---|---|---|---|---|
| | 1A* | 2A | 3A | 4A | 5A | 6A | 7A |
| Buffer | 5 mM Tris | 5 mM Tris | 5 mM Tris | 1.5 mM Potassium Phosphate Monobasic Crystal, 8.1 mM Sodium Phosphate Dibasic Anhydrous | 5 mM Tris | 5 mM NaPi | 5 mM Tris |
| Salt | 20 mM Na citrate | 20 mM Na citrate | 20 mM Na citrate | 2.7 mM Potassium Chloride, 60 mM NaCl | 30 mM Na$_2$SO$_4$ | 20 mM Na citrate | 20 mM Na citrate |
| Cryo/ Lyoprotectant | 210 mM sucrose | 210 mM sucrose | 210 mM sucrose | 409 mM sucrose | 263 mM sucrose | 14.6 mM sucrose | 45 mM sucrose |
| Bulking Agent | | | | | | 210 mM mannitol | 180 mM mannitol |
| Surfactant | 0.002% (w/v) P188 | 0.002% (w/v) P188 | 0.002% (w/v) P188 | 0.001% (w/v) P188 | 0.005% (w/v) P188 | 0.002% (w/v) P188 | 0.002% (w/v) P188 |
| Plasticizer | | 0.25% (w/v) glycerol | 0.5% (w/v) Sorbitol | | | | |

* Formulation 1A in Lyophilization Study 2 is the same composition as Formulation 5 in Lyophilization Study 1, a new numbering is used for easy discussion.

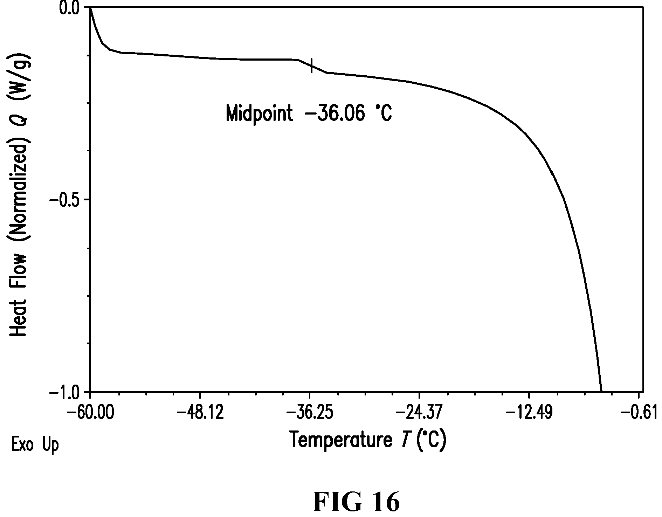

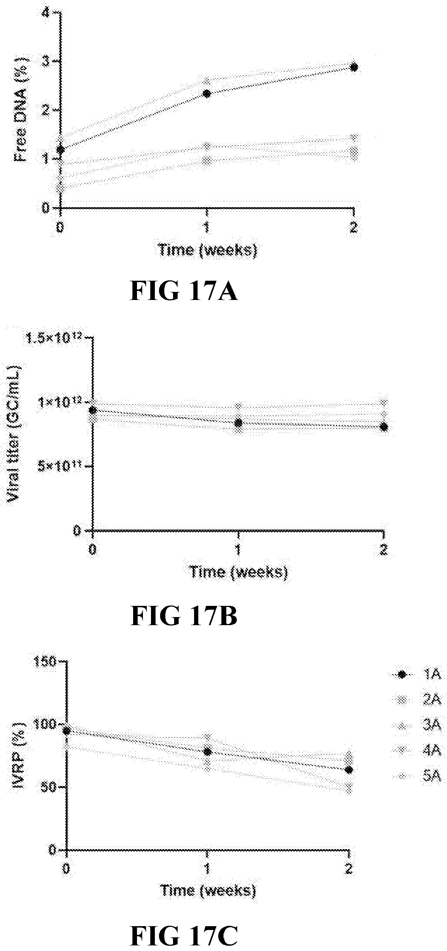

The impact of sucrose was further tested at different concentrations, 210, 263 and 409 mM in Formulation 1A, 5A, and 4A, respectively. Sodium chloride

can greatly reduce the collapse temperature of a formulation, if a fraction of the salt does not crystallize. Crystallization of NaCl during freezing and annealing can be inhibited by other excipients. (Carpenter, 2002) The thermal analysis of Formulation 4A was characterized by low-temperature DSC in Figure 16. There was no crystallization event detected below -37 °C. The Tg' at - 36.1 °C was due to the glass transition of sucrose. When a very high sucrose concentration was used in Formulation 4A, the crystallization of NaCl was suppressed. Formulation 4A and 5A (409 mM and 263 mM sucrose) showed less release of free DNA caused by capsid damage than Formulation 1A (210 mM sucrose), demonstrating the criticality of sucrose inhibiting NaCl crystallization and the concentration-dependent protective effect of sucrose in AAV formulation. Figure 17.

Table 4. Comparison of AAV stability in crystalline and amorphous formulation right after lyophilization

| Formulatio n | Moistur e (%) | Increase in fluorescence intensity | Log titer loss | Potency (%) |
|---|---|---|---|---|
| 1A | 1.1% | 7348 | 0.163 | 108 |
| 6A | 1.2% | 39944 | 0.416 | 25 |
| 7A | 0.9% | 21455 | 0.441 | 57 |

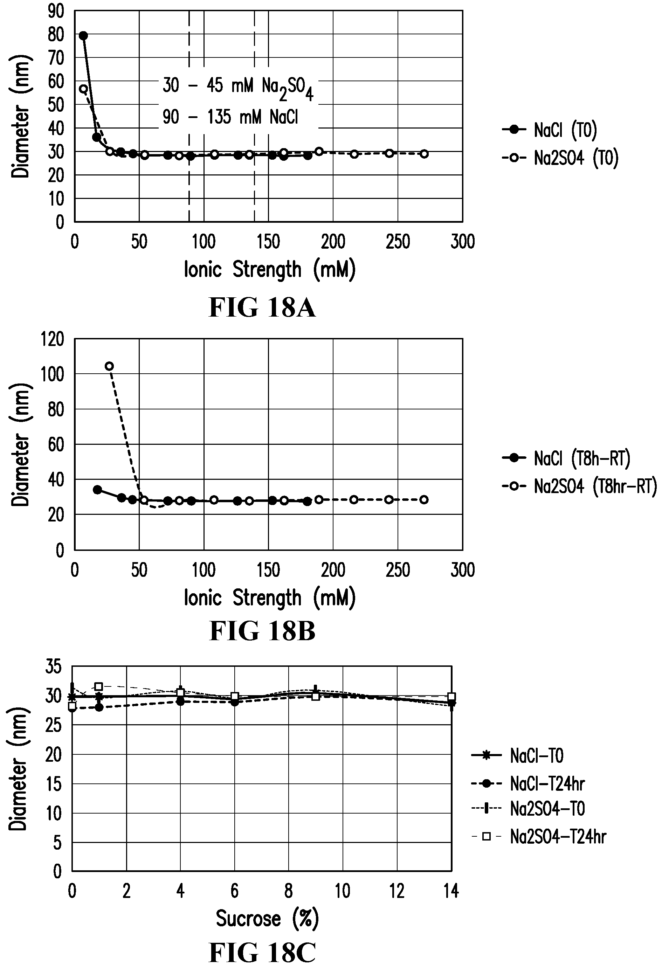

In addition to sodium citrate, alternative multivalent ions were sought for inhibiting AAV aggregation while ensuring the solution Tg' remained amenable to lyophilization cycle development. Citrate combined with sucrose has benefits in that the dried cake glass transition temperature (Tg) is higher, which is expected to result in higher stability of lyophilized AAV) (Kets, 2003). However, citrate is also known to cause injection site pain, especially for intramuscular or subcutaneous injections (Brazeau, 1998). Removal of citrate was recommended for subcutaneous formulations of erythropoietin (Brazeau, 1998). Another example is 'Humira citrate-free' which is marketed as an improved formulation offering 'with less pain immediately following injection'. (Humira citrate-free website, accessed 19Mar20). For some routes of administration, the presence of citrate is not expected to impact pain sensation either because of the route and presence or absence of pain receptors or because a surgery may be performed which masks the relative perception of pain of citrate. For example, slow intravenous infusion may dilute and minimize the sensation of pain, or subretinal injection surgery may not have a relatively higher perception of pain with a citrate formulation. In these cases, the presence of citrate may not be an issue for pain on injection. For cases such as intramuscular, suprachoroidal, and subcutaneous injection the presence of citrate may cause a greater perception of pain. To address this issue for some routes of administration we evaluated alternatives to citrate for a lyophilized formulation. As shown in Figure 18A, a much lower concentration of sodium sulfate than sodium chloride was needed to achieve similar AAV aggregation inhibition effect. This is a benefit in terms of sucrose being able to more fully suppress the crystallization of a lower concentration of sodium sulfate. Crystallization appears to negatively impact the stability of AAV as shown by the lack of stability of the mannitol-containing formulations. The particle size of AAV remained constant after stressed at RT for 24 hours in the presence of $Na_2SO_4$. Figure 18B&C. More importantly, 30 mM $Na_2SO_4$ can effectively inhibit AAV aggregation in the presence of up to 14% sucrose (409 mM). Figure 18C. Other similar multivalent salts, $MgSO_4$ and $(NH4)_2SO_4$, can be used as alternative salts for inhibiting AAV aggregation in lyophilization formulation.

To evaluate the feasibility of using sodium sulfate in a lyophilization formulation, the Tg' of different formulation buffers is shown in Table 5. For a robust lyophilization process, a higher (less negative, less cold) Tg' is desired, ideally above -40 °C. Formulation 1A, 2A, 3A, 4A, and 5A all showed desirable Tg' temperature while PBS and NaCl containing formulations (Buffer 1 and 2) would result in longer lyophilization cycles that are more prone to collapse due to salts crystallization.

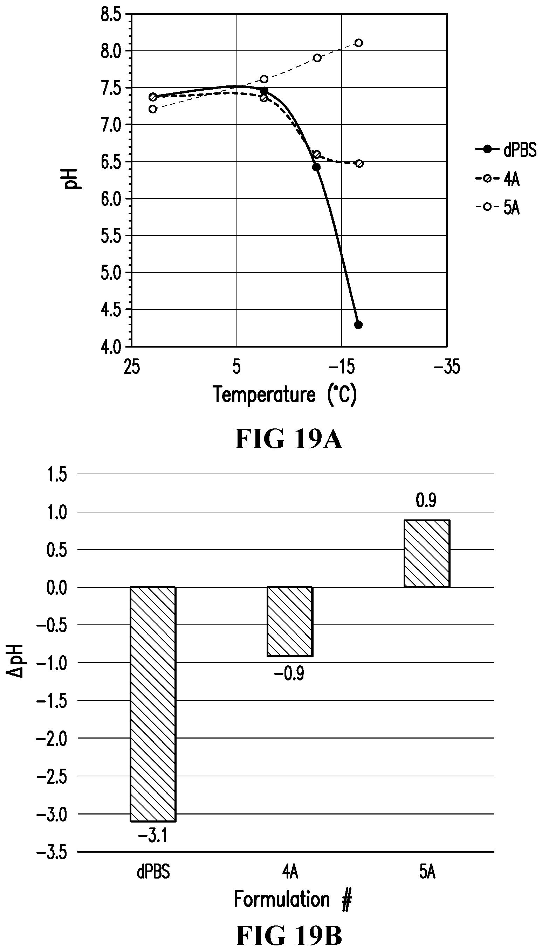

In addition, pH stability during freezing is critical in maintaining AAV potency. A 3 pH units shift was observed in dPBS based formulation buffer upon freezing. However, only 1 pH unit shift was seen in Formulation 4A and 5A. Figure 19. This may in part explain why tris-buffered systems are more suitable for lyophilization than phosphate-buffered systems. The exposure of AAV to low pH during the freezing of phosphate buffers is destabilizing.

Table 5. Tg' of lyophilization formulations composed of different excipients

| Formulation Description | Tg' (°C) |
|---|---|
| **Buffer 1:** 2.70 mM potassium chloride, 1.47 mM potassium phosphate monobasic, 100 mM sodium chloride, 8.10 mM sodium phosphate dibasic anyhydrous, 4% sucrose, pH 7.4, 0.001% poloxamer 188 | -44.8 |
| **Buffer 2:** 5 mM Tris, 6% sucrose, 80 mM NaCl, pH 7.4, 0.001% poloxamer 188 | -41.2 |
| **1A** | -35 |
| **2A** | -37.2 |
| **3A** | -35.6 |
| **4A** | -36.1 |
| **5A** | -35.6 |

There are examples and theories that addition of small quantities of excipient additives, such as glycerol or sorbitol, can stabilize some lyophilized biologics, but it is not known if such an approach can work with AAV (Cicerone, 2003). Theories, such as 'water replacement' by replacing water-disaccharide sugar H-bonds with stronger glycerol-sugar H-bonds or 'plasticizer' behavior are used to explain how these additives improve stability of biologics, but the impact of these types of excipients on the stability of lyophilized AAV is not clear. (Starciuc, 2017; Cicerone, 2003) To evaluate the possibility that 'water replacement' or 'plasticizers' might help with stability, glycerol and sorbitol (Formulation 2A and 3A) were added to Formulation 1A to evaluate if better lyophilized AAV stability can be achieved. The Tg of lyophilized cake is determined by the Tg of all ingredients, their weight percentage, and moisture content. As discussed in the previous section, the intermolecular interaction between citrate and sucrose lifted the Tg of 1A to 58.7 °C, while 2A and 3A had Tg at 42.0 °C and 37.8 °C, respectively (Table 6). It has been reported that although plasticizers such as glycerol and sorbitol decreased amorphous lyophilized cake Tg, they can stabilize protein therapeutics by slowing the β relaxation of a glass matrix through antiplasticization when stored at a temperature below the glass transition temperature of lyophilized cake. (Cicerone, 2003). At 35 °C which is below the Tg of Formulation 1A, 2A and 3A, less AAV capsid disruption resulting in release of free DNA and higher potency were achieved in Formulation 2A with added glycerol but not Formulation 3A with added sorbitol, demonstrating glycerol's unique ability in stabilizing AAV in a lyophilization formulation, which was not reported previously. Figure 17. Surprisingly, the sorbitol-containing formulation (formulation 3A) did not provide the same benefit as glycerol and had an almost identical release of free DNA to the matching formulation composition without sorbitol. This suggests that the ability of glycerol to stabilize lyophilized AAV is specific and unique and the general concept of using 'water replacement' or 'plasticizer' excipients does not fully explain the stabilizing benefit of glycerol for lyophilized AAV.

Table 6. Moisture content and Tg of the lyophilized cakes of Formulations 1A-5A

| Formulation Description | Moisture (%) | Solid cake Tg (°C) |
|---|---|---|
| 1A | 1.5 | 58.7 |
| 2A | 1.1 | 42.0 |
| 3A | 1.5 | 37.8 |
| 4A | 1.8 | 37.5 |
| 5A | 1.4 | 39.3 |

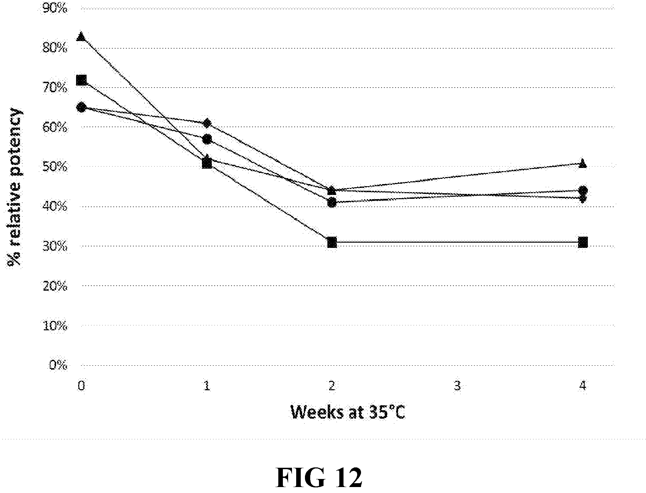

The viral titer and *in vitro* relative potency of 1A, 2A, 3A, 4A, and 5A were examined after short-term stress study at 35 °C for 2 weeks. The five formulations were able to retain 47% to 75% potency after 35 °C for 2 weeks (Figure 17C). Formulation 1A that had 35% potency after 40 °C for 4 weeks showed 90% potency after 12 months at 5 °C (Formulation 5 in Table 1 is Formulation 1A in Table 3). As shown in Figure 12, potency decrease due to 35 °C temperature stress plateaued from 2 weeks to 4 weeks. Although we don't have long term stability for Formulation 1A-5A at 1% moisture content range, the 35 °C stress study result is suggesting better long-term stability.

Here we demonstrated that formulations buffered with tris, containing either a minimum amount of citrate or sulfate at either 20 or 30 mM as a salt to prevent AAV self-association, sucrose to inhibit salt crystallization and provide a cryoprotective and lyoprotective glassy-state, poloxamer 188 to prevent AAV adsorption to surfaces, and the 'plasticizer' glycerol provide suitable lyophilized stability to AAV. Formulation 1A, 2A, 4A, and 5A are all candidate formulations for AAV lyophilization due to the unique selection of different excipients to inhibit AAV aggregation while remaining a favorable Tg' temperature and high potency by protecting AAV capsid against the freezing and drying stress.

**Claims**

1. A stable formulation comprising a recombinant adeno-associated virus (rAAV) particle and

    a) Tris,
    b) a sugar, and
    c) an amorphous salt,
    wherein the formulation is suitable for lyophilization; and
    wherein the rAAV particle comprises an AAV8 capsid protein.

2. The formulation of claim 1, wherein the amorphous salt has an ionic strength greater than or equal to 60 mM, optionally wherein the amorphous salt has an ionic strength between 60 mM and 150 mM.

3. The formulation of claim 1 or 2, wherein the formulation comprises:

    a) between about 1 mM and about 50 mM Tris; or
    b) between about 1 mM and about 30 mM, between about 1 mM and about 20 mM, between about 5 mM and about 30 mM, between about 5 mM and about 20 mM, between about 10 mM and about 30 mM, between about 10 mM and about 20 mM, or between about 20 mM and about 50 mM Tris; or
    c) about 1 mM, about 2 mM, about 3 mM, about 5 mM, about 10 mM, about 15 mM, about 20 mM, about 25 mM, about 30 mM, or about 40 mM Tris, optionally about 5 mM Tris.

4. The formulation of any one of claims 1 to 3, wherein the amorphous salt is sodium citrate, sodium sulfate, ammonium sulfate, magnesium sulfate, or a combination thereof.

5. The formulation of any one of claims 1 to 4, wherein the formulation comprises about 10 mM, about 20 mM, about 30 mM, about 40 mM, about 50 mM, about 60 mM, about 70 mM, about 80 mM, about 90 mM, about 100 mM, about 120 mM, about 140 mM, about 150 mM, or about 200 mM sodium sulfate.

6. The formulation of any one of claims 1 to 5 having a pH of:

    a) between about 6.5 and 8.0;
    b) between about 7.2 and 7.8;

c) about 7.2, 7.3, 7.4, 7.5, 7.6, 7.7, or 7.8;
d) about 7.5; or
e) about 7.1.

7. The formulation of any one of claims 1 to 6 comprising:

a) between about 50 mM and about 400 mM sugar;
b) between about 50 mM and about 350 mM, between about 50 mM and about 300 mM, between about 50 mM and about 250 mM, between about 50 mM and about 200 mM, or between about 50 mM and about 150 mM sugar;
c) between about 100 mM and about 400 mM, between about 150 mM and about 400 mM, between about 200 mM and about 400 mM, between about 250 mM and about 400 mM, or between about 300 mM and about 400 mM sugar;
d) between about 100 mM and about 300 mM, between about 150 mM and about 250 mM, between about 200 mM and about 300 mM, or between about 250 mM and about 350 mM sugar;
e) about 50 mM, about 100 mM, about 150 mM, about 160 mM, about 170 mM, about 180 mM, about 190 mM, about 200 mM, about 210 mM, about 220 mM, about 230 mM, about 240 mM, about 250 mM, about 260 mM, about 270 mM, about 280 mM, about 290 mM, about 300 mM, or about 350 mM sugar;
f) between about 190 mM and about 230 mM, between about 170 mM and about 250 mM, or between about 250 mM and about 270 mM sugar; or
g) about 210 mM sugar.

8. The formulation of any one of claims 1 to 7, wherein the sugar is:

a) a non-reducing sugar, optionally wherein the non-reducing sugar is sucrose, trehalose, or raffinose; or
b) a reducing sugar, optionally wherein the reducing sugar is glucose, fructose, mannose, galactose, or lactose.

9. The formulation of any one of claims 1 to 8 comprising:

a) less than about 100 mM sodium citrate;
b) between about 10 mM and about 100 mM sodium citrate;
c) between about 10 mM and about 100 mM, between about 20 mM and about 100 mM, between about 30 mM and about 100 mM, between about 40 mM and about 100 mM, between about 50 mM and about 100 mM, between about 10 mM and about 80 mM, between about 10 mM and about 60 mM, between about 10 mM and about 50 mM, between about 10 mM and about 40 mM, or between about 10 mM and about 30 mM sodium citrate;
d) between about 10 mM and about 50 mM, between about 20 mM and about 60 mM, between about 30 mM and about 70 mM, or between about 10 mM and about 30 mM sodium citrate;
e) about 10 mM, about 20 mM, about 30 mM, about 40 mM, about 50 mM, or about 60 mM sodium citrate; or
f) about 20 mM sodium citrate.

10. The formulation of any one of claims 1 to 9, further comprising:

a) between about 0.0005% and about 0.01% nonionic surfactant; or
b) about 0.002% nonionic surfactant,

optionally wherein the nonionic surfactant comprises poloxamer 188, poloxamer 407, polysorbate 80, polysorbate 20, Pluronic F-68, or BRIJ 35.

11. The formulation of any one of claims 1 to 10, further comprising a stabilizer selected from the group consisting of glycerol, or sorbitol, optionally wherein the formulation comprises:

a) between about 0.1% and between about 5% glycerol;
b) between about 0.1% and between about 2% glycerol; or
c) between about 0.25% and between about 2% glycerol.

12. The formulation of any one of claims 1 to 11, wherein:

a) the formulation does not comprise mannitol; or
b) the formulation comprises less than 10 mM, 20 mM, 50mM, 100mM or 150mM mannitol.

**13.** The formulation of any one of claims 1 to 12 comprising:

A.

    a) between about 1 mM and about 25 mM Tris,
    b) between about 50 mM and about 400 mM sugar,
    c) between about 10 mM and about 100 mM sodium citrate, and
    d) between about 0.0005% and about 0.01% non-ionic surfactant,

wherein the formulation has a pH of between about 7.2 and about 7.8;
B.

    a) between about 2 mM and about 10 mM Tris,
    b) between about 150 mM and about 250 mM sugar,
    c) between about 10 mM and about 20 mM sodium citrate, and
    d) between about 0.001% and about 0.005% non-ionic surfactant,

wherein the formulation has a pH of between about 7.2 and about 7.8;
C.

    a) about 5 mM Tris,
    b) about 210 mM sugar,
    c) about 20 mM sodium citrate, and
    d) about 0.002% non-ionic surfactant,

wherein the formulation has a pH of about 7.5;
D.

    a) about 5 mM Tris,
    b) about 210 mM sucrose,
    c) about 20 mM sodium citrate, and
    d) about 0.002% (w/v) poloxamer 188;

E.

    a) about 5 mM Tris,
    b) about 210 mM sucrose,
    c) about 20 mM sodium citrate,
    d) about 0.002% (w/v) poloxamer 188, and,
    e) about 0.25% (w/v) glycerol;

F.

    a) about 5 mM Tris,
    b) about 210 mM sucrose,
    c) about 20 mM sodium citrate,
    d) about 0.002% (w/v) poloxamer 188, and
    e) about 0.5% (w/v) sorbitol;

G.

    a) about 5 mM Tris,
    b) about 30 mM sodium sulfate,
    c) about 263 mM sucrose, and
    d) about 0.005% (w/v) poloxamer 188;

H. between about 1.0E+11 genome copy/ mL (GC/mL) and about 1.0E+15 GC/mL rAAV particles; or
I. about 1.0E+11 GC/mL, about 1.0E+12 GC/mL, about 1.0E+13 GC/mL, about 1.0E+14 GC/mL, or about 1.0E+15 GC/mL rAAV particles.

14. The formulation of any one of claims 1 to 13 having a residual moisture content of:

a) between about 1% and about 7%;
b) between about 1% and about 7%, between about 2% and about 7%, between about 3% and about 7%, between about 4% and about 7%, between about 5% and about 7%, between about 1% and about 6%, between about 1% and about 5%, between about 1% and about 4%, or between about 1% and about 3%;
c) between about 3% and about 7%, between about 3% and about 6%, or between about 3% and about 5%;
d) about 3%, about 3.5%, about 4%, about 4.5%, about 5%, about 5.5%, or about 6%;
e) between about 1% to about 2%;
f) about 1.5%, about 1.4%, about 1.3%, about 1.2%, or about 1.1%; or
g) about 1%.

15. The formulation of any one of claims 1 to 14, wherein:

a) the glass transition temperature (Tg) of the lyophilized cakes of the formulation is higher than 35 °C; or
b) the glass transition temperature of the maximally freeze-concentrated solution (Tg') of the formulation is higher than -40 °C.

16. The formulation of any one of claims 1 to 15, wherein the % relative potency of the rAAV particles is:

a) at least about 60%, at least about 70%, or at least about 80% after storing the formulation for 3 months at room temperature; wherein the reference rAAV particles are stored at -70°C in DPBS with 0.001% poloxamer 188 buffer;
b) at least about 60%, at least about 70%, or at least about 80% after storing the formulation for 6 months at room temperature; wherein the reference rAAV particles are stored at -70°C in Dulbecco's phosphate-buffered saline (DPBS) with 0.001% poloxamer 188 buffer;
c) at least about 30%, at least about 40%, at least about 50%, or at least 60% after storing the formulation for 1 week at 35°C, wherein the reference rAAV particles are stored at -70°C in DPBS with 0.001% poloxamer 188 buffer;
d) at least about 30%, at least about 40%, at least about 50%, or at least 60% after storing the formulation for 2 weeks at 35°C, wherein the reference rAAV particles are stored at -70°C in DPBS with 0.001% poloxamer 188 buffer; or
e) at least about 30%, at least about 40%, at least about 50%, or at least 60% after storing the formulation for 4 weeks at 35°C, wherein the reference rAAV particles are stored at -70°C in DPBS with 0.001% poloxamer 188 buffer.

17. The formulation of claim 16, wherein the formulation is lyophilized prior to storing, optionally wherein the lyophilized formulation is reconstituted after storing.

18. The formulation of any one of claims 1 to 17, wherein:

A. the % relative potency of the rAAV particles is at least about 25%, at least about 30%, at least about 40%, at least about 50%, at least about 60%, at least about 70%, at least about 80%, at least about 90%, least about 95%, or at least about 99% right after lyophilization;
B. the level of rAAV particle aggregation of the formulation is decreased about 10%, about 20%, about 30%, about 40%, about 50%, about 60%, about 70%, about 80%, about 90%, or about 100% as compared to the level of rAAV particle aggregation in a reference formulation; or
C. the stability of the formulation is assessed by:

a) vector genome content or viral titer assay, wherein the formulation has at least about 5%, about 10%, about 20%, about 30%, about 40%, about 50%, about 60%, about 70%, about 80%, about 90%, about 100%, or about 200%, more genome content after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the genome content of a reference formulation stored under the same condition;
b) measuring the relative potency of the formulation, wherein the formulation has at least about 5%, about

**EP 3 955 891 B1**

10%, about 20%, about 30%, about 40%, about 50%, about 60%, about 70%, about 80%, about 90%, about 100%, or about 200%, more relative potency after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the genome content of a reference formulation stored under the same condition;

c) loss of infectivity, wherein the formulation has at least about 5%, about 10%, about 20%, about 30%, about 40%, about 50%, about 60%, about 70%, about 80%, about 90%, about 100%, or about 200%, less infectivity loss after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the infectivity loss of a reference formulation stored under the same condition; or

d) rAAV genome release, wherein the rAAV genome release is determined by measuring relative fluorescence in preference of a DNA specific florescent stain, and wherein the formulation has at least about 5%, about 10%, about 20%, about 30%, about 40%, about 50%, about 60%, about 70%, about 80%, about 90%, about 100%, or about 200%, less relative fluorescence level after storing the formulation for 1 day, 2 days, 3 days, 4 days, 5 days, 6 days, 1 week, 2 weeks, 3 weeks, 4 weeks, 1 month, 2 months, 3 months, 4 months, 5 months, 6 months, 7 months, 8 months, 9 months, 10 months, 11 months, 1 year, 2 years, or 3 years as compared to the relative fluorescence level of a reference formulation stored under the same condition, optionally wherein the reference formulation is:

(i) DPBS with 0.001% poloxamer 188 buffer;
(ii) a formulation not comprising sugar; or
(iii) a formulation not comprising plasticizer.

19. The formulation of any one of claims 1 to 18, wherein

a) the formulation is stored at -80°C, -70°C, -20°C, 4°C, 20°C, 25°C, 30°C, 35°C, or 40 °C;
b) the formulation is frozen to a temperature of about - 20 °C in the process of lyophilization;
c) the frozen formulation maintains pH between about pH 6 to about pH 9 when freezing down to -20 °C;
d) the frozen formulation maintains a pH value within a range of plus or minus 1 unit of the pH value prior to freezing when freezing down to -20 °C; or
e) the formulation is a stabilized aqueous formulation of rAAV for lyophilization.

20. A method of producing a stable formulation according to any one of claims 1 to 19, wherein the method comprises combining rAAV particles which comprise an AAV8 capsid, with Tris, a sugar, an amorphous salt, optionally a plasticizer, and/or a nonionic surfactant.

21. A method of reducing rAAV genome release from rAAV particles, comprising:

a) producing a formulation according to any one of claims 1 to 19, wherein rAAV genome release from the rAAV particles after three freeze-thaw cycles is reduced compared to rAAV genome release in a formulation not comprising the sugar; or
b) producing a formulation according to any one of claims 1 to 19, wherein rAAV genome release from the rAAV particles after lyophilization and reconstitution is reduced compared to rAAV genome release in a formulation not comprising the sugar.

22. A method of producing a stable lyophilized formulation of an rAAV product, comprising a step of subjecting to lyophilization a pre-lyophilized formulation, wherein pre-lyophilized formulation is according to any one of claims 1 to 19.

23. A formulation as defined in any one of claims 1 to 19 for use in a method of treating or preventing a disease, wherein the formulation is lyophilized and reconstituted before being administered to a subject in need thereof.

**Patentansprüche**

1. Stabile Formulierung, umfassend ein rekombinantes adeno-assoziiertes Viruspartikel (rAAV) und

78

a) Tris,
b) ein Zucker, und
c) ein amorphes Salz,
wobei die Formulierung zur Lyophilisierung geeignet ist; und
wobei das rAAV-Partikel ein AAV8-Kapsidprotein umfasst.

2. Formulierung gemäß Anspruch 1, wobei das amorphe Salz eine Ionenstärke von größer oder gleich 60 mM aufweist, optional wobei das amorphe Salz eine Ionenstärke zwischen 60 mM und 150 mM aufweist.

3. Formulierung gemäß Anspruch 1 oder 2, die Formulierung umfassend:

a) zwischen etwa 1 mM und etwa 50 mM Tris; oder
b) zwischen etwa 1 mM und etwa 30 mM, zwischen etwa 1 mM und etwa 20 mM, zwischen etwa 5 mM und etwa 30 mM, zwischen etwa 5 mM und etwa 20 mM, zwischen etwa 10 mM und etwa 30 mM, zwischen etwa 10 mM und etwa 20 mM oder zwischen etwa 20 mM und etwa 50 mM Tris; oder
c) etwa 1 mM, etwa 2 mM, etwa 3 mM, etwa 5 mM, etwa 10 mM, etwa 15 mM, etwa 20 mM, etwa 25 mM, etwa 30 mM oder etwa 40 mM Tris, optional etwa 5 mM Tris.

4. Formulierung gemäß einem der Ansprüche 1 bis 3, wobei das amorphe Salz Natriumcitrat, Natriumsulfat, Ammoniumsulfat, Magnesiumsulfat oder eine Kombination davon ist.

5. Formulierung gemäß einem der Ansprüche 1 bis 4, wobei die Formulierung etwa 10 mM, etwa 20 mM, etwa 30 mM, etwa 40 mM, etwa 50 mM, etwa 60 mM, etwa 70 mM, etwa 80 mM, etwa 90 mM, etwa 100 mM, etwa 120 mM, etwa 140 mM, etwa 150 mM oder etwa 200 mM Natriumsulfat umfasst.

6. Formulierung gemäß einem der Ansprüche 1 bis 5, mit einem pH-Wert:

a) zwischen etwa 6,5 und 8,0;
b) zwischen etwa 7,2 und 7,8;
c) etwa 7,2, 7,3, 7,4, 7,5, 7,6, 7,7 oder 7,8;
d) etwa 7,5; oder
e) etwa 7,1.

7. Formulierung gemäß einem der Ansprüche 1 bis 6, umfassend:

a) zwischen etwa 50 mM und etwa 400 mM Zucker;
b) zwischen etwa 50 mM und etwa 350 mM, zwischen etwa 50 mM und etwa 300 mM, zwischen etwa 50 mM und etwa 250 mM, zwischen etwa 50 mM und etwa 200 mM oder zwischen etwa 50 mM und etwa 150 mM Zucker;
c) zwischen etwa 100 mM und etwa 400 mM, zwischen etwa 150 mM und etwa 400 mM, zwischen etwa 200 mM und etwa 400 mM, zwischen etwa 250 mM und etwa 400 mM oder zwischen etwa 300 mM und etwa 400 mM Zucker;
d) zwischen etwa 100 mM und etwa 300 mM, zwischen etwa 150 mM und etwa 250 mM, zwischen etwa 200 mM und etwa 300 mM oder zwischen etwa 250 mM und etwa 350 mM Zucker;
e) etwa 50 mM, etwa 100 mM, etwa 150 mM, etwa 160 mM, etwa 170 mM, etwa 180 mM, etwa 190 mM, etwa 200 mM, etwa 210 mM, etwa 220 mM, etwa 230 mM, etwa 240 mM, etwa 250 mM, etwa 260 mM, etwa 270 mM, etwa 280 mM, etwa 290 mM, etwa 300 mM oder etwa 350 mM Zucker;
f) zwischen etwa 190 mM und etwa 230 mM, zwischen etwa 170 mM und etwa 250 mM oder zwischen etwa 250 mM und etwa 270 mM Zucker; oder
g) etwa 210 mM Zucker.

8. Formulierung gemäß einem der Ansprüche 1 bis 7, wobei der Zucker:

a) ein nicht reduzierender Zucker ist, optional wobei der nicht reduzierende Zucker Saccharose, Trehalose oder Raffinose ist; oder
b) ein reduzierender Zucker ist, optional wobei der reduzierende Zucker Glucose, Fructose, Mannose, Galactose oder Lactose ist.

9. Formulierung gemäß einem der Ansprüche 1 bis 8, umfassend:

a) weniger als etwa 100 mM Natriumcitrat;

b) zwischen etwa 10 mM und etwa 100 mM Natriumcitrat;

c) zwischen etwa 10 mM und etwa 100 mM, zwischen etwa 20 mM und etwa 100 mM, zwischen etwa 30 mM und etwa 100 mM, zwischen etwa 40 mM und etwa 100 mM, zwischen etwa 50 mM und etwa 100 mM, zwischen etwa 10 mM und etwa 80 mM, zwischen etwa 10 mM und etwa 60 mM, zwischen etwa 10 mM und etwa 50 mM, zwischen etwa 10 mM und etwa 40 mM oder zwischen etwa 10 mM und etwa 30 mM Natriumcitrat;

d) zwischen etwa 10 mM und etwa 50 mM, zwischen etwa 20 mM und etwa 60 mM, zwischen etwa 30 mM und etwa 70 mM oder zwischen etwa 10 mM und etwa 30 mM Natriumcitrat;

e) etwa 10 mM, etwa 20 mM, etwa 30 mM, etwa 40 mM, etwa 50 mM oder etwa 60 mM Natriumcitrat; oder

f) etwa 20 mM Natriumcitrat.

10. Formulierung gemäß einem der Ansprüche 1 bis 9, ferner umfassend:

a) zwischen etwa 0,0005% und etwa 0,01% nichtionisches Tensid; oder

b) etwa 0,002 % nichtionisches Tensid,

optional wobei das nichtionische Tensid Poloxamer 188, Poloxamer 407, Polysorbat 80, Polysorbat 20, Pluronic F-68 oder BRIJ 35 umfasst.

11. Formulierung gemäß einem der Ansprüche 1 bis 10, ferner umfassend einen Stabilisator, ausgewählt aus der Gruppe, bestehend aus Glycerin oder Sorbit, optional wobei die Formulierung umfasst:

a) zwischen etwa 0,1 % und zwischen etwa 5 % Glycerin;

b) zwischen etwa 0,1 % und zwischen etwa 2 % Glycerin; oder

c) zwischen etwa 0,25 % und zwischen etwa 2 % Glycerin.

12. Formulierung gemäß einem der Ansprüche 1 bis 11, wobei:

a) die Formulierung kein Mannitol umfasst; oder

b) die Formulierung weniger als 10 mM, 20 mM, 50 mM, 100 mM oder 150 mM Mannitol umfasst.

13. Formulierung gemäß einem der Ansprüche 1 bis 12, umfassend:

A.

a) zwischen etwa 1 mM und etwa 25 mM Tris,

b) zwischen etwa 50 mM und etwa 400 mM Zucker,

c) zwischen etwa 10 mM und etwa 100 mM Natriumcitrat, und

d) zwischen etwa 0,0005% und etwa 0,01% nichtionischem Tensid, wobei die Formulierung einen pH-Wert zwischen etwa 7,2 und etwa 7,8 hat;

B.

a) zwischen etwa 2 mM und etwa 10 mM Tris,

b) zwischen etwa 150 mM und etwa 250 mM Zucker,

c) zwischen etwa 10 mM und etwa 20 mM Natriumcitrat, und

d) zwischen etwa 0,001 % und etwa 0,005 % nichtionischem Tensid, wobei die Formulierung einen pH-Wert zwischen etwa 7,2 und etwa 7,8 hat;

C.

a) etwa 5 mM Tris,

b) etwa 210 mM Zucker,

c) etwa 20 mM Natriumcitrat, und

d) etwa 0,002 % nichtionisches Tensid,

wobei die Formulierung einen pH-Wert von etwa 7,5 hat;

D.

a) etwa 5 mM Tris,
b) etwa 210 mM Saccharose,
c) etwa 20 mM Natriumcitrat, und
d) etwa 0,002 % (w/v) Poloxamer 188;

E.

a) etwa 5 mM Tris,
b) etwa 210 mM Saccharose,
c) etwa 20 mM Natriumcitrat,
d) etwa 0,002 % (w/v) Poloxamer 188, und
e) etwa 0,25 % (w/v) Glycerin;

F.

a) etwa 5 mM Tris,
b) etwa 210 mM Saccharose,
c) etwa 20 mM Natriumcitrat,
d) etwa 0,002 % (w/v) Poloxamer 188, und
e) etwa 0,5 % (w/v) Sorbit;

G.

a) etwa 5 mM Tris,
b) etwa 30 mM Natriumsulfat,
c) etwa 263 mM Saccharose, und
d) etwa 0,005 % (w/v) Poloxamer 188;

H. zwischen etwa 1,0E+11 Genomkopien/ml (GC/ml) und etwa 1,0E+15 GC/ml rAAV-Partikel; oder
I. etwa 1,0E+11 GC/ml, etwa 1,0E+12 GC/ml, etwa 1,0E+13 GC/ml, etwa 1,0E+14 GC/ml oder etwa 1,0E+15 GC/ml rAAV-Partikel.

**14.** Formulierung gemäß einem der Ansprüche 1 bis 13, mit einem Restfeuchtigkeitsgehalt von:

a) zwischen etwa 1% und etwa 7%;
b) zwischen etwa 1% und etwa 7%, zwischen etwa 2% und etwa 7%, zwischen etwa 3% und etwa 7%, zwischen etwa 4% und etwa 7%, zwischen etwa 5% und etwa 7%, zwischen etwa 1% und etwa 6%, zwischen etwa 1% und etwa 5%, zwischen etwa 1% und etwa 4% oder zwischen etwa 1% und etwa 3%;
c) zwischen etwa 3 % und etwa 7 %, zwischen etwa 3 % und etwa 6 % oder zwischen etwa 3% und etwa 5 %;
d) etwa 3%, etwa 3,5%, etwa 4%, etwa 4,5%, etwa 5%, etwa 5,5% oder etwa 6%;
e) zwischen etwa 1% und etwa 2%;
f) etwa 1,5%, etwa 1,4%, etwa 1,3%, etwa 1,2% oder etwa 1,1%; oder
g) etwa 1%.

**15.** Formulierung gemäß einem der Ansprüche 1 bis 14, wobei:

a) die Glasübergangstemperatur (Tg) der lyophilisierten Kuchen der Formulierung höher als 35°C ist; oder
b) die Glasübergangstemperatur der maximal gefrierkonzentrierten Lösung (Tg') der Formulierung höher als -40 °C ist.

**16.** Formulierung gemäß einem der Ansprüche 1 bis 15, wobei die prozentuale relative Wirksamkeit der rAAV-Partikel beträgt:

a) mindestens etwa 60%, mindestens etwa 70% oder mindestens etwa 80% nach Lagerung der Formulierung für 3 Monate bei Raumtemperatur; wobei die Referenz-rAAV-Partikel bei -70°C in DPBS mit 0,001% Poloxamer 188-Puffer gelagert werden;
b) mindestens etwa 60%, mindestens etwa 70% oder mindestens etwa 80% nach Lagerung der Formulierung für 6 Monate bei Raumtemperatur; wobei die Referenz-rAAV-Partikel bei -70°C in Dulbecco's phosphatgepufferter Kochsalzlösung (DPBS) mit 0,001% Poloxamer 188-Puffer gelagert werden;

c) mindestens etwa 30%, mindestens etwa 40%, mindestens etwa 50% oder mindestens 60% nach Lagerung der Formulierung für 1 Woche bei 35°C, wobei die Referenz-rAAV-Partikel bei -70°C in DPBS mit 0,001% Poloxamer 188-Puffer gelagert werden;

d) mindestens etwa 30%, mindestens etwa 40%, mindestens etwa 50% oder mindestens 60% nach Lagerung der Formulierung für 2 Wochen bei 35°C, wobei die Referenz-rAAV-Partikel bei -70°C in DPBS mit 0,001% Poloxamer 188-Puffer gelagert werden; oder

e) mindestens etwa 30%, mindestens etwa 40%, mindestens etwa 50% oder mindestens 60% nach Lagerung der Formulierung für 4 Wochen bei 35°C, wobei die Referenz-rAAV-Partikel bei -70°C in DPBS mit 0,001% Poloxamer 188-Puffer gelagert werden.

17. Formulierung gemäß Anspruch 16, wobei die Formulierung vor der Lagerung lyophilisiert wird, optional wobei die lyophilisierte Formulierung nach der Lagerung rekonstituiert wird.

18. Formulierung gemäß einem der Ansprüche 1 bis 17, wobei:

A. die prozentuale relative Wirksamkeit der rAAV-Partikel unmittelbar nach der Lyophilisierung mindestens etwa 25%, mindestens etwa 30%, mindestens etwa 40%, mindestens etwa 50%, mindestens etwa 60%, mindestens etwa 70%, mindestens etwa 80%, mindestens etwa 90%, mindestens etwa 95% oder mindestens etwa 99% beträgt;

B. das Niveau der rAAV-Partikelaggregation der Formulierung im Vergleich zum Niveau der rAAV-Partikelaggregation in einer Referenzformulierung um etwa 10%, etwa 20%, etwa 30%, etwa 40%, etwa 50%, etwa 60%, etwa 70%, etwa 80%, etwa 90% oder etwa 100% verringert ist; oder

C. die Stabilität der Formulierung bewertet wird durch:

a) Vektorgenomgehalt oder Virustiter-Assay, wobei die Formulierung mindestens etwa 5 %, etwa 10 %, etwa 20 %, etwa 30 %, etwa 40 %, etwa 50 %, etwa 60 %, etwa 70 %, etwa 80 %, etwa 90 %, etwa 100 %, oder etwa 200 % mehr Genomgehalt aufweist, nach Lagerung der Formulierung für 1 Tag, 2 Tage, 3 Tage, 4 Tage, 5 Tage, 6 Tage, 1 Woche, 2 Wochen, 3 Wochen, 4 Wochen, 1 Monat, 2 Monaten, 3 Monaten, 4 Monaten, 5 Monaten, 6 Monaten, 7 Monaten, 8 Monaten, 9 Monaten, 10 Monaten, 11 Monaten, 1 Jahr, 2 Jahren oder 3 Jahren im Vergleich zum Genomgehalt einer unter den gleichen Bedingungen gelagerten Referenzformulierung;

b) Messen der relativen Wirksamkeit der Formulierung, wobei die Formulierung mindestens etwa 5%, etwa 10%, etwa 20%, etwa 30%, etwa 40%, etwa 50%, etwa 60%, etwa 70%, etwa 80%, etwa 90%, etwa 100% oder etwa 200% mehr relative Wirksamkeit aufweist, nach Lagerung der Formulierung für 1 Tag, 2 Tage, 3 Tage, 4 Tage, 5 Tage, 6 Tage, 1 Woche, 2 Wochen, 3 Wochen, 4 Wochen, 1 Monat, 2 Monate, 3 Monate, 4 Monate, 5 Monate, 6 Monate, 7 Monaten, 8 Monaten, 9 Monaten, 10 Monaten, 11 Monaten, 1 Jahr, 2 Jahren oder 3 Jahren im Vergleich zum Genomgehalt einer unter den gleichen Bedingungen gelagerten Referenzformulierung;

c) Verlust der Infektiosität, wobei die Formulierung mindestens etwa 5%, etwa 10%, etwa 20%, etwa 30%, etwa 40%, etwa 50%, etwa 60%, etwa 70%, etwa 80%, etwa 90%, etwa 100% oder etwa 200% weniger Infektiositätsverlust nach Lagerung der Formulierung für 1 Tag, 2 Tage, 3 Tage, 4 Tage, 5 Tage, 6 Tage, 1 Woche, 2 Wochen, 3 Wochen, 4 Wochen, 1 Monat, 2 Monate, 3 Monate, 4 Monate, 5 Monate, 6 Monate, 7 Monate, 8 Monate, 9 Monate, 10 Monate, 11 Monaten, 1 Jahr, 2 Jahren oder 3 Jahren im Vergleich zum Infektiositätsverlust einer unter den gleichen Bedingungen gelagerten Referenzformulierung; oder

d) rAAV-Genomfreisetzung, wobei die rAAV-Genomfreisetzung durch Messen der relativen Fluoreszenz unter Bevorzugung eines DNA-spezifischen Fluoreszenzfarbstoffs bestimmt wird, und wobei die Formulierung mindestens etwa 5%, etwa 10%, etwa 20%, etwa 30%, etwa 40%, etwa 50%, etwa 60%, etwa 70%, etwa 80%, etwa 90%, etwa 100% oder etwa 200% weniger relatives Fluoreszenzniveau nach Lagerung der Formulierung für 1 Tag, 2 Tage, 3 Tage, 4 Tage, 5 Tage, 6 Tage, 1 Woche, 2 Wochen, 3 Wochen, 4 Wochen, 1 Monat, 2 Monate, 3 Monaten, 4 Monaten, 5 Monaten, 6 Monaten, 7 Monaten, 8 Monaten, 9 Monaten, 10 Monaten, 11 Monaten, 1 Jahr, 2 Jahren oder 3 Jahren im Vergleich zum relativen Fluoreszenzniveau einer unter den gleichen Bedingungen gelagerten Referenzformulierung, optional wobei die Referenzformulierung ist:

(i) DPBS mit 0,001% Poloxamer 188-Puffer;
(ii) eine Formulierung, die keinen Zucker umfasst; oder
(iii) eine Formulierung, die keinen Weichmacher umfasst.

**19.** Formulierung gemäß einem der Ansprüche 1 bis 18, wobei

a) die Formulierung bei -80°C, -70°C, -20°C, 4°C, 20°C, 25°C, 30°C, 35°C oder 40°C gelagert wird;

b) die Formulierung im Lyophilisierungsprozess auf eine Temperatur von etwa -20°C gefroren wird;

c) die gefrorene Formulierung beim Einfrieren auf -20°C einen pH-Wert zwischen etwa pH 6 und etwa pH 9 beibehält;

d) die gefrorene Formulierung beim Einfrieren auf -20°C einen pH-Wert innerhalb eines Bereichs von plus oder minus 1 Einheit des pH-Werts vor dem Einfrieren beibehält; oder

e) die Formulierung eine stabilisierte wässrige Formulierung von rAAV zur Lyophilisierung ist.

**20.** Verfahren zum Produzieren einer stabilen Formulierung gemäß einem der Ansprüche 1 bis 19, wobei das Verfahren ein Kombinieren von rAAV-Partikeln, die ein AAV8-Kapsid umfassen, mit Tris, einem Zucker, einem amorphen Salz, optional einem Weichmacher und/oder einem nichtionischen Tensid umfasst.

**21.** Verfahren zum Reduzieren der Freisetzung des rAAV-Genoms aus rAAV-Partikeln, umfassend:

a) Produzieren einer Formulierung gemäß einem der Ansprüche 1 bis 19, wobei die Freisetzung des rAAV-Genoms aus den rAAV-Partikeln nach drei Einfrier-Auftau-Zyklen im Vergleich zur Freisetzung des rAAV-Genoms in einer Formulierung, die den Zucker nicht umfasst, reduziert ist; oder

b) Produzieren einer Formulierung gemäß einem der Ansprüche 1 bis 19, wobei die Freisetzung des rAAV-Genoms aus den rAAV-Partikeln nach Lyophilisierung und Rekonstitution im Vergleich zur Freisetzung des rAAV-Genoms in einer Formulierung, die den Zucker nicht umfasst, reduziert ist.

**22.** Verfahren zum Produzieren einer stabilen lyophilisierten Formulierung eines rAAV-Produkts, umfassend einen Schritt des Unterwerfens einer vorlyophilisierten Formulierung einer Lyophilisierung, wobei die vorlyophilisierte Formulierung gemäß einem der Ansprüche 1 bis 19 ist.

**23.** Formulierung gemäß einem der Ansprüche 1 bis 19 zur Verwendung in einem Verfahren zur Behandlung oder Vorbeugung einer Krankheit, wobei die Formulierung lyophilisiert und rekonstituiert wird, bevor sie einem Subjekt, das sie benötigt, verabreicht wird.


**Revendications**

**1.** Une formulation stable comprenant une particule de virus adéno-associé recombinant (rAAV) et

a) du Tris,

b) un sucre, et

c) un sel amorphe,

dans laquelle la formulation convient à une lyophilisation ; et

dans laquelle la particule de rAAV comprend une protéine capsidique AAV8.

**2.** La formulation selon la revendication 1, dans laquelle le sel amorphe a une force ionique supérieure ou égale à 60 mM, facultativement dans laquelle le sel amorphe a une force ionique comprise entre 60 mM et 150 mM.

**3.** La formulation selon la revendication 1 ou la revendication 2 , dans laquelle la formulation comprend :

a) entre environ 1 mM et environ 50 mM de Tris ; ou

b) entre environ 1 mM et environ 30 mM, entre environ 1 mM et environ 20 mM, entre environ 5 mM et environ 30 mM, entre environ 5 mM et environ 20 mM, entre environ 10 mM et environ 30 mM, entre environ 10 mM et environ 20 mM, ou entre environ 20 mM et environ 50 mM de Tris ; ou

c) environ 1 mM, environ 2 mM, environ 3 mM, environ 5 mM, environ 10 mM, environ 15 mM, environ 20 mM, environ 25 mM, environ 30 mM, ou environ 40 mM de Tris, facultativement environ 5 mM de Tris.

**4.** La formulation selon l'une quelconque des revendications 1 à 3, dans laquelle le sel amorphe est le citrate de sodium, le sulfate de sodium, le sulfate d'ammonium, le sulfate de magnésium, ou une de leurs combinaisons.

**5.** La formulation selon l'une quelconque des revendications 1 à 4, dans laquelle la formulation comprend environ 10 mM, environ 20 mM, environ 30 mM, environ 40 mM, environ 50 mM, environ 60 mM, environ 70 mM, environ 80 mM, environ 90 mM, environ 100 mM, environ 120 mM, environ 140 mM, environ 150 mM, ou environ 200 mM de sulfate de sodium.

6. La formulation selon l'une quelconque des revendications 1 à 5 ayant un pH de :

   a) entre environ 6,5 et 8,0 ;
   b) entre environ 7,2 et 7,8 ;
   c) environ 7,2, 7,3, 7,4, 7,5, 7,6, 7,7 ou 7,8 ;
   d) environ 7,5 ; ou
   e) environ 7,1.

7. La formulation selon l'une quelconque des revendications 1 à 6 comprenant :

   a) entre environ 50 mM et environ 400 mM de sucre ;
   b) entre environ 50 mM et environ 350 mM, entre environ 50 mM et environ 300 mM, entre environ 50 mM et environ 250 mM, entre environ 50 mM et environ 200 mM, ou entre environ 50 mM et environ 150 mM de sucre ;
   c) entre environ 100 mM et environ 400 mM, entre environ 150 mM et environ 400 mM, entre environ 200 mM et environ 400 mM, entre environ 250 mM et environ 400 mM, ou entre environ 300 mM et environ 400 mM de sucre ;
   d) entre environ 100 mM et environ 300 mM, entre environ 150 mM et environ 250 mM, entre environ 200 mM et environ 300 mM, ou entre environ 250 mM et environ 350 mM de sucre ;
   e) environ 50 mM, environ 100 mM, environ 150 mM, environ 160 mM, environ 170 mM, environ 180 mM, environ 190 mM, environ 200 mM, environ 210 mM, environ 220 mM, environ 230 mM, environ 240 mM, environ 250 mM, environ 260 mM, environ 270 mM, environ 280 mM, environ 290 mM, environ 300 mM, ou environ 350 mM de sucre ;
   f) entre environ 190 mM et environ 230 mM, entre environ 170 mM et environ 250 mM, ou entre environ 250 mM et environ 270 mM de sucre ; ou
   g) environ 210 mM de sucre.

8. La formulation selon l'une quelconque des revendications 1 à 7, dans laquelle le sucre est :

   a) un sucre non réducteur, facultativement dans laquelle le sucre non réducteur est le saccharose, le tréhalose ou le raffinose ; ou
   b) un sucre réducteur, facultativement dans laquelle le sucre réducteur est le glucose, le fructose, le mannose, le galactose ou le lactose.

9. La formulation selon l'une quelconque des revendications 1 à 8 comprenant :

   a) moins d'environ 100 mM de citrate de sodium ;
   b) entre environ 10 mM et environ 100 mM de citrate de sodium ;
   c) entre environ 10 mM et environ 100 mM, entre environ 20 mM et environ 100 mM, entre environ 30 mM et environ 100 mM, entre environ 40 mM et environ 100 mM, entre environ 50 mM et environ 100 mM, entre environ 10 mM et environ 80 mM, entre environ 10 mM et environ 60 mM, entre environ 10 mM et environ 50 mM, entre environ 10 mM et environ 40 mM, ou entre environ 10 mM et environ 30 mM de citrate de sodium ;
   d) entre environ 10 mM et environ 50 mM, entre environ 20 mM et environ 60 mM, entre environ 30 mM et environ 70 mM, ou entre environ 10 mM et environ 30 mM de citrate de sodium ;
   e) environ 10 mM, environ 20 mM, environ 30 mM, environ 40 mM, environ 50 mM, ou environ 60 mM de citrate de sodium ; ou
   f) environ 20 mM de citrate de sodium.

10. La formulation selon l'une quelconque des revendications 1 à 9, comprenant en outre :

   a) entre environ 0,0005 % et environ 0,01 % de tensioactif non ionique ; ou
   b) environ 0,002 % de tensioactif non ionique,

   facultativement dans laquelle le tensioactif non ionique comprend le poloxamère 188, le poloxamère 407, le polysorbate 80, le polysorbate 20, le Pluronic F-68 ou le BRIJ 35.

11. La formulation selon l'une quelconque des revendications 1 à 10, comprenant en outre un stabilisant sélectionné parmi le groupe consistant en le glycérol, ou le sorbitol, facultativement dans laquelle la formulation comprend :

    a) entre environ 0,1 % et entre environ 5 % de glycérol ;
    b) entre environ 0,1 % et entre environ 2 % de glycérol ; ou
    c) entre environ 0,25 % et entre environ 2 % de glycérol.

12. La formulation selon l'une quelconque des revendications 1 à 11, dans laquelle :

    a) la formulation ne comprend pas de mannitol ; ou
    b) la formulation comprend moins de 10 mM, 20 mM, 50 mM, 100 mM ou 150 mM de mannitol.

13. La formulation selon l'une quelconque des revendications 1 à 12 comprenant :

    A.

        a) entre environ 1 mM et environ 25 mM de Tris,
        b) entre environ 50 mM et environ 400 mM de sucre,
        c) entre environ 10 mM et environ 100 mM de citrate de sodium, et
        d) entre environ 0,0005 % et environ 0,01 % de tensioactif non ionique,

    dans laquelle la formulation a un pH compris entre environ 7,2 et environ 7,8 ;
    B.

        a) entre environ 2 mM et environ 10 mM de Tris,
        b) entre environ 150 mM et environ 250 mM de sucre,
        c) entre environ 10 mM et environ 20 mM de citrate de sodium, et
        d) entre environ 0,001 % et environ 0,005 % de tensioactif non ionique,

    dans laquelle la formulation a un pH compris entre environ 7,2 et environ 7,8 ;
    C.

        a) environ 5 mM de Tris,
        b) environ 210 mM de sucre,
        c) environ 20 mM de citrate de sodium, et
        d) environ 0,002 % de tensioactif non ionique,

    dans laquelle la formulation a un pH d'environ 7,5 ;
    D.

        a) environ 5 mM de Tris,
        b) environ 210 mM de saccharose,
        c) environ 20 mM de citrate de sodium, et
        d) environ 0,002 % (p/v) de poloxamère 188 ;

    E.

        a) environ 5 mM de Tris,
        b) environ 210 mM de saccharose,
        c) environ 20 mM de citrate de sodium,
        d) environ 0,002 % (p/v) de poloxamère 188, et,
        e) environ 0,25 % (p/v) de glycérol ;

    F.

        a) environ 5 mM de Tris,
        b) environ 210 mM de saccharose,
        c) environ 20 mM de citrate de sodium,

d) environ 0,002 % (p/v) de poloxamère 188, et
e) environ 0,5 % (p/v) de sorbitol ;

G.

a) environ 5 mM de Tris,
b) environ 30 mM de sulfate de sodium,
c) environ 263 mM de saccharose, et
d) environ 0,005 % (p/v) de poloxamère 188 ;

H. entre environ 1,0E+11 copies de génome/mL (GC/mL) et environ 1,0E+15 GC/mL de particules de rAAV ; ou
I. environ 1,0E+11 GC/mL, environ 1,0E+12 GC/mL, environ 1,0E+13 GC/mL, environ 1,0E+14 GC/mL, ou environ 1,0E+15 GC/mL de particules de rAAV.

**14.** La formulation selon l'une quelconque des revendications 1 à 13 ayant une teneur en humidité résiduelle de :

a) entre environ 1 % et environ 7 % ;
b) entre environ 1 % et environ 7 %, entre environ 2 % et environ 7 %, entre environ 3 % et environ 7 %, entre environ 4 % et environ 7 %, entre environ 5 % et environ 7 %, entre environ 1 % et environ 6 %, entre environ 1 % et environ 5 %, entre environ 1 % et environ 4 %, ou entre environ 1 % et environ 3 % ;
c) entre environ 3 % et environ 7 %, entre environ 3 % et environ 6 %, ou entre environ 3 % et environ 5 % ;
d) environ 3 %, environ 3,5 %, environ 4 %, environ 4,5 %, environ 5 %, environ 5,5 % ou environ 6 % ;
e) entre environ 1 % et environ 2 % ;
f) environ 1,5 %, environ 1,4 %, environ 1,3 %, environ 1,2 % ou environ 1,1 % ; ou
g) environ 1 %.

**15.** La formulation selon l'une quelconque des revendications 1 à 14, dans laquelle :

a) la température de transition vitreuse (Tg) des gâteaux lyophilisés de la formulation est supérieure à 35 °C ; ou
b) la température de transition vitreuse de la solution concentrée au maximum au congélateur (Tg') de la formulation est supérieure à -40 °C.

**16.** La formulation selon l'une quelconque des revendications 1 à 15, dans laquelle le % de puissance relative des particules de rAAV est :

a) au moins environ 60 %, au moins environ 70 %, ou au moins environ 80 % après stockage de la formulation pendant 3 mois à température ambiante ; dans laquelle les particules de rAAV de référence sont stockées à -70 °C dans du DPBS avec un tampon de poloxamère 188 à 0,001 % ;
b) au moins environ 60 %, au moins environ 70 %, ou au moins environ 80 % après stockage de la formulation pendant 6 mois à température ambiante ; dans laquelle les particules de rAAV de référence sont stockées à -70 °C dans une solution saline tamponnée au phosphate de Dulbecco (DPBS) avec un tampon de poloxamère 188 à 0,001 % ;
c) au moins environ 30 %, au moins environ 40 %, au moins environ 50 %, ou au moins 60 % après stockage de la formulation pendant 1 semaine à 35 °C, dans laquelle les particules de rAAV de référence sont stockées à -70 °C dans du DPBS avec un tampon de poloxamère 188 à 0,001 % ;
d) au moins environ 30 %, au moins environ 40 %, au moins environ 50 %, ou au moins 60 % après stockage de la formulation pendant 2 semaines à 35 °C, dans laquelle les particules de rAAV de référence sont stockées à -70 °C dans du DPBS avec un tampon de poloxamère 188 à 0,001 % ; ou
e) au moins environ 30 %, au moins environ 40 %, au moins environ 50 %, ou au moins 60 % après stockage de la formulation pendant 4 semaines à 35 °C, dans laquelle les particules de rAAV de référence sont stockées à -70 °C dans du DPBS avec un tampon de poloxamère 188 à 0,001 %.

**17.** La formulation selon la revendication 16, dans laquelle la formulation est lyophilisée avant stockage, facultativement dans laquelle la formulation lyophilisée est reconstituée après stockage.

**18.** La formulation selon l'une quelconque des revendications 1 à 17, dans laquelle :

A. le % de puissance relative des particules de rAAV est d'au moins environ 25 %, d'au moins environ 30 %, d'au moins environ 40 %, d'au moins environ 50 %, d'au moins environ 60 %, d'au moins environ 70 %, d'au moins environ 80 %, d'au moins environ 90 %, d'au moins environ 95 %, ou d'au moins environ 99 % juste après la lyophilisation ;

B. le niveau d'agrégation particulaire de rAAV de la formulation est diminué d'environ 10 %, d'environ 20 %, d'environ 30 %, d'environ 40 %, d'environ 50 %, d'environ 60 %, d'environ 70 %, d'environ 80 %, d'environ 90 %, ou d'environ 100 % par rapport au niveau d'agrégation de particules de rAAV dans une formulation de référence ; ou

C. la stabilité de la formulation est évaluée par :

a) le dosage du contenu du génome vectoriel ou du titre viral, dans laquelle la formulation a au moins environ 5 %, environ 10 %, environ 20 %, environ 30 %, environ 40 %, environ 50 %, environ 60 %, environ 70 %, environ 80 %, environ 90 %, environ 100 % ou environ 200 %, de plus de contenu génomique après stockage de la formulation pendant 1 jour, 2 jours, 3 jours, 4 jours, 5 jours, 6 jours, 1 semaine, 2 semaines, 3 semaines, 4 semaines, 1 mois, 2 mois, 3 mois, 4 mois, 5 mois, 6 mois, 7 mois, 8 mois, 9 mois, 10 mois, 11 mois, 1 an, 2 ans ou 3 ans par rapport au contenu génomique d'une formulation de référence stockée dans les mêmes conditions ;

b) la mesure de la puissance relative de la formulation, dans laquelle la formulation a au moins environ 5 %, environ 10 %, environ 20 %, environ 30 %, environ 40 %, environ 50 %, environ 60 %, environ 70 %, environ 80 %, environ 90 %, environ 100 %, ou environ 200 %, de plus de puissance relative après stockage de la formulation pendant 1 jour, 2 jours, 3 jours, 4 jours, 5 jours, 6 jours, 1 semaine, 2 semaines, 3 semaines, 4 semaines, 1 mois, 2 mois, 3 mois, 4 mois, 5 mois, 6 mois, 7 mois, 8 mois, 9 mois, 10 mois, 11 mois, 1 an, 2 ans ou 3 ans par rapport au contenu génomique d'une formulation de référence stockée dans les mêmes conditions ;

c) la perte d'infectiosité, dans laquelle la formulation a au moins environ 5 %, environ 10 %, environ 20 %, environ 30 %, environ 40 %, environ 50 %, environ 60 %, environ 70 %, environ 80 %, environ 90 %, environ 100 % ou environ 200 %, de moins de perte d'infectiosité après stockage de la formulation pendant 1 jour, 2 jours, 3 jours, 4 jours, 5 jours, 6 jours, 1 semaine, 2 semaines, 3 semaines, 4 semaines, 1 mois, 2 mois, 3 mois, 4 mois, 5 mois, 6 mois, 7 mois, 8 mois, 9 mois, 10 mois, 11 mois, 1 an, 2 ans, ou 3 ans par rapport à la perte d'infectiosité d'une formulation de référence stockée dans les mêmes conditions ; ou

d) la libération du génome rAAV, dans laquelle la libération du génome rAAV est déterminée par la mesure de la fluorescence relative de préférence d'une coloration fluorescente spécifique à l'ADN, et dans laquelle la formulation a au moins environ 5 %, environ 10 %, environ 20 %, environ 30 %, environ 40 %, environ 50 %, environ 60 %, environ 70 %, environ 80 %, environ 90 %, environ 100 %, ou environ 200 %, de moins de niveau de fluorescence relative après stockage de la formulation pendant 1 jour, 2 jours, 3 jours, 4 jours, 5 jours, 6 jours, 1 semaine, 2 semaines, 3 semaines, 4 semaines, 1 mois, 2 mois, 3 mois, 4 mois, 5 mois, 6 mois, 7 mois, 8 mois, 9 mois, 10 mois, 11 mois, 1 an, 2 ans ou 3 ans par rapport au niveau relatif de fluorescence d'une formulation de référence stockée dans les mêmes conditions, facultativement dans laquelle la formulation de référence est :

(i) le DPBS avec le tampon de poloxamère 188 à 0,001 % ;
(ii) une formulation ne comprenant pas de sucre ; ou
(iii) une formulation ne comprenant pas de plastifiant.

**19.** La formulation selon l'une quelconque des revendications 1 à 18, dans laquelle

a) la formulation est stockée à -80 °C, -70 °C, -20 °C, 4 °C, 20 °C, 25 °C, 30 °C, 35 °C, ou 40 °C ;
b) la formulation est congelée à une température d'environ -20 °C au cours du processus de lyophilisation ;
c) la formulation congelée maintient le pH entre environ pH 6 et environ pH 9 lors de la congélation jusqu'à -20 °C ;
d) la formulation congelée maintient une valeur de pH dans une plage de plus ou moins 1 unité de la valeur de pH avant la congélation lors de la congélation jusqu'à -20 °C ; ou
e) la formulation est une formulation aqueuse stabilisée de rAAV pour la lyophilisation.

**20.** Un procédé de production d'une formulation stable selon l'une quelconque des revendications 1 à 19, dans lequel le procédé comprend une combinaison de particules de rAAV qui comprennent un capside AAV8, avec du Tris, un sucre, un sel amorphe, facultativement un plastifiant, et/ou un tensioactif non ionique.

**21.** Un procédé de réduction de la libération du génome de rAAV à partir de particules de rAAV, comprenant :

a) une production d'une formulation selon l'une quelconque des revendications 1 à 19, dans lequel la libération du génome de rAAV à partir des particules de rAAV après trois cycles de congélation-décongélation est réduite par rapport à la libération du génome de rAAV dans une formulation ne comprenant pas le sucre ; ou

b) une production d'une formulation selon l'une quelconque des revendications 1 à 19, dans lequel la libération du génome de rAAV à partir des particules de rAAV après lyophilisation et reconstitution est réduite par rapport à la libération du génome de rAAV dans une formulation ne comprenant pas le sucre.

22. Un procédé de production d'une formulation lyophilisée stable d'un produit rAAV, comprenant une étape de soumission à lyophilisation d'une formulation pré-lyophilisée, dans lequel la formulation pré-lyophilisée est selon l'une quelconque des revendications 1 à 19.

23. Une formulation telle que définie dans l'une quelconque des revendications 1 à 19 pour l'utilisation dans un procédé de traitement ou de prévention d'une maladie, dans laquelle la formulation est lyophilisée et reconstituée avant d'être administrée à un sujet en ayant besoin.

**FIG 1A**

**FIG 1B**

**FIG 2**

**FIG 3**

**FIG 4**

**FIG 5A**

**FIG 5B**

**FIG 6**

**FIG 7A**

**FIG 7B**

**FIG 8**

FIG 9

| | | Form. 1 | Form. 2 | Form. 3 |
|---|---|---|---|---|
| liquid | | | | |
| frozen | | | | |
| approx initial pH | | 7.5 | 7.5 | 6.5 |
| pH shift? | | yes | no | no |
| relative potency | T0 | 80% | 87% | 75% |
| | 6M at 25°C | 18% | 74% | 6% |

Universal Indicator pH Color Chart

4  5  6  7  8  9  10

# FIG 10

**FIG 11**

**FIG 12**

**FIG 13**

FIG 14

FIG 15A  FIG 15B  FIG 15C

# FIG 15

**FIG 16**

**FIG 17A**

**FIG 17B**

**FIG 17C**

**FIG 18A**

**FIG 18B**

**FIG 18C**

**FIG 19A**

**FIG 19B**

VP1₁₋₇₃₆→

VP1<sub>1-736</sub>→

```
        VP1_{1-736}→
AAV1    MAADGYLPDWLEDNLSEGIREWWDLKPGAPKPKANQQKQDDGRGLVLPGYKYLGPFNGLD  60
AAV2    MAADGYLPDWLEDTLSEGIRQWWKLKPGPPPPKPAERHKDDSRGLVLPGYKYLGPFNGLD  60
AAV3-3  MAADGYLPDWLEDNLSEGIREWWALKPGVPQPKANQQHQDNRRGLVLPGYKYLGPGNGLD  60
AAV4-4  -MTDGYLPDWLEDNLSEGVREWWALQPGAPKPKANQQHQDNARGLVLPGYKYLGPGNGLD  59
AV5     MSFVDHPPDWLEE-VGEGLREFLGLEAGPPKPKPNQQHQDQARGLVLPGYNYLGPGNGLD  59
AAV6    MAADGYLPDWLEDNLSEGIREWWDLKPGAPKPKANQQKQDDGRGLVLPGYKYLGPFNGLD  60
AAV7    MAADGYLPDWLEDNLSEGIREWWDLKPGAPKPKANQQKQDNGRGLVLPGYKYLGPFNGLD  60
AAV8    MAADGYLPDWLEDNLSEGIREWWALKPGAPKPKANQQKQDDGRGLVLPGYKYLGPFNGLD  60
hu31    MAADGYLPDWLEDTLSEGIRQWWKLKPGPPPPKPAERHKDDSRGLVLPGYKYLGPGNGLD  60
hu32    MAADGYLPDWLEDTLSEGIRQWWKLKPGPPPPKPAERHKDDSRGLVLPGYKYLGPGNGLD  60
AAV9    MAADGYLPDWLEDNLSEGIREWWALKPGAPQPKANQQHQDNARGLVLPGYKYLGPGNGLD  60
SUBS    -STVDHP-----ETVG--V-QFLK-QA-P-K---PAERKK-DG--------N----F----
          MF           L    DE V  P              QS
                                G    Q              R


AAV1    KGEPVNAADAAALEHDKAYDQQLKAGDNPYLRYNHADAEFQERLQEDTSFGGNLGRAVFQ  120
AAV2    KGEPVNEADAAALEHDKAYDRQLDSGDNPYLKYNHADAEFQERLKEDTSFGGNLGRAVFQ  120
AAV3-3  KGEPVNEADAAALEHDKAYDQQLKAGDNPYLKYNHADAEFQERLQEDTSFGGNLGRAVFQ  120
AAV4-4  KGEPVNAADAAALEHDKAYDQQLKAGDNPYLKYNHADAEFQQRLQGDTSFGGNLGRAVFQ  119
AV5     RGEPVNRADEVAREHDISYNEQLEAGDNPYLKYNHADAEFQEKLADDTSFGGNLGKAVFQ  119
AAV6    KGEPVNAADAAALEHDKAYDQQLKAGDNPYLRYNHADAEFQERLQEDTSFGGNLGRAVFQ  120
AAV7    KGEPVNAADAAALEHDKAYDQQLKAGDNPYLRYNHADAEFQERLQEDTSFGGNLGRAVFQ  120
AAV8    KGEPVNAADAAALEHDKAYDQQLQAGDNPYLRYNHADAEFQERLQEDTSFGGNLGRAVFQ  120
hu31    KGEPVNAADAAALEHDKAYDQQLKAGDNPYLKYNHADAEFQERLKEDTSFGGNLGRAVFQ  120
hu32    KGEPVNAADAAALEHDKAYDQQLKAGDNPYLKYNHADAEFQERLKEDTSFGGNLGRAVFQ  120
AAV9    KGEPVNAADAAALEHDKAYDQQLKAGDNPYLKYNHADAEFQERLKEDTSFGGNLGRAVFQ  120
SUBS    R-----E--EV-R---IS-NE--DS------R---------QK-QD---------K----
              R        R   E                      AG
                          Q


        VP2_{138}→   ┌--NVR1--┐
AAV1    AKKRVLEPLGLVEEGAKTAPGKKRPVEQSPQ-EPDSSSGIGKTGQQPAKKRLNFGQTGDS  179
AAV2    AKKRVLEPLGLVEEPVKTAPGKKRPVEHSPV-EPDSSSGTGKAGQQPARKRLNFGQTGDA  179
AAV3-3  AKKRILEPLGLVEEAAKTAPGKKGAVDQSPQ-EPDSSSGVGKSGKQPARKRLNFGQTGDS  179
AAV4-4  AKKRVLEPLGLVEQAGETAPGKKRPLIESPQ-QPDSSTGIGKKGKQPAKKKLVFEDETGA  178
AV5     AKKRVLEPFGLVEEGAKTAPTGKRIDDHFP---------------KRKKARTEEDSKPSTSSDA  168
AAV6    AKKRVLEPFGLVEEGAKTAPGKKRPVEQSPQ-EPDSSSGIGKTGQQPAKKRLNFGQTGDS  179
AAV7    AKKRVLEPLGLVEEGAKTAPAKKRPVEPSPQRSPDSSTGIGKKGQQPARKRLNFGQTGDS  180
AAV8    AKKRVLEPLGLVEEGAKTAPGKKRPVEPSPQRSPDSSTGIGKKGQQPARKRLNFGQTGDS  180
hu31    AKKRLLEPLGLVEEAAKTAPGKKRPVEQSPQ-EPDSSAGIGKSGSQPAKKKLNFGQTGDT  179
hu32    AKKRLLEPLGLVEEAAKTAPGKKRPVEQSPQ-EPDSSAGIGKSGSQPAKKKLNFGQTGDT  179
AAV9    AKKRLLEPLGLVEEAAKTAPGKKRPVEQSPQ-EPDSSAGIGKSGAQPAKKRLNFGQTGDT  179
SUBS    ----V---F----QGGE---TG-GIDDHF-V-S----S-T--KKQARTREKSVPEDETGA
            I        FV    A   ALIP   Q        TV   T K   E D K STSS S
                              E                      A S
                                                     R A
```

# FIG 20

```
              ┌─HVR2─┐                    VP3₂₀₃→
AAV1    ESVPD--PQPLGEPPATPAAVGPTTMASGGGAPMADNNEGADGVGNASGNWHCDSTWLGDR 238
AAV2    DSVPD--PQPLGQPPAAPSGLGTNTMATGSGAPMADNNEGADGVGNSSGNWHCDSTWMGDR 238
AAV3-3  ESVPD--PQPLGEPPAAPTSLGSNTMASGGGAPMADNNEGADGVGNSSGNWHCDSQWLGDR 238
AAV4-4  GDSP-----PEGSTSGAMS--DDSEMRAAAGGAAVEGGQGADGVGNASGDWHCDSTWSEGH 232
AV5     EAGPSGSQQLQIPAQPASSLGADTMSAGGGGPLGDNNQGADGVGNASGDWHCDSTWMGDR 228
AAV6    ESVPD--PQPLGEPPATPAAVGPTTMASGGGAPMADNNEGADGVGNASGNWHCDSTWLGDR 238
AAV7    ESVPD--PQPLGEPPAAPSSVGSGTVAAGGGAPMADNNEGADGVGNASGNWHCDSTWLGDR 239
AAV8    ESVPD--PQPLGEPPAAPSGVGPNTMAAGGGAPMADNNEGADGVGSSSGNWHCDSTWLGDR 239
hu31    ESVPD--PQPIGEPPAAPSGVGSLTMASGGGAPVADNNEGADGVGSSSGNWHCDSQWLGDR 238
hu32    ESVPD--PQPIGEPPAAPSGVGSLTMASGGGAPVADNNEGADGVGSSSGNWHCDSQWLGDR 238
AAV9    ESVPD--PQPIGEPPAAPSGVGSLTMASGGGAPVADNNEGADGVGSSSGNWHCDSQWLGDR 238
SUBS    GDG-S-S-QLQQTSGTMASLDPNEVRAAA-GAMGEGGQ------NA--D-----T-MEGH
        DA      E  S AQPATA  AG    ST S    LV              S
                I          --  DT             A
                               TD
                               S


              ┌─HVR3─┐
AAV1    VITTSTRTWALPTYNNHLYKQIS-SASTGASNDNHYFGYSTPWGYFDFNRFHCHFSPRDW 297
AAV2    VITTSTRTWALPTYNNHLYKQIS--SQSGASNDNHYFGYSTPWGYFDFNRFHCHFSPRDW 296
AAV3-3  VITTSTRTWALPTYNNHLYKQIS--SQSGASNDNHYFGYSTPWGYFDFNRFHCHFSPRDW 296
AAV4-4  VTTTSTRTWVLPTYNNHLYKRLG-----ESLQSNTYNGFSTPWGYFDFNRFHCHFSPRDW 287
AV5     VVTKSTRTWVLPSYNNHQYREIKS-GSVDGSNANAYFGYSTPWGYFDFNRFHSHWSPRDW 287
AAV6    VITTSTRTWALPTYNNHLYKQISSAST-GASNDNHYFGYSTPWGYFDFNRFHCHFSPRDW 297
AAV7    VITTSTRTWALPTYNNHLYKQISS-ETAGSTNDNTYFGYSTPWGYFDFNRFHCHFSPRDW 298
AAV8    VITTSTRTWALPTYNNHLYKQISNGTSGGATNDNTYFGYSTPWGYFDFNRFHCHFSPRDW 299
hu31    VITTSTRTWALPTYNNHLYKQISNSTSGGSSNDNAYFGYSTPWGYFDFNRFHCHFSPRDW 298
hu32    VITTSTRTWALPTYNNHLYKQISNSTSGGSSNDNAYFGYSTPWGYFDFNRFHCHFSPRDW 298
AAV9    VITTSTRTWALPTYNNHLYKQISNSTSGGSSNDNAYFGYSTPWGYFDFNRFHCHFSPRDW 298
SUBS    -T-K-----V--S----Q-RRLGSGSQSDATQA-T--------------S-W-----
        V              E K AATTEGL S H
                           G V
                           E A


AAV1    QRLINNNWGFRPKRLNFKLFNIQVKEVTTNDGVTTIANNLTSTVQVFSDSEYQLPYVLGS 357
AAV2    QRLINNNWGFRPKRLNFKLFNIQVKEVTQNDGTTTIANNLTSTVQVFTDSEYQLPYVLGS 356
AAV3-3  QRLINNNWGFRPKKLSFKLFNIQVRGVTQNDGTTTIANNLTSTVQVFTDSEYQLPYVLGS 356
AAV4-4  QRLINNNWGMRPKAMRVKIFNIQVKEVTTSNGETTVANNLTSTVQIFADSSYELPYVMDA 347
AV5     QRLINNYWGFRPRSLRVKIFNIQVKEVTVQDSTTTIANNLTSTVQVFTDDDYQLPYVVGN 347
AAV6    QRLINNNWGFRPKRLNFKLFNIQVKEVTTNDGVTTIANNLTSTVQVFSDSEYQLPYVLGS 357
AAV7    QRLINNNWGFRPKKLRFKLFNIQVKEVTTNDGVTTIANNLTSTIQVFSDSEYQLPYVLGS 358
AAV8    QRLINNNWGFRPKRLSFKLFNIQVKEVTQNEGTKTIANNLTSTIQVFTDSEYQLPYVLGS 359
hu31    QRLINNNWGFRPKRLNFKLFNIQVKEVTDNNGVKTIANNLTSTVQVFTDSDYQLPYVLGS 358
hu32    QRLINNNWGFRPKRLNFKLFNIQVKEVTDNNGVKTIANNLTSTVQVFTDSDYQLPYVLGS 358
AAV9    QRLINNNWGFRPKRLNFKLFNIQVKEVTDNNGVKTIANNLTSTVQVFTDSDYQLPYVLGS 358
SUBS    ---------M--RAMRV-I--------VQDSTT---------I-I-S-DE-E----MDA
                 K S                QSE E          A  S
                 S
```

# FIG 20 (continued)

```
                                        ┌HVR4┐
AAV1     AHQGCLPPFPADVFMIPQYGYLTLNNG---SQAVGRSSFYCLEYFPSQMLRTGNNFTFSY 414
AAV2     AHQGCLPPFPADVFMVPQYGYLTLNNG---SQAVGRSSFYCLEYFPSQMLRTGNNFTFSY 413
AAV3-3   AHQGCLPPFPADVFMVPQYGYLTLNNG---SQAVGRSSFYCLEYFPSQMLRTGNNFQFSY 413
AAV4-4   GQEGSLPPFPNDVFMVPQYGYCGLVTGNTSQQQTDRNAFYCLEYFPSQMLRTGNNFEITY 407
AV5      GTEGCLPAFPPQVFTLPQYGYATLRD-NTENPTERSSFFCLEYFPSKMLRTGNNFEFTY 406
AAV6     AHQGCLPPFPADVFMIPQYGYLTLNNG---SQAVGRSSFYCLEYFPSQMLRTGNNFTFSY 414
AAV7     AHQGCLPPFPADVFMIPQYGYLTLNNG---SQSVGRSSFYCLEYFPSQMLRTGNNFEFSY 415
AAV8     AHQGCLPPFPADVFMIPQYGYLTLNNG---SQAVGRSSFYCLEYFPSQMLRTGNNFQFTY 416
hu31     AHEGCLPPFPADVFMIPQYGYLTLNDG---GQAVGRSSFYCLEYFPSQMLRTGNNFQFSY 415
hu32     AHEGCLPPFPADVFMIPQYGYLTLNDG---SQAVGRSSFYCLEYFPSQMLRTGNNFQFSY 415
AAV9     AHEGCLPPFPADVFMIPQYGYLTLNDG---SQAVGRSSFYCLEYFPSQMLRTGNNFQFSY 415
SUBS     GQQ-S--A--PQ--TL-----CG-VND---GNPTD-NA-F----------------EIT-
         T         N   V      A    T    Q Q E                  T
                                       R   E S


                                     ┌---------HVR5---------┐
AAV1     TFEEVPFHSSYAHSQSLDRLMNPLIDQYLYYLNRTQ-NQSGSAQNKDLLFSRGSPAGMSV 473
AAV2     TFEDVPFHSSYAHSQSLDRLMNPLIDQYLYYLSRTN-TPSGTTTQSRLQFSQAGASDIRD 472
AAV3-3   TFEDVPFHSSYAHSQSLDRLMNPLIDQYLYYLNRTQGTTSGTTTQSRLFFSQAGPQSMSL 473
AAV4-4   SFEKVPFHSMYAHSQSLDRLMNPLIDQYLWGLQSTTTPTTLNAGTATTNFTKLRFTNFSN 467
AV5      NFEEVPFHSSFAPSQNLFKLANPLVDQYLRFVSTN-------NFGGVQFNKNLAGRYAN 459
AAV6     TFEDVPFHSSYAHSQSLDRLMNPLIDQYLYYLNRTQ-NQSGSAQNKDLLFSRGSPAGMSV 473
AAV7     SFEDVPFHSSYAHSQSLDRLMNPLIDQYLYYLARTQSNPGGTAGNRELQFYQGGPSTMAE 475
AAV8     TFEDVPFHSSYAHSQSLDRLMNPLIDQYLYYLSRTQT-TGGTANTQTLGFSQGGPNTMAN 475
hu31     EFENVPFHSSYAHSQSLDRLMNPLIDQYLYYLSKTINGSG--QNQQTLKFSVAGPSNMAV 473
hu32     EFENVPFHSSYAHSQSLDRLMNPLIDQYLYYLSKTINGSG--QNQQTLKFSVAGPSNMAV 473
AAV9     EFENVPFHSSYAHSQSLDRLMNPLIDQYLYYLSKTINGSG--QNQQTLKFSVAGPSNMAV 473
SUBS     T--D-----MF---------A---V----WGFNR-QTNTS--AGTKRTQ-TQGSAATFSN
         S    E                     QS  NSTPT   TQNSDVN NKNL QGYRD
         N    K                     V   TG Q     T AE L YRLR TRI L
         A                                RG  G        GS  E
                                                          ND


         ┌┐                   ┌HVR6┐       ┌HVR7┐      ┌---HVR8--┐
AAV1     QPKNWLPGPCYRQQRVSKTKTDN-----NNSNFTWTGASKYNLNGRESIINPGTAMASHK 528
AAV2     QSRNWLPGPCYRQQRVSKTSADN-----NNSEYSWTGATKYHLNGRDSLVNPGPAMASHK 527
AAV3-3   QARNWLPGPCYRQQRLSKTANDN-----NNSNFPWTAASKYHLNGRDSLVNPGPAMASHK 528
AAV4-4   FKKNWLPGPSIKQQGFSKTANQNYKIPATGSDSLIKYETHSTLDGRWSALTPGPPMATAG 527
AV5      TYKNWFPGPMGRTQGWNLGSGVN-----RASVSAFATTNRMELEGASYQVPFQFNGMTNN 514
AAV6     QPKNWLPGPCYRQQRVSKTKTDN-----NNSNFTWTGASKYNLNGRESIINPGTAMASHK 528
AAV7     QAKNWLPGPCFRQQRVSKTLDQN-----NNSNFAWTGATKYHLNGRNSLVNPGVAMATHK 530
AAV8     QAKNWLPGPCYRQQRVSTTTGQN-----NNSNFAWTAGTKYHLNGRNSLANPGIAMATHK 530
hu31     QGRNYIPGPSYRQQRVSTTVTQN-----NNSEPAWPGASSWALNGRNSLMNPGPAMASHK 528
hu32     QGRNYIPGPSYRQQRVSTTVTQN-----NNSEPAWPGASSWALNGRNSLMNPGPAMASHK 528
AAV9     QGRNYIPGPSYRQQRVSTTVTQN-----NNSEPAWPGASSWALNGRNSLMNPGPAMASHK 528
SUBS     FAK-WL---CIKT-GWNLGSGV------TG-DSLIKYETHST-D-ASYQVP-QTPGMTAG
         TP    F   MG    F K AND     RA NYTFATTNRME E  D ALT  VN  NN
         K         F     L KA       V  P TAG KYN   W II   I
         Y               LD         S    TAG   H   E  A
         S               T
```

# FIG 20 (continued)

```
                        ┌---------HVR9----------┐                              ┌---┐
AAV1     DDEDKFFPMSGVMIFGKESA---GASNTALD-NVMITDEEEIKATNPVATERFGTVAVNFQ   585
AAV2     DDEEKFFPQSGVLIFGKQGS---ERTNVDIE-KVMITDEEEIRTTNPVATEQYGSVSTNLQ   584
AAV3-3   DDEEKFFPMHGNLIFGKEGT---TASNAELD-NVMITDEEEIRTTNPVATEQYGTVANNLQ   585
AAV4-4   PADSKFS-NSQLIFAGPKQN---GNTATVPG-TLIFTSEEELAATNATDTDMWGNLPGGDQ   583
AV5      LQGSNTYALENTMIFNSQPANPGTTATYLEGNMLITSESETQPVIRVAYNVGGQMATNNQ   574
AAV6     DDKDKFFPMSGVMIFGKESA---GASNTALD-NVMITDEEEIKATNPVATERFGTVAVNLQ   585
AAV7     DDEDRFFPSSGVLIFGKTGA---TN-KTTLE-NVLMTNEEEIRPTNPVATEEYGIVSSNLQ   596
AAV8     DIEEREFPSNGILIFGKQNA---ARDNADYG-DVMLTSEEEIKTTNPVATEEYGIVADNLQ   587
hu31     EGEDRFFPLSGSLIFGKQGT---GRDNVDAD-KVMITNEEEIKTTNPVATESYGQVATNHQ   585
hu32     EGEDRFFPLSGSLIFGKQGT---GRDNVDAD-KVMITNEEEIKTTNPVATESYGQVATNHQ   585
AAV9     EGEDRFFPLSGSLIFGKQGT---GRDNVDAD-KVMITNEEEIKTTNPVATESYGQVATNHQ   585
SUBS     LQGSNTYAMENTMFANPKQN--TNTATVPG-TLIF-S-S-TQFV-ATDYDMW-NLPGGD-
         PADEK S QHQLI    SESA   EASKAALE-NMLM D   RA  R    NVF TMSN L
         DDK      NN V    TPS    AK  KTY   L      A        QG  I   V N
                   S  I      N        EI                       E       S  S F
                   N            Y                           R         D
```

```
         ┌--HVR10---┐
AAV1     SSSTDPATGDVHAMGALPGMVWQDRDVYLQGPIWAKIPHTDGHFHPSPLMGGFGLKNPPP   645
AAV2     RGNRQAATADVNTQGVLPGMVWQDRDVYLQGPIWAKIPHTDGHFHPSPLMGGFGLKHPPP   644
AAV3-3   SSNTAPTTGTVNHQGALPGMVWQDRDVYLQGPIWAKIPHTDGHFHPSPLMGGFGLKHPPP   645
AAV4-4   CNSNLPTVDRLTALGAVPGMVWQNRDIYYQGPIWAKIPHTDGHFHPSPLIGGFGLKHPPP   643
AV5      SSTTAPATGTYNLQEIVPGSVWMERDVYLQGPIWAKIPETGAHFHPSPAMGGFGLKHPPP   634
AAV6     SSSTDPATGDVHVMGALPGMVWQDRDVYLQGPIWAKIPHTDGHFHPSPLMGGFGLKHPPP   645
AAV7     AANTAAQTQVVNNQGALPGMVWQNRDVYLQGPIWAKIPHTDGNFHPSPLMGGFGLKHPPP   646
AAV8     QQNTAPQIGTVNSQGALPGMVWQNRDVYLQGPIWAKIPHTDGNFHPSPLMGGFGLKHPPP   647
hu31     SAQAQAQTGWVQNQGILPGMVWQDRDVYLQGPIWAKIPHTDGNFHPSPLMGGFGMKHPPP   645
hu32     SAQAQAQTGWVQNQGILPGMVWQDRDVYLQGPIWAKIPHTDGNFHPSPLMGGFGMKHPPP   645
AAV9     SAQAQAQTGWVQNQGILPGMVWQDRDVYLQGPIWAKIPHTDGNFHPSPLMGGFGMKHPPP   645
SUBS     RNSNLPTVDRLTALEAV--S--ME--I----------E-GAH-----AI----L-N---
         ASNTA AIADYHTM V           N
         QGTRD     QT NH
           Q       V  L
                      V
                      S
```

```
                 ┌---HVR11-┐
AAV1     QILIKNTPVPANPPAEFSATKFASFITQYSTGQVSVEIEWELQKENSKRWNPEVQYTSNY   705
AAV2     QILIKNTPVPANPSTTFSAAKFASFITQYSTGQVSVEIEWELQKENSKRWNPEIQYTSNY   704
AAV3-3   QIMIKNTPVPANPPTTFSPAKFASFITQYSTGQVSVEIEWELQKENSKRWNPEIQYTSNY   705
AAV4-4   QIFIKNTPVPANPATTFSSTPVNSFITQYSTGQVSVQIDWEIQKERSKRWNPEVQFTSNY   703
AV5      MMLIKNTPVPGNI-TSFSDVPVSSFITQYSTGQVTVEMEWELKKENSKRWNPEIQYTNNY   693
AAV6     QILIKNTPVPANPPAEFSATKFASFITQYSTGQVSVEIEWELQKENSKRWNPEVQYTSNY   705
AAV7     QILIKNTPVPANPPEVFTPAKFASFITQYSTGQVSVEIEWELQKENSKRWNPEIQYTSNF   706
AAV8     QILIKNTPVPADPPTTFNQSKLNSFITQYSTGQVSVEIEWELQKENSKRWNPEIQYTSNY   707
hu31     QILIKNTPVPADPPTAFNKDKLNSFITQYSTGQVSVEIEWELQKENSKRWNPEIQYTSNY   705
hu32     QILIKNTPVPADPPTAFNKDKLNSFITQYSTGQVSVEIEWELQKENSKRWNPEIQYTSNY   705
AAV9     QILIKNTPVPADPPTAFNKDKLNSFITQYSTGQVSVEIEWELQKENSKRWNPEIQYTSNY   705
SUBS     MMM------G-IAAE-SDVPVS--------------QMD--IK--R-------V------
           F          SET TAA FA
                      S   PT
                      V   QS
                          S
```

# FIG 20 (continued)

```
                    ┌─────HVR12──────┐
AAV1      AKSANVDFTVDNNGLYTEPRPIGTRYLTRPL 736   (SEQ ID NO. 41)
AAV2      NKSVNVDFTVDINGVYSEPRPIGTRYLTRNL 735   (SEQ ID NO. 42)
AAV3-3    NKSVNVDFTVDINGVYSEPRPIGTRYLTRNL 736   (SEQ ID NO. 43)
AAV4-4    GQQNSLLWAPDAACKYTEPRAIGTRYLTHHL 734   (SEQ ID NO. 44)
AV5       NDPQFVDFAPDSTGEYRTTRPIGTRYLTRPL 724   (SEQ ID NO. 45)
AAV6      AKSANVDFTVDNNGLYTEPRPIGTRYLTRPL 736   (SEQ ID NO. 46)
AAV7      EKQTGVDFAVDSQGVYSEPRPIGTRYLTRNL 737   (SEQ ID NO. 47)
AAV8      YKSTSVDFAVNTEGVYSEPRPIGTRYLTRNL 738   (SEQ ID NO. 48)
hu31      YKSNNVEFAVSTEGVYSEPRPIGTRYLTRNL 736   (SEQ ID NO. 49)
hu32      YKSNNVEFAVNTEGVYSEPRPIGTRYLTRNL 736   (SEQ ID NO. 50)
AAV9      YKSNNVEFAVNTEGVYSEPRPIGTRYLTRNL 736   (SEQ ID NO. 51)
SUBS      GQQVSLLWTPDAA-K-RTT-A-------HP-
          NDPQF D    SSN E T             H
          A     TG   NQ  L
          E     A        T
```

# FIG 20 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018128689 A1
- US 2002031527 A1
- WO 0029024 A1
- US 1956042 W
- US 9193956 B
- US 9458517 B
- US 9587282 B
- US 20160376323
- US 9585971 B
- US 9840719 B
- WO 2015013313 A
- WO 2014172669 A
- WO 2017070491 A
- US 8628966 B
- US 8927514 B
- US 9923120 B
- WO 2016049230 A
- US 7282199 B
- US 7906111 B
- US 8524446 B
- US 8999678 B
- US 8734809 B
- US 9284357 B
- US 9409953 B
- US 9169299 B
- US 20150374803
- US 20150126588 A
- US 20170067908 A

- US 20130224836 A
- US 20160215024 A
- US 20170051257 A
- US 2015034799 W
- EP 2015053335 W
- WO 2003052051 A
- WO 2005033321 A
- WO 03042397 A
- WO 2006068888 A
- WO 2006110689 A
- WO 2009104964 A
- WO 2010127097 A
- WO 2015191508 A
- US 20150023924 A
- US 6596535 B
- US 7125717 B
- US 7456683 B
- US 6723551 B
- US 6995006 B
- US 9783826 B
- US 20120122155
- US 2020033842 W
- WO 2019212921 A1
- US 6989264 B
- WO 2019241535 A
- WO 2019212922 A1
- US 9051542 B

**Non-patent literature cited in the description**

- **WRIGHT J FRASER et al.** Current Opinion in Drug Discovery and Development. *Current Drugs, London, GB*, 01 March 2003, vol. 6, 174-178
- **ASOKAN et al.** *Mol. Ther.*, 2012, vol. 20 (4), 699-708
- Remington: The Science and Practice of Pharmacy. Mack Publishing Co., 2003
- Remington's Pharmaceutical Sciences. Mack Publishing Co., 1990
- The Merck Index. Merck Publishing Group, 1996
- Pharmaceutical Principles of Solid Dosage Forms. Technonic Publishing Co., Inc., 1993
- **ANSEL** ; **STOKLOSA**. Pharmaceutical Calculations. Lippincott Williams & Wilkins, 2001
- **POZNANSKY et al.** Drug Delivery Systems. 1980, 253-315

- **ZINN et al.** *Cell Rep.*, 2015, vol. 12 (6), 1056-1068
- **GEORGIADIS et al.** *Gene Therapy*, 2016, vol. 23, 857-862
- **GEORGIADIS et al.** *Gene Therapy*, 2018, vol. 25, 450
- **PUZZO et al.** *Sci. Transl. Med.*, 2017, vol. 29 (9), 418
- **DUAN et al.** *J. Virol.*, 2001, vol. 75, 7662-7671
- **HALBERT et al.** *. Virol.*, 2000, vol. 74, 1524-1532
- **ZOLOTUKHIN et al.** *Methods*, 2002, vol. 28, 158-167
- **AURICCHIO et al.** *Hum. Molec. Genet.*, 2001, vol. 10, 3075-3081

- **WU**. *Human Gene Therapy*, 2007, vol. 18 (2), 171-82
- **MCCARTY et al.** *Gene Therapy*, 2001, vol. 8 (16), 1248-1254
- **HALBERT et al.** *J. Virol.*, 2000, vol. 74, 1524-1532
- **PAUL et al.** *Human Gene Therapy*, 1993, vol. 4, 609-615
- **BRUMENT et al.** *Mol. Therapy*, 2002, vol. 6 (5), 678-686
- **GAO et al.** *Hum. Gene Therapy*, 2000, vol. 11, 2079-2091
- **LI et al.** *Cell & Gene Therapy Insights*, 2019, vol. 5 (4), 537-547
- **WRIGHT et al.** *Molecular Therapy*, 2005, vol. 12 (1), 171-189
- *United States Pharmacopeia (USP)*, 2019
- **FRANGOIS et al.** *Molecular Therapy Methods & Clinical Development*, 2018, vol. 10, 223-236
- **CROYLE et al.** *Gene Ther.*, 2001, vol. 8 (17), 1281-90
- **CHANG** ; **RANDALL**. *Cryobiology*, 1992, vol. 29, 632-656
- **IZUTSU et al.** *Chem Pharm Bull*, 2009, vol. 57, 1231-1236
- **KETS et al.** *Cryobiology*, 2004, vol. 48, 46-54